(19) Europäisches Patentamt
European Patent Office
Office européen des brevets

(11) EP 0 640 618 B1

# (12) EUROPÄISCHE PATENTSCHRIFT

(45) Veröffentlichungstag und Bekanntmachung des Hinweises auf die Patenterteilung:
**20.10.1999 Patentblatt 1999/42**

(51) Int Cl.[6]: **C07K 5/10**, C07K 7/06, C07K 7/08

(21) Anmeldenummer: **94112544.5**

(22) Anmeldetag: **11.08.1994**

## (54) Tetrahydronaphthalin-Peptidderivate

Tetrahydronaphthalene-peptide derivatives

Dérivés peptidiques de la tétrahydronaphthalène

(84) Benannte Vertragsstaaten:
**AT BE CH DE DK ES FR GB IT LI NL**

(30) Priorität: **27.08.1993 CH 255293**

(43) Veröffentlichungstag der Anmeldung:
**01.03.1995 Patentblatt 1995/09**

(73) Patentinhaber: **F. HOFFMANN-LA ROCHE AG**
**4002 Basel (CH)**

(72) Erfinder:
- **Abrecht, Christine**
**CH-2543 Lengnau (CH)**
- **Müller, Klaus**
**CH-4142 Münchenstein (CH)**
- **Obrecht, Daniel**
**CH-4055 Basel (CH)**
- **Trzeciak, Arnold**
**D-79650 Schopfheim (DE)**

(74) Vertreter: **Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 592 791**

- **TETRAHEDRON, (INCL. TETRAHEDRON REPORTS), Bd.49, Nr.17, 23. April 1993, OXFORD GB Seiten 3479 - 3488 J.F. CALLAHAN ET AL. 'The Use of gamma-Turn mimetics to Define Peptide Secondary structure'**
- **JOURNAL OF MEDICINAL CHEMISTRY, Bd.35, Nr.21, 16. Oktober 1992, WASHINGTON US Seiten 3970 - 3972 J.F. CALLAHAN ET AL. 'Design and Synthesis of a C7 miemtic for the Predicted gamma-Turn Conformation Found in Several Constrained RGD Antagonists'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 640 618 B1

Printed by Jouve, 75001 PARIS (FR)

## Beschreibung

Die vorliegende Erfindung betrifft Tetrahydronaphthalin-Derivate, und zwar in erster Linie Tetrahydronaphthalinderivate der allgemeinen Formeln

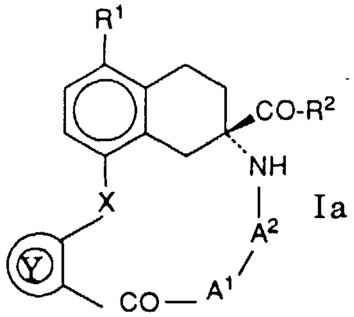
Ia und
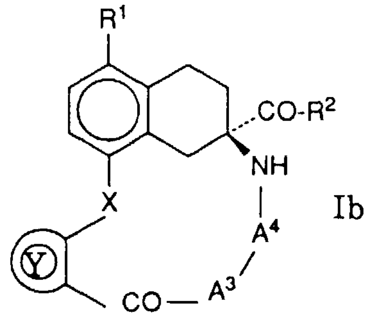
Ib

worin

| | |
|---|---|
| $R^1$ | Wasserstoff, Brom, Cyano, Formyl, Hydroxy, niederes Alkyl, niederes Alkenyl, niederes Alkinyl, niederes Alkoxy, Aryloxy, niederes Aralkoxy oder Aryl; |
| $R^2$ | eine gegebenenfalls geschützte Kette von bis zu 20 Aminosäureresten, deren C-terminale Aminosäure als freie oder geschützte Säure oder in Form eines Amids vorliegen kann; |
| $A^1$, $A^2$, $A^3$ und $A^4$ | je Reste von α-Aminosäuren, deren α-C-Atom, wenn es unsymmetrisch ist, in $A^1$ und $A^2$ in der L-Konfiguration und in $A^3$ und $A^4$ in der D-Konfiguration vorliegt; |
| X | Sauerstoff oder Schwefel; und |
| Y | zusammen mit den beiden C-Atomen einen gegebenenfalls substituierten Benzol-, Furan-, Thiophen-, Pyridin- oder Pyrimidinring bedeuten; |

sowie Salze davon.

Die Verbindungen der allgemeinen Formeln Ia und Ib und deren Salze sind neu. Diese Verbindungen und Salze, insbesondere diejenigen, welche keine Schutzgruppe(n) enthalten, sind wertvolle Hilfsmittel, um biologisch aktive Peptidsequenzen zu ermitteln und stellen somit sogenannte "Research Tools" dar; sie sind aber auch potentiell als Heilmittel geeignet.

Gegenstand der vorliegenden Erfindung sind die Verbindungen der allgemeinen Formeln Ia und Ib und Salze davon, deren Herstellung, Zwischenprodukte zu deren Herstellung und die Verwendung von Verbindungen der allgemeinen Formeln Ia und Ib und von Salzen davon als Research Tools.

Der Ausdruck "niederes Alkyl" umfasst geradkettige oder verzweigte gesättigte Kohlenwasserstoffreste mit bis zu 7, vorzugsweise bis zu 4 Kohlenstoffatomen, wie Methyl, Aethyl, n-Propyl, Isopropyl, n-Butyl, Isobutyl, tert.Butyl u.dgl. In analoger Weise umfassen die Ausdrücke "niederes Alkenyl" und "niederes Alkinyl" ungesättigte, eine Doppel- bzw. Dreifachbindung enthaltende Kohlenwasserstoffreste mit bis zu 7, vorzugsweise bis zu 4 Kohlenstoffatomen, wie Vinyl, Ethinyl, Allyl, Propargyl u.dgl. Der Ausdruck "niederes Alkoxy" umfasst Alkyloxygruppen im Sinne der obigen Umschreibung des Begriffs "niederes Alkyl". Der Ausdruck "Aryl" umfasst gegebenenfalls durch niederes Alkyl oder niederes Alkoxy mono- oder disubstituierte oder durch niederes Alkylendioxy substituierte Phenylreste. Der Ausdruck "Halogen" bezeichnet die vier Formen Fluor, Chlor, Brom und Jod, sofern nicht ausdrücklich anders angegeben.

Als Aminosäurereste kommen in erster Linie solche in Betracht, welche sich von α-Aminosäuren ableiten, insbesondere von natürlichen α-Aminosäuren; die Aminosäurereste können, sofern deren α-C-Atom unsymmetrisch ist, nicht nur in der L-Form, sondern auch in der D-Form vorliegen, und sie können gegebenenfalls geschützt sein. Nachstehend folgt eine Liste von Aminosäuren, welche bzw. deren Reste für die Zwecke der vorliegenden Erfindung geeignet sind, wobei die Abkürzungen den einschlägigen IUPAC-Regeln (Biochemistry 11, 1726 (1972)) und den allgemeinen üblichen Gepflogenheiten entsprechen.

| | |
|---|---|
| $Ac_3c$ | 1-Aminocyclopropancarbonsäure |
| $Ac_4c$ | 1-Aminocyclobutancarbonsäure |
| $Ac_5c$ | 1-Aminocyclopentancarbonsäure |
| $Ac_6c$ | 1-Aminocyclohexancarbonsäure |
| $Ac_7c$ | 1-Aminocycloheptancarbonsäure |
| Aib | 2-Amino-2-methylpropionsäure |
| Ala | L-Alanin |
| D-Ala | D-Alanin |
| β-Ala | β-Alanin |
| Arg | L-Arginin |
| D-Arg | D-Arginin |
| Asn | L-Asparagin |
| D-Asn | D-Asparagin |
| Asp | L-Asparaginsäure |
| D-Asp | D-Asparaginsäure |
| D-Asp (ONa) | Natrium-D-aspartat |
| $C_3al$ | L-3-cyclopropyalanin |
| $C_4al$ | L-3-cyclobutylalanin |
| $C_5al$ | L-3-cyclopentylalanin |
| $C_6al$ | L-3-cyclohexylalanin |
| Cys | L-Cystein |
| D-Cys | D-Cystein |
| Glu | L-Glutaminsäure |
| D-Glu | D-Glutaminsäure |
| Gln | L-Glutamin |
| D-Gln | D-Glutamin |
| Gly | Glycin |
| His | L-Histidin |
| D-His | D-Histidin |
| Hyp | 4-Hydroxy-L-prolin |
| Ile | L-Isoleucin |
| alle | L-Alloisoleucin |
| D-Ile | D-Isoleucin |
| D-alle | D-Alloisoleucin |
| D-Itg | D-2-(Isothiazolyl)glycin |
| Leu | L-Leucin |
| D-Leu | D-Leucin |
| tert.-Leu | L-2-Amino-3,3-dimethylbuttersäure |
| D-tert.-Leu | D-2-Amino-3,3-dimethylbuttersäure |
| Lys | L-Lysin |
| D-Lys | D-Lysin |
| Lys (CHO) | $N^6$-Formyl-L-lysin |
| MeAla | N-Methyl-L-alanin |
| MeLeu | N-Methyl-L-leucin |
| MeMet | N-Methyl-L-methionin |
| Met | L-Methionin |
| D-Met | D-Methionin |
| Met(O) | L-Methionin-sulfoxid |
| D-Met(O) | D-Methionin-sulfoxid |
| $Met(O_2)$ | L-Methionin-sulfon |
| $D-Met(O_2)$ | D-Methionin-sulfon |
| Nal | L-3-(1-Naphthylalanin) |
| D-Nal | D-3-(1-Naphthylalanin) |
| Nle | L-Norleucin |
| D-Nle | D-Norleucin |
| Nva | L-Norvalin |
| D-Nva | D-Norvalin |
| Orn | L-Ornithin |

| | |
|---|---|
| D-Orn | D-Ornithin |
| Orn(CHO) | $N^5$-Formyl-L-ornithin |
| Phe | L-Phenylalanin |
| D-Phe | D-Phenylalanin |
| L-Phg | L-Phenylglycin |
| D-Phg | D-Phenylglycin |
| Pip | L-Pipecolinsäure |
| D-Pip | D-Pipecolinsäure |
| Pro | L-Prolin |
| D-Pro | D-Prolin |
| Sar | Sarcosin |
| Ser | L-Serin |
| D-Ser | D-Serin |
| Thr | L-Threonin |
| D-Thr | D-Threonin |
| Thz | L-Thiazolidin-4-carbonsäure |
| D-Thz | D-Thiazolidin-4-carbonsäure |
| Trp | L-Tryptophan |
| D-Trp | D-Tryptophan |
| D-Trp(CHO) | $N^{in}$-Formyl-D-tryptophan |
| D-Trp(O) | D-3-(2,3-Dihydro-2-oxoindol-3-yl)alanin |
| Tyr | L-Tyrosin |
| D-Tyr | D-Tyrosin |
| Tza | L-3-(2-Thiazolyl)alanin |
| D-Tza | D-3-(2-Thiazolyl)alanin |
| Tzg | L-2-(Thiazolyl)glycin |
| D-Tzg | D-2-(Thiazolyl)glycin |
| Val | L-Valin |
| D-Val | D-Valin |

Geeignete Schutzgruppe für Aminosäuren bzw. deren Reste sind beispielsweise

- für die Aminogruppe (wie sie z.B. auch in der Seitenkette von Lysin vorliegt)

| | |
|---|---|
| Z | Benzyloxycarbonyl |
| Boc | tert.- Butyloxycarbonyl |
| Fmoc | Fluoren-9-ylmethoxycarbonyl |
| Alloc | Allyloxycarbonyl |
| Teoc | Trimethylsilyläthoxycarbonyl |
| Tcc | Trichloräthoxycarbonyl |
| Nps | o-Nitrophenylsulfenyl; |

- für die Carboxylgruppe (wie sie z.B. auch in der Seitenkette von Asparginsäure und Glutaminsäure vorliegt) durch Ueberführung in entsprechende Ester mit den Alkoholkomponenten

| | |
|---|---|
| tBu | tert.-Butyl |
| Bzl | Benzyl |
| Me | Methyl |
| Ph | Phenyl |
| Pac | Phenacyl |
| | Allyl |
| | Trimethylsilyläthyl |
| | Trichloräthyl; |

- für die Guanidingruppe (wie sie beispielsweise in der Seitenkette von Arginin vorliegt)

| | |
|---|---|
| Pmc | 2,2,5,7,8,-Pentamethylchroman-6-sulfonyl |
| Ts | Tosyl |

Z Benzyloxycarbonyl;

- für die Hydroxygruppe (wie sie beispielsweise in der Seitenkette von Threonin und Serin vorliegt)

tBu tert.-Butyl
Bzl Benzyl
Trityl;

- und für die Mercaptogruppe (wie sie beispielsweise in der Seitenkette von Cystein vorliegt)

tBu tert.-Butyl
Bzl Benzyl
Trityl
2-Methoxytrityl.

Wenn in Formel I $R^1$ Aryl bedeutet, dann handelt es sich zweckmässigerweise um einen durch niederes Alkyl oder niederes Alkoxy mono- oder disubstituierten oder einen durch niederes Alkylendioxy substituierten Phenylrest. $R^2$ enthält zweckmässigerweise 9 bis 15 Aminosäurereste, insbesondere 9, 12 oder 15 Aminosäurereste. In erster Linie kommen Reste von α-Aminosäuren in Betracht, deren α-C-Atom, wenn es asymmetrisch ist, vorzugsweise in der L-Konfiguration vorliegt. Beispielsweise können die in $R^2$ enthaltenen Aminosäurereste von L-Alanin, L-Glutamin, L-Glutaminsäure, L-Isoleucin, L-Leucin, L-Lysin oder 2-Amino-2-methylpropionsäure abgeleitet sein. Wenn die C-terminale Aminosäure im Rest $R^2$ in Form eines Amids vorliegt, so kann die Amidgruppe am Stickstoffatom unsubstituiert sein oder auch substituiert sein, beispielsweise durch 4-Jodphenyl. Zweckmässigerweise bedeuten $A^1$ bzw. $A^3$ einen Rest von L- bzw. D-Alanin, Asparagin, Asparaginsäure, Glutamin, Glutaminsäure oder Lysin oder von 2-Amino-2-methylpropionsäure und $A^2$ bzw. $A^4$ einen Rest von L- bzw. D-Alanin, Asparagin, Asparaginsäure, Glutamin, Glutaminsäure oder Lysin. Vorzugsweise bedeuten $A^1$ bzw. $A^3$ L- bzw. D-Alanyl oder Lysyl und $A^2$ bzw. $A^4$ L- bzw. D-Alanyl.

Wenn der durch Y bezeichnete Benzol-, Furan-, Thiophen-, Pyridin- oder Pyrimidinring substituiert ist, so ist er zweckmässigerweise durch Nitro, Amino, niederes Alkanoylamino, Hydroxy, niederes Alkoxy, niederes Alkanoyloxy, niederes Alkyl, Fluor, Cyano, Carboxy oder Formyl mono- oder disubstituiert oder mit einem Benzolring zu einem bicyclischen System kondensiert.

Repräsentative Verbindungen der Formeln Ia und Ib im Rahmen der vorliegenden Erfindung sind:

[(12S,15S,18S)-12,15-Dimethyl-10,13,16-trioxo-10,11,12,13,14,15,16,17, 18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-jodophenyl)-amid;
[(12R,15R,18R)-12,15-Dimethyl-10,13,16-trioxo-10,11,12,13,14,15,16,17,18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-jodophenyl)-amid;
[(12S,15S,18S)-12,15-Dimethyl-10,13,16-trioxo-10,11,12,13,-14,15,16,17,18,19,20,21-dodecahydrol-1,18-etheno-dibenz[b,n][1,5,8,11]oxatriaza-cyclopentadecin-18-yl-carbonyl]-L-leucyl-L-lysyl-L-alanyl-L-glutamyl-L-isoleucyl-L-alanyl-L-glutaminyl-L-lysyl-L-leucinamid trifluoracetat;
[(12R,15R,18R)-12,15-Dimethyl-10,13,16-trioxo-10,11,12,13,14,15,16,17,18,19,20,21-dodecahydro-1,18-etheno-dibenz[b,n][1,5,8,11]-oxatriazacyclopentadecin-18-yl-carbonyl]-L-leucyl-L-lysyl-L-alanyl-L-glutamyl-L-isoleucyl-L-alanyl-L-glutaminyl-L-lysyl-L-leucinamidtrifluoracetat;
[(14aS,17S,20S)-8-Acetylamino-17-methyl-10,15,18-trioxo-11,12,13,14,14a, 15,16,17, 18,19, 20,21,22,23-tetradecahydro-1,20-etheno-10H-pyrrolo[1,2-e]dibenz[b,n][1,5,8,11]-oxatriazacyclopentadecin-20-ylcarbonyl]-L-alanyl-L-alanyl-L-alanyl-L-alanyl-L-alanyl-L-alanyl-(2-amino-2-methyl-propionyl)-L-alanyl-L-alanin-(4-jodophenyl)-amid;
[(12S,15S,18S)-12-(2-Methoxycarbonyl-aethyl)-15-methyl-10,13,16-trioxo-10,11,12,13,14,15,16,17,18,19,20,21-dodecahydro-1,18-etheno-pyrido[2,3-b]benz-[n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid;
[(12S,16S,19S)-16-Methyl-10,13,17,23-tetraoxo-10,11,12,13,14,15,16,17,18,19,20,21,22,23-tetradecahydro-1,19-etheno-12,15-metheno-pyrido[2,3-b]benz[o][1,5,9,12]-oxatriazacyclohexadecin-19-ylcarbonyl]-L-alanyl-L-alanyl-L-alanyl-L-alanyl-L-alanyl-L-alanyl-(2-amino-2-methyl-propionyl)-L-alanyl-L-alanin-(4-iodo-phenyl)-amid;
[(12S,15S,18S)-12-(2-Carboxy-ethyl)-15-methyl-10,13,16-trioxo-10,11,12,13,14,15,16,17,18,19,20,21-dodecahydro-1,18-etheno-pyrido[2,3-b]benz[n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid; und

[(12S,15S,18S)-12-(2-Carboxymethyl)-15-methyl-10,13,16-trioxo-10,11,12,13,14,15,16,17,18,19,20,21-dodecahydro-1,18-etheno-pyrido[2,3-b]benzo[n][1,5,8]-oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanyl-(4-iodophenyl)-amid.

Die Verbindungen der Formeln Ia und Ib sowie deren Salze können erfindungsgemäss dadurch hergestellt werden, dass man

a) eine Verbindung der allgemeinen Formel

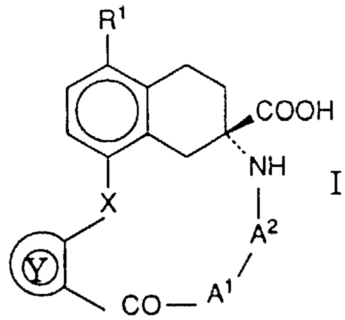
IIa bzw.
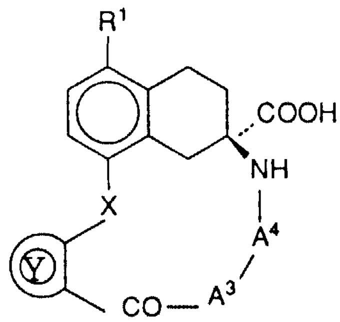
IIb

worin $R^1$, $A^1$, $A^2$, $A^3$, $A^4$, X und Y obige Bedeutung besitzen,
mit einer gegebenenfalls geschützten Kette von bis zu 20 Aminosäuren, deren C-terminale Aminosäure als freie oder geschützte Säure oder in Form eines Amids vorliegen kann, kuppelt; oder

b) eine Verbindung der allgemeinen Formel

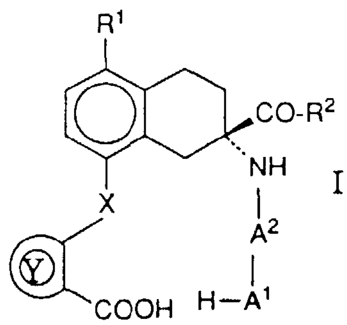
IIIa bzw.
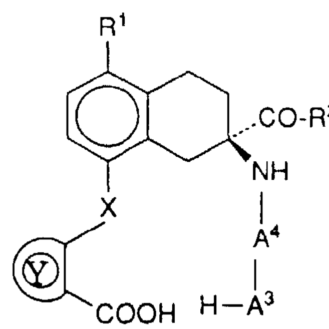
IIIb

worin $R^1$, $R^2$, $A^1$, $A^2$, $A^3$, $A^4$, X und Y obige Bedeutung besitzen,
cyclisiert; oder

c) aus einer Verbindung der Formel Ia bzw. Ib, welche mindestens eine Schutzgruppe enthält, die Schutzgruppe(n) abspaltet; oder

d) eine Verbindung der Formel Ia bzw. Ib, welche ein basisches bzw. ein saures Zentrum enthält, mittels einer Säure bzw. einer Base in ein Salz überführt.

Die Verbindungen der Formeln IIa, IIb, IIIa und IIIb sind neu und ebenfalls Gegenstand der Erfindung.
Gemäss Verfahrensvariante a) wird die freie Carboxylgruppe einer Verbindung der Formel IIa bzw. IIb mit einer Peptidkomponente mit N-terminaler freier Aminogruppe gekuppelt, und bei der Cyclisation einer Verbindung der Formel IIIa bzw. IIIb werden eine freie Carboxylgruppe und eine freie Aminogruppe unter Bildung einer Amidbindung miteinander gekuppelt.
Zur Durchführung dieser Verfahrensvarianten werden in der Peptidchemie allgemein übliche und jedem Fachmann geläufige Methoden verwendet. Dabei kann durchaus auch die Methode der Festphasensynthese verwendet werden; als Träger eignen sich beispielsweise Aminomethylpolystyrolharz u.dgl.

Um die Kupplung gemäss Verfahrensvarianten a) und b) herbeizuführen, können alle möglichen in der Peptidchemie gebräuchlichen Aktivierungsreagenzien verwendet werden, wie z.B. O-Benzotriazol-1-yl-N,N,N',N'-tetramethyl-uroniumhexafluorphosphat (HBTU), O-(1,2-Dihydro-2-oxo-1-pyridyl)-N,N,N',N'-tetramethyl-uroniumtetrafluoroborat (TPTU), 2-(1H-Benzotriazol-1-yl)-1,1,3,3-tetramethyluroniumtetrafluoroborat (TBTU), 1-Hydroxybenzotriazol (HOBT) in Kombination mit N,N-Dicyclohexylcarbodiimid u.dgl.

Durch Abspaltung der Schutzgruppe(n) aus einer mindestens eine Schutzgruppe enthaltenden Verbindung der Formel Ia bzw. Ib erhält man gemäss Verfahrensvariante c) eine entsprechende Verbindung der Formel Ia bzw. Ib, welche keine Schutzgruppe(n) enthält. Die Abspaltung der Schutzgruppe(n) erfolgt nach in der Peptidchemie allgemein üblichen und jedem Fachmann geläufigen Methoden, wobei natürlich jeweils die Natur der Schutzgruppe(n) in Betracht zu ziehen ist. So können die weiter oben genannten Schutzgruppen beispielsweise wie folgt abgespalten werden:

| | |
|---|---|
| Z: | Katalytische Hydrierung in Gegenwart von Pd/ C in einem niederen Alkanol, wie Methanol oder Aethanol. |
| Boc: | Mittels Trifluoressigsäure/ Methylenchlorid (1:1) oder mittels gesättigter Chlorwasserstofflösung in Essigsäureäthylester. |
| Fmoc : | Mittels Piperidin oder 1,8-Diazabicyclo[5.4.0]undec-7-en in Dimethylformamid. |
| Alloc: | Mittels Palladium-tetrakis-triphenylphosphin in Tetrahydrofuran/Dimethylsulfoxid / 0,1 N Salzsäure. |
| Teoc: | Mittels Caesiumfluorid oder Tetrabutylammoniumfluorid in Dimethylformamid o.dgl. |
| Tcc : | Mittels Zink in Eisessig oder Methanol. |
| Nps: | Mittels Natrium- oder Kaliumrhodanid in leicht saurem Milieu. |
| $^t$Bu: | Mittels Trifluoressigsäure/ Methylenchlorid (1:1). |
| Bzl: | Durch katalytische Hydrierung in Gegenwart von Pd / C in einem niederen Alkanol, wie Methanol oder Aethanol. |
| Me: | Mittels Lithiumhydroxid in Tetrahydrofuran/Methanol/Wasser (3:1:1). |
| Ph : | Mittels Natriumperoxyd bei pH 10,5. |
| Pac : | Mittels Zink in Eisessig oder Methanol oder mittels Natriumthiophenolat in Dimethylformamid. |
| Allyl : | Mittels Palladium-bis-triphenylphosphin-dichlorid und Tributylzinnhydrid oder mittels Palladium-tetrakis-triphenylphosphin in Tetrahydrofuran/ Dimethylsulfoxyd/0,5 N Salzsäure. |
| Trimethylsilyläthyl : | Mittels Caesiumfluorid oder Tetrabutylammoniumfluorid in Dimethylformamid o.dgl. |
| Trichloräthyl: | Mittels Zink in Eisessig oder Methanol. |
| Pmc : | Mittels wässriger Trifluoressigsäure. |
| Ts : | Mittels Natrium in flüssigem Ammoniak oder flüssigem Fluorwasserstoff. |

In analoger Weise kann eine an einem Aminomethyl-polystyrolharz o.dgl. durch Festphasensynthese hergestellte Verbindung der Formel Ia bzw. Ib von dem Trägerharz abgespalten werden, beispielsweise mittels "Field's Reagens", d.h. einer Mischung aus 82,5% Trifluoressigsäure, 5% Phenol. 5% Wasser, 5% Thioanisol und 2,5% 1,2-Aethandithiol.

Gemäss Verfahrensvariante d) kann eine Verbindung der Formel Ia bzw. Ib, welche ein basisches bzw. ein

saures Zentrum enthält, mittels einer Säure bzw. einer Base in ein Salz übergeführt werden, was nach allgemein üblichen und jedem Fachmann geläufigen Methoden erfolgen kann. Als Säuren kann man hier für anorganische Säuren, wie Salzsäure, Schwefelsäure, Phosphorsäure o.dgl., oder organische Säuren, wie Trifluoressigsäure, Methansulfonsäure, p-Toluolsulfonsäure o.dgl., verwenden und als Base anorganischen Basen, wie Kaliumhydroxyd, Natriumhydroxyd o.dgl., oder organische Basen, wie Triaethylamin, Dimethylaminopyridin o.dgl.

Die für Verfahrensvarianten a) und b) benötigten Ausgangsprodukte der Formeln IIa bzw. IIb und IIIa bzw. IIIb können aus Verbindungen der allgemeinen Formeln

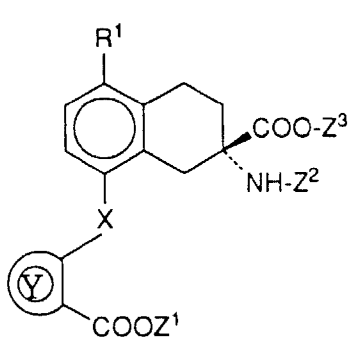 IVa bzw. 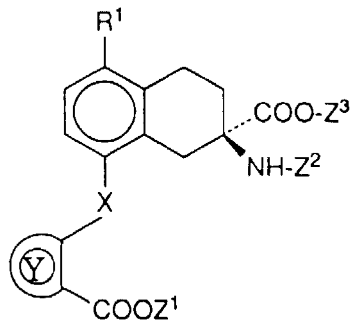 IVb

worin $R^1$ und Y obige Bedeutung besitzen und $Z^1$ und $Z^3$ Carboxylschutzgruppen und $Z^2$ eine Aminoschutzgruppe bedeuten,

hergestellt werden. Die Verbindungen der Formeln IVa und IVb sind neu und ebenfalls Gegenstand der vorliegenden Erfindung. Zweckmässigerweise bedeuten $Z^1$ Benzyl, tert.-Butyl, Allyl oder Pentafluorphenyl, $Z^2$ Benzyloxycarbonyl, tert.-Butyloxycarbonyl oder 9-Fluorenylmethoxycarbonyl und $Z^3$ Methyl, tert.-Butyl, Benzyl, Trimethylsilyläthyl oder Pentafluorphenyl.

Die Herstellung der Verbindungen der Formeln IVa und IVb und deren Ueberführung in Verbindungen der Formen IIa bzw. IIb und IIIa bzw. IIIb werden nachstehend, z.T. anhand von Reaktionsschemata, näher erläutert.

## Reaktionsschema 1

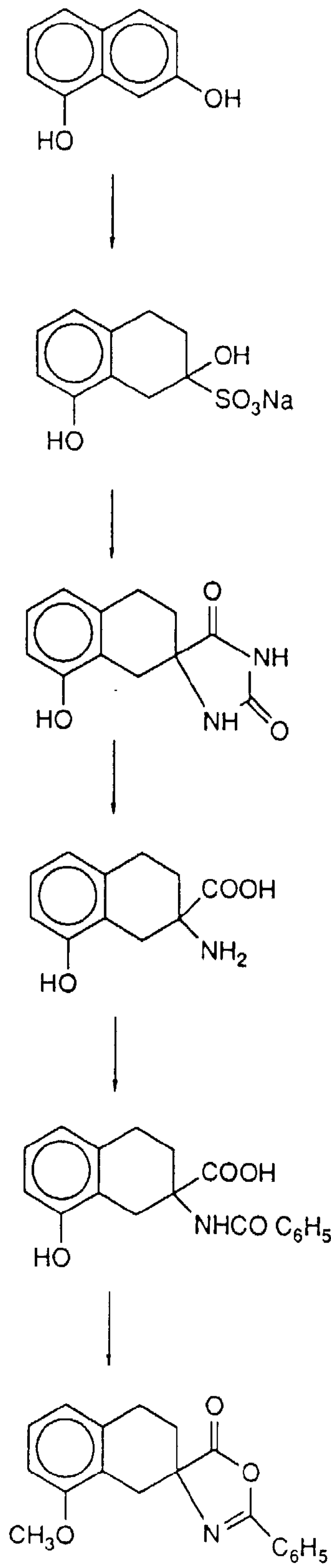

V

VI

VII

VIII

IX

X

## Reaktionsschema 2

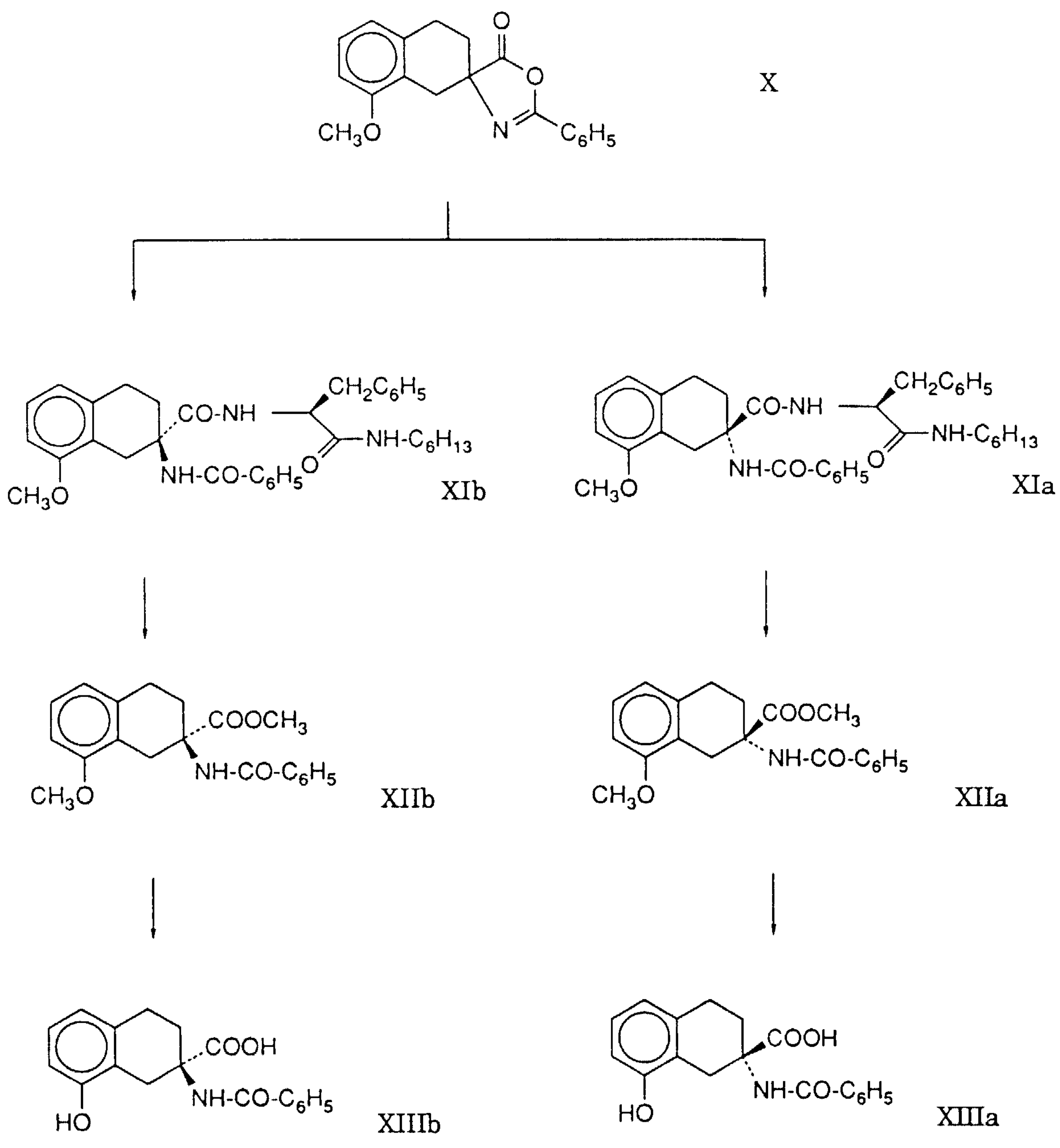

X

XIb XIa

XIIb XIIa

XIIIb XIIIa

V → VI

1,7-Dihydroxynaphthalin (V) kann mittels Lithium in flüssigem Ammoniak/Tetrahydrofuran/tert.-Butanol in 8-Hydroxy-1,2,3,4-tetrahydronaphthalin-2-on übergeführt werden, welches - ohne dass es isoliert zu werden braucht - mittels Natriumhydrogensulfit in (rac)-2,8-Dihydroxy-1,2,3,4-tetrahydronaphthalin-2-sulfonsäure-Natriumsalz (VI) übergeführt werden kann.

VI → VII

Das Natriumsalz der Formel VI kann mittels Kaliumcyanid und Ammoniumcarbonat in Aethanol/Wasser in die racemische Spiroverbindung der Formel VII übergeführt werden.

VII → VIII

Die Spiroverbindung der Formel VII kann durch Erhitzen mit Bariumhydroxid in Wasser und anschliessendes Ansäuern, z.B. mit wässeriger Schwefelsäure, in (rac)-2-Amino-8-hydroxy-1,2,3,4-tetrahydronaphthalin-2-carbonsäure (VIII) übergeführt werden.

VIII → IX

Die Verbindung der Formel VIII wird zunächst mittels Benzoylchlorid o.dgl. acyliert, zweckmässigerweise in Gegenwart eines säurebindenden Mittels, wie Natriumhydroxid o.dgl. Durch Behandlung des Acylierungsprodukts mit Natronlauge in Dioxan kann man dann zu (rac)-2-Benzamido-8-hydroxy-1,2,3,4-tetrahydronaphthalin-2-carbonsäure gelangen.

IX → X

Durch Behandlung der Verbindung der Formel IX mit Dicyclohexylcarbodiimid in Dichlormethan o.dgl. und anschliessende Methylierung, zweckmässigerweise mittels Dimethylsulfat in Gegenwart einer starken Base, wie Natriumhydrid, in Dioxan o.dgl. kann man zu (rac)-3,4-Dihydro-8-methoxy-2-phenylspiro[naphthalin-2(1H),4'(5'H)-oxazol]-5'-on gelangen.

X → XIa und XIb

Die Verbindung der Formel X kann mittels L-Phenylalanin-cyclohexylamid in ein Gemisch der beiden diastereoisomeren Verbindungen der Formeln XIa und XIb übergeführt werden, welches durch Kristallisation in seine Bestandteile XIa und XIb zerlegt werden kann.

XIa → XIIa und XIb → XIIb

Durch Behandlung der Verbindung der Formel XIa bzw. XIb mit Trifluormethansulfonsäure erhält man L-Phenylalanin-cyclohexylamidtrifluormethansulfonat und die Verbindung der Formel XIIa bzw. XIIb.

XIIa → XIIIa und XIIb → XIIIb

Durch Behandlung der Verbindung der Formel XIIa bzw. XIIb mit Bortribromid in Dichlormethan o.dgl. erhält man die Verbindung der Formel XIIIa bzw. XIIIb.

Ausgehend von der Verbindung der Formel XIIIa sind verschiedene Abwandlungen möglich, welche im nachstehenden Reaktionsschema 3 veranschaulicht werden; dieselben Abwandlungen sind natürlich auch ausgehend von der Verbindung der Formel XIIIb möglich.

## Reaktionsschema 3

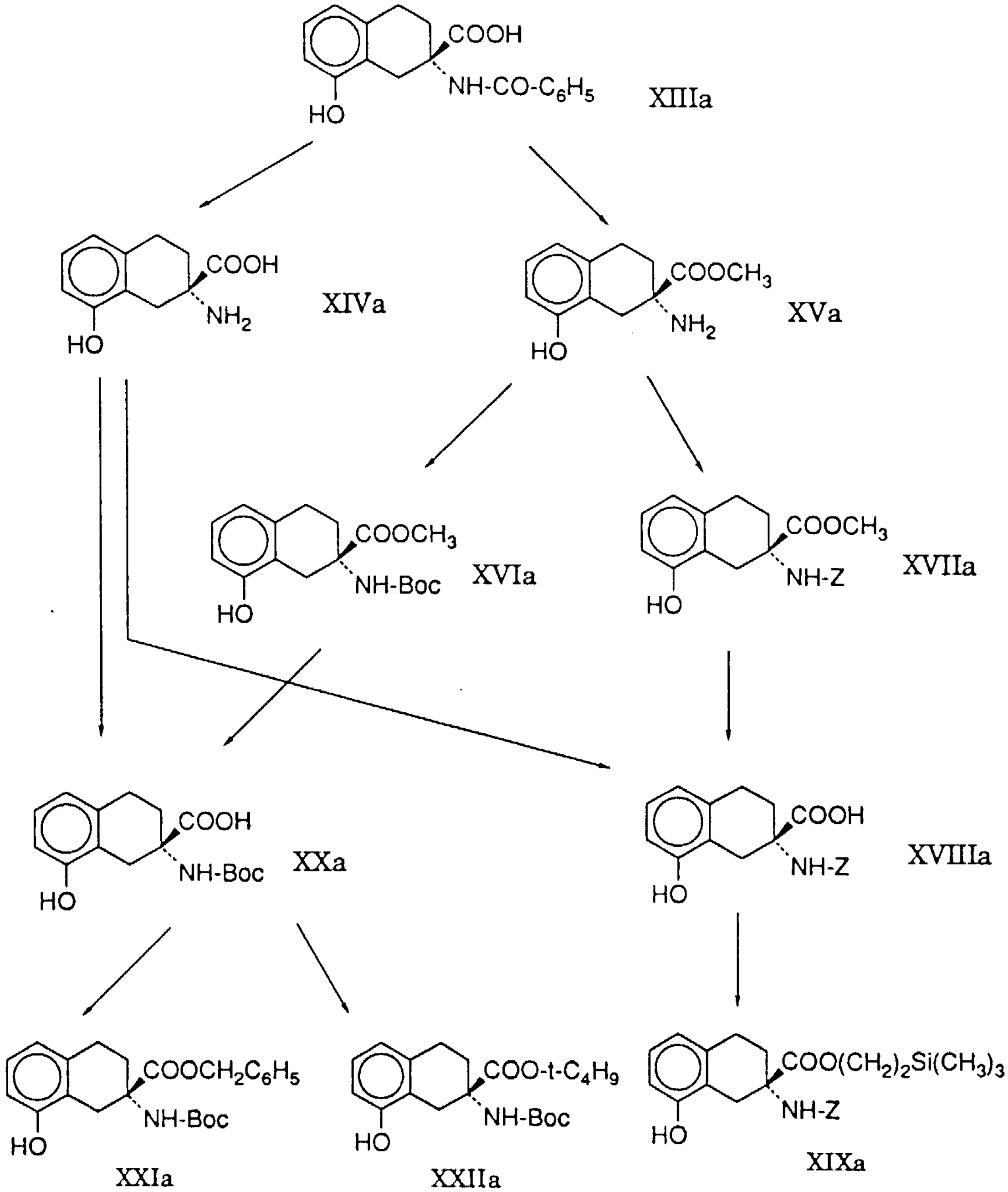

XIIIa

XIVa XVa

XVIa XVIIa

XXa XVIIIa

XXIa XXIIa XIXa

Z bedeutet Benzyloxycarbonyl

Boc bedeutet tert.-Butyloxycarbonyl

Die verschiedenen Abwandlungen gemäss Reaktionsschema 3 werden nach an sich bekannten und jedem Fachmann geläufigen Methoden durchgeführt, zweckmässigerweise mittels der nachfolgend erwähnten Reagenzien.

<u>XIIIa → XIVa</u>

25%ige Salzsäure in Dioxan bei 100° (Bombenrohr).

<u>XIIIa → XVa</u>

25%ige Salzsäure in Dioxan bei 100° (Bombenrohr), dann Oxalylchlorid in methanolischer Salzsäure.

XVa → XVIa

Di-tert.-Butyldicarbonat in Dimethylformamid.

XVa → XVIIa

N-(Benzyloxycarbonyloxy)-succinimid und Natriumhydrogencarbonat in Dioxan.

XVIIa → XVIIIa

Lithiumhydroxid in Tetrahydrofuran/Methanol/Wasser.

XIVa → XVIIIa

N-(Benzyloxycarbonyloxy)-succinimid und Natriumhydrogencarbonat in Dioxan.

XVIIIa → XIXa

Trimethylsilyläthanol/N-Aethyl-N'-(3-dimethylaminopropyl)-carbodiimid-hydrochlorid/1-N-Hydroxybenzotriazol/Aethyl-diisopropylamin/N,N-Dimethylaminopyridin in Dimethylformamid.

XIVa → XXa

Di-tert.-butyldicarbonat/Trimethylchlorsilan/Ethyl-diisopropylamin in Dichlormethan.

XVIa → XXa

Lithiumhydroxid in Tetrahydrofuran/Methanol/Wasser.

XXa → XXIa

Benzylbromid/1,8-Diazabicyclo[5.4.0]undec-7-en in Dimethylformamid oder Phenyldiazomethan in Dichlormethan.

XXa → XXIIa

N,N-Dimethylformamid-di-tert.-butylacetal in Toluol.

Verbindungen vom Typus XVIa, XVIIa, XIXa, XXIa und XXIIa können in 5-Stellung bromiert werden, zweckmässigerweise mittels N-Bromsuccinimid in Trifluoräthanol, und anschliessend sind bereits auf dieser Stufe gewisse weitere Variationen (wie weiter unten beschrieben) in 5-Stellung des Tetrahydronaphthalingerüstes möglich (vgl. die verschiedenen Bedeutungsmöglichkeiten von $R^1$ in Formeln Ia und Ib), wobei die phenolische Hydroxylgruppe unter Umständen temporär geschützt werden muss. Weiterhin kann in Verbindungen vom Typus XVIa, XVIIa, XIXa, XXIa und XXIIa oder in entsprechenden 5-Bromverbindungen oder in Abwandlungsprodukten davon mit anderen Substituenten in 5-Stellung die phenolische Hydroxylgruppe durch eine SH-Gruppe ersetzt werden, beispielsweise nach einer der folgenden Methoden:

a) Umsetzung mit N,N-Diethyl-thiocarbamoylchlorid, gefolgt von Erhitzen und dann alkalischer Hydrolyse, vgl. M. S. Newman, H.A. Karnes, J. Org. Chem. 31, 3980 (1966);

b) Behandlung mit Trifluormethansulfonsäureanhydrid in Pyridin; dann Umsetzung des erhaltenen Trifluormethansulfonats mit Thioharnstoff in Gegenwart eines Nickelkatalysators, gefolgt von Behandlung mit Alkali und dann mit Säure, vgl. K. Takagi, Chem. Lett. 1985, 1307.

Zu Verbindungen der Formeln IVa und IVb gelangt man ausgehend von Verbindungen vom Typus XVIa, XVIIa, XIXa, XXIa und XXIIa oder von Abwandlungsprodukten davon, wie vorstehend beschrieben, bzw. von entsprechenden, jedoch aus Verbindung XIIIb hergestellten Verbindungen durch Umsetzung mit entsprechenden Halogen-arylcarbonsäureestern, wie 2-Iodbenzoesäurebenzylester, 3-Bromthiophen-2-carbonsäure-benzylester, 2-Iod-benzoesäure-allylester, 2-Brom-5-nitrobenzoesäurebenzylester, 2-Brom-nicotinsäure-tert.-butylester, 2-Brom-nicotinsäureallylester u.

dgl. in Pyridin in Gegenwart von Natriumhydrid und von Kupferbromid-dimethylsulfid-Komplex.

In den erhaltenen Verbindungen der Formeln IVa und IVb sind verschiedene Abwandlungen möglich.

Einerseits können Variationen bezüglich der Schutzgruppen durchgeführt werden. So kann z.B. eine tert.-Butoxycarbonylaminogruppe in eine Fluorenylmethoxycarbonylaminogruppe, ein tert.-Butylester in einen Pentafluorphenylester oder ein Benzylester in einen Pentafluorphenylester übergeführt werden, was nach allgemein üblichen und jedem Fachmann geläufigen Methoden erfolgen kann.

Anderseits werden Variationen bezüglich der 5-Stellung des Tetrahydronaphthalingerüstes zweckmässigerweise auf dieser Stufe durchgeführt; die Einführung von Brom erfolgt jedoch zweckmässigerweise bereits auf einer früheren Stufe, nämlich auf der Stufe der Verbindungen von Typus XVIa, XVIIa, XIXa, XXIa und XXIIa. So können Verbindungen der Formel IVa und IVb, worin $R^1$ Brom bedeutet, beispielsweise wie folgt abgewandelt werden:

a) Umsetzung mit Organozinnverbindungen in Gegenwart eines Palladiumkatalysators, vgl. T.N. Mitchell, Synthesis 1992, 803-815; J.K. Stille, Angew. Chem. 98, 504-519 (1986); D.R. McKean, G. Parinello, A.F. Renaldo, J.K. Stille, J. Org. Chem. 52, 422 (1987). Beispielsweise erhält man mittels 3,4-Dimethoxyphenyl-trimethylstannan in Gegenwart von Tetrakis-(triphenylphosphin)-palladium in Dioxan eine Verbindung der Formel IVa bzw. IVb, worin $R^1$ 3,4-Dimethoxyphenyl bedeutet.

b) Umsetzung mit Boronaten in Gegenwart eines Palladiumkatalysators, vgl. X. Wang, V. Sniekus, Tetrahedron Lett. 1991, 4879; B.I. Alo, A. Kandil, P.A. Patil, M.J. Sharp, M.A. Siddiqui, V. Snieders, J. Org. Chem. 56, 3763 (1991); T.Oh-e, N. Miyaura, A. Suzuki, Synlett 1990, 221.

Methoden a) und b) eignen sich zur Einführung von Aryl-, Alkenyl-, Alkinyl- und Alkyl-Resten.

c) Umsetzung mit Kohlenmonoxid und einem Alkohol in Gegenwart von Palladium-diacetat o.dgl. und einer Base, vgl. J.K. Stille, P.K. Wong, J. Org. Chem. 40, 532 (1975); A. Cowell, J.K. Stille, J. Amer. Chem. Soc. 102, 4193 (1980). Diese Methode eignet sich zur Einführung von Alkoxycarbonylgruppen.

d) Umsetzung mit Kohlenmonoxid und Wasserstoff in Gegenwart von Triethylamin o.dgl. und Bis-(triphenylphosphin)Palladiumdichlorid o.dgl., vgl. A. Schoenberg, R.F. Heck, J. Amer. Chem. Soc. 96, 7761 (1974); H. Yoshida, N. Sugita, K. Kudo, Y. Takezaki, Bull. Chem. Soc. Japan 49, 1681 (1976). Diese Methode eignet sich zur Einführung der Formylgruppe.

e) Umsetzung mit Kaliumcyanid in Gegenwart von 1,1'-Bis-(diphenylphosphino)-ferrocen und Bis-(dibenzylidenaceton)-di-palladium in N,N-Dimethylacetamid, vgl. K. Takagi, Y. Sakakibara, Chem. Lett. 1989, 1957. Diese Methode eignet sich zur Einführung der Cyanogruppe.

f) Umwandlung der Formylgruppe in eine Alkenylgruppe durch Wittig-Reaktion.

g) Behandlung mit tert.-Butyllithium und anschliessende Umsetzung mit geeigneten Elektrophilen wie z.B. N-Formylpiperidin (liefert Formyl), Chlorameisensäureester (liefert Alkoxycarbonyl) usw.

h) Ullmann-Kopplung mit einem Phenol in Gegenwart von Natriumhydrid und Kupferbromid-Dimethylsulfid-Komplex in Pyridin, vgl. D.L. Boger, D. Yohannes, J. Org. Chem. 55, 6000 (1990); D.A. Evans, J.A. Ellmann, J. Amer. Chem. Soc. 111, 1063 (1989). Diese Methode eignet sich zur Einführung von Aryloxygruppen.

i) Umsetzung Alkoholen, insbesondere primären Alkoholen, unter Phasentransfer-Katalyse (z.B. PEG-6000, KOH), vgl. R. Neumann, Y. Sasson, Tetrahedron 39, 3437 (1983). Diese Methode eignet sich zur Einführung von Alkoxy- und Aralkoxygruppen.

k) Umwandlung der Formylgruppe in die Hydroxygruppe mittels modifizierter Bayer-Villiger-Reaktion (Natriumpercarbonat/Trifluoressigsäure), vgl. G. Olah, Synthesis 1991, 739.

Ausgehend von Verbindungen der Formel IVa bzw. IVb kann man zu Verbindungen der Formel IIa bzw. IIb gelangen, indem man zunächst die durch $Z^2$ geschützte Aminogruppe freisetzt, dann mit einer den Rest $A^2$ bzw. $A^4$ liefernden Aminosäurekomponente und hierauf mit einer den Rest $A^1$ bzw. $A^3$ liefernden Aminosäurekomponente kuppelt, anschliessend cyclisiert und abschliessend die durch $Z^3$ geschützte Carboxylgruppe freisetzt. Im Verlauf dieser Synthese können an anderen Stellen des Moleküls gewisse Variationen erfolgen. So kann eine sich am Rest Y befindliche Nitrogruppe anlässlich einer Reduktion zur Aminogruppe abgewandelt werden, welche acyliert werden kann.

Zu Verbindungen der Formel IIIa bzw. IIIb kann man ausgehend von Verbindungen der Formel IVa bzw. IVb gelangen, indem man zunächst die durch $Z^3$ geschützte Carboxylgruppe freisetzt, diese mit einer den Rest $R^2$ liefernden Peptidkomponente kuppelt, hierauf die durch $Z^2$ geschützte Aminogruppe freisetzt und dann mit einer den Rest $A^2$ bzw. $A^4$ und hierauf mit einer den Rest $A^1$ bzw. $A^3$ liefernden Aminosäurekomponente kuppelt.

Die Herstellung der Verbindungen der Formel IIa bzw. IIb und der Formel IIIa bzw. IIIb aus den Verbindungen der Formel IVa bzw. IVb erfolgt nach in der Peptidchemie üblichen und jedem Fachmann geläufigen Methoden.

Wird im Zuge dieser Synthese als den Rest $A^1$ bzw. $A^3$ liefernde Aminosäurekomponente ein Asparaginsäurederivat eingesetzt, so kann in der erhaltenen Verbindung die Carboxylgruppe des Aspartylrests nicht in freier Form, sondern als Glied eines mit dem Stickstoffatom der $A^1$ bzw. $A^3$ mit $A^2$ bzw. $A^4$ verbindenden Amidbindung gebildeten fünfgliedrigen Rings vorliegen; dieser Ring kann - zweckmässigerweise erst nach der Ueberführung in eine Verbindung der Formel Ia bzw. Ib - wieder geöffnet werden, z.B. mittels Lithiumhydroxid o.dgl.

Die Verbindungen der Formeln Ia und Ib enthalten Aminosäuresequenzen (vgl. den Rest $R^2$), wobei in den Verbindungen der Formel Ia die Konformationen von rechtsgängigen peptidischen $\alpha$-Helices (mit L-Aminosäureresten) stabilisiert und die Konformationen von linksgängigen peptidischen $\alpha$-Helices (mit D-Aminosäureresten) destabilisiert werden, wogegen in den Verbindungen der Formel Ib die Konformationen von linksgängigen peptidischen $\alpha$-Helices (mit D-Aminosäureresten) stabilisiert und die Konformationen von rechtsgängigen peptidischen $\alpha$-Helices (mit L-Aminosäureresten) destabilisiert werden.

Die den Verbindungen der Formel IIa bzw. IIb entsprechenden Strukturelemente in den Verbindungen der Formel Ia bzw. Ib können als Mimetica einer $\alpha$-helicalen Windung aufgefasst werden. Die Stabilisierung bzw. Fortpflanzung der Helizität in Verbindungen der Formel Ia bzw. Ib beruht darauf, dass sich einerseits das für $\alpha$-Helices charakteristische $i \rightarrow i+4$ Wasserstoffbrücken-Netzwerk [vgl. L. Pauling, R.B. Corey, H.R. Branson, Proc. Natl. Acad. Sci. 37, 205 (1951)] ausbilden kann und dass andererseits der 2-Amino-1,2,3,4-tetrahydro-naphthalin-2-carbonsäure-Baustein am asymmetrischen C-Atom in 2-Stellung inhaerent $\alpha$-helicale Konformationen induziert, vgl. C. Spiegler, Synthesis and conformational studies of peptides containing novel $\alpha,\alpha$-disubstituted amino acids, Dissertation Universität Zürich, 1993).

Wie aus Figur 1 hervorgeht, beträgt die in Trifluoraethanol/Wasser (1:1) ermittelte Helizität des Endprodukts von Beispiel 8.1.a 87%, diejenige des Endprodukts von Beispiel 8.1.b 0% und diejenige eines Referenz-Peptids gleicher Länge 50% [die Berechnung der Helizität wurde analog zu Y.-H. Chen, J.T. Wang, H.H. Martinez, Biochemistry 11, 4120 (1972) und Y.-H. Chen, J.T. Wang, K.H. Chau, Biochemistry 13, 3330 (1974) durchgeführt].

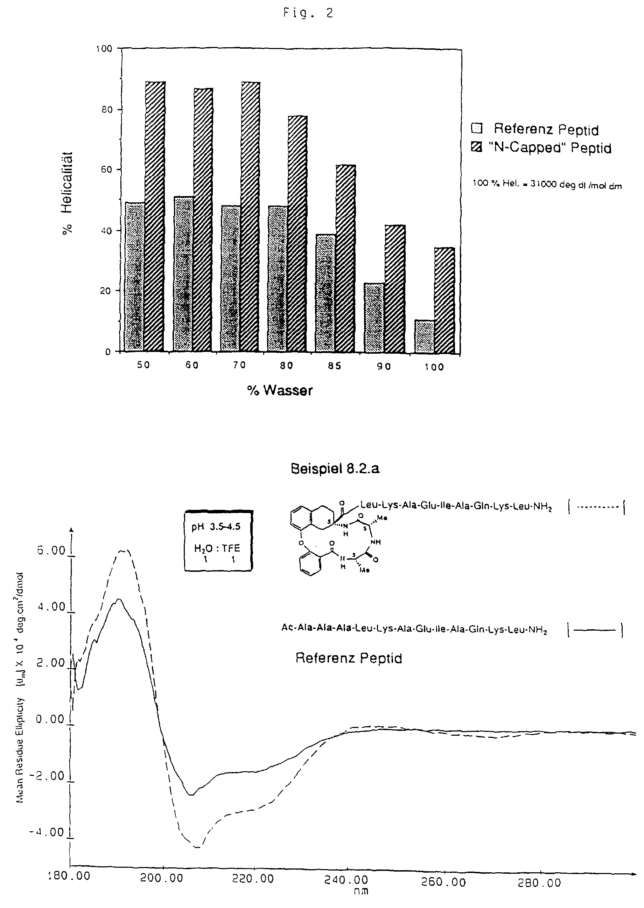

Die signifikante Stabilisierung der $\alpha$-helicalen Konformation kann auch in reinem Wasser beobachtet werden. Wie aus Figur 2 hervorgeht beträgt die Helizität für das Endprodukt von Beispiel 8.2.a 40%, für das Referenz-Peptid gleicher Länge jedoch nur etwa 10%.

Die Priorität der Aminosäurereste $A^1$ und $A^2$ bzw. $A^3$ und $A^4$ in den Verbindungen der Formel Ia bzw. Ib ist etwa analog zur Kompatibilität von L- und D-Aminosäuren in peptidischen $\alpha$-Helices, vgl. A. Horovitz, J.M. Matthews, A.R. Fersht, J. Mol. Biol. 227, 560-568 (1992).

Aufgrund ihrer Eigenschaften eignen sich Verbindungen der Formel Ia bzw. Ib als Mimetica von exponierten helicalen Domänen von Proteinen, um deren Rolle bezüglich der Wechselwirkungen mit anderen Proteinen (Rezeptoren, Enzyme o.dgl.) oder mit DNA bzw. RNA aufzuklären. Insbesondere können so mittels Verbindungen der Formel Ia bzw. Ib Aminosäuresequenzen mit biologischer Aktivität ermittelt werden. Die Verbindungen der Formel Ia bzw. Ib eignen sich deshalb als Forschungshilfsmittel ("Research Tools"), um biologisch aktive Peptidsequenzen bereitzustellen. Die Verbindungen der Formel Ia bzw. Ib sind aber auch potentiell als Arzneimittel geeignet, wobei der jeweilige therapeutische Verwendungszweck in erster Linie von der Natur, der Anzahl und der Reihenfolge der im Molekül vorhandenen Aminosäurereste abhängt.

In den nachfolgenden Beispielen, welche die Erfindung näher erläutern, ihren Umfang aber in keiner Weise einschränken sollen, sind sämtliche Temperaturen in Celsiusgraden angegeben. Die Bezeichnung der Beispiele, deren Endprodukte am asymmetrischen C-Atom in 2-Stellung des 2-Amino-1,2,3,4-tetrahydro-naphthalin-2-carbonsäure-Bausteins die S-Konfiguration aufweisen, endet stets mit "a", diejenige der Beispiele, deren Endprodukte am fraglichen C-Atom die R-Konfiguration aufweisen, endet durchwegs mit "b".

Beispiel 1.1

Zu einer Lösung von 120.0 g (750 mMol) 1,7-Dihydroxy-naphthalin in einem Gemisch von 300 ml Tetrahydrofuran, 142 ml tert.-Butylalkohol und 1000 ml flüssigem Ammoniak wurden unter Argon und Kühlen (Trockeneis/Aceton) analog zu D.W. Johnson, L.N. Mander, Aust. J. Chem. 1974, 27, 1277 innerhalb von 20 Minuten 15,0 g Lithiummetallstücke zugegeben. Das Gemisch wurde 1 Stunde gerührt und dann mit 400 ml Methanol versetzt, worauf 1 Stunde bei -70° weitergerührt, der Ammoniak unter $N_2$-Strom entfernt, der Rückstand mit wässriger 10%iger HCl angesäuert und mit 3 x 2.0 l Essigsäureethylester extrahiert wurde. Die organische Phase wurde zweimal mit 1,0 l gesättigter Kochsalzlösung gewaschen, über $MgSO_4$ getrocknet und eingedampft. Der Rückstand wurde in 500 ml Essigsäureäthylester und 40 ml Ethanol gelöst, worauf mit 1,0 l 38-40%iger Natriumhydrogensulfitlösung versetzt und das Gemisch während 18 Stunden geschüttelt wurde. Der Niederschlag wurde abfiltriert, mit verdünnter $NaHSO_3$-Lösung und Essigester gewaschen und am Vakuum über Sicapent getrocknet, wobei 96.2 g (48%) (rac)-2,8-Dihydroxy-1,2,3,4-tetrahydronaphthalin-2-sulfonsäure-Natriumsalz erhalten wurden; Smp. 160-162°.

Beispiel 1.2

Eine Suspension von 48.0 g (180 mMol) (rac)-2,8-Dihydroxy-1,2,3,4-tetrahydro-naphthalin-2-sulfonsäure-Natriumsalz, 20.2 g (310 mMol) Kaliumcyanid und 118.1 g (1 Mol) Ammoniumcarbonat in 800 ml Ethanol/Wasser (4:1) wurde während 2 Stunden bei 65° Innentemperatur gerührt, dann abgekühlt und auf ein Gemisch von Eis und 1.2 l 2N wässriger HCl gegossen. Die Suspension wurde über Nacht stehengelassen und dann filtriert. Der Rückstand wurde mit $H_2O$ gewaschen und am Vakuum über Sicapent getrocknet, wobei 31.5 g (75.4%) (rac)-3',4'-Dihydroxy-8'-hydroxyspiro[imidazolidin-4,2'(1'H)-naphthalin]-2,5-dion erhalten wurden; Smp. 232-234° (Zers.).

Beispiel 1.3

Eine Suspension von 30.0 g (129 mMol) (rac)-3',4'-Dihydroxy-8'-hydroxyspiro[imidazolidin-4',2'(1'H)-naphthalin]-2,5-dion und 203.8 g $Ba(OH)_2 \bullet 8H_2O$ in 800 ml Wasser wurde im Stahlautoklaven während 24 Stunden bei 125° gerührt, dann abgekühlt und mit 4N $H_2SO_4$-Lösung angesäuert, worauf während 1 Stunde auf dem Wasserbad erhitzt, abgekühlt, filtriert und der Filterrückstand mit verdünnter $H_2SO_4$-Lösung gewaschen wurde. Das saure Filtrat wurde bis auf ein Volumen von ca. 300 ml eingeengt und mit konzentrierter wässriger Ammoniaklösung neutralisiert, worauf ein Niederschlag ausfiel. Die Suspension wurde über Nacht stehengelassen und dann filtriert. Der Rückstand wurde am Vakuum über Sicapent getrocknet, wobei 20.5 g (83.1%) (rac)-2-Amino-8-hydroxy-1,2,3,4-tetrahydro-naphthalin-2-carbonsäure erhalten wurden; Smp. >280° (Zers.).

Beispiel 1.4

Zu einer Lösung von 19.0 g (99.4 mMol) (rac)-2-Amino-8-hydroxy-1,2,3,4-tetrahydro-naphthalin-2-carbonsäure in 210 ml 1N NaOH wurden unter Eiskühlung und Rühren gleichzeitig aus 2 Tropftrichtern 50 ml 2N NaOH und 40,3 ml (345 mMol) Benzoylchlorid so zugetropft, dass die Innentemperatur nicht über 15° anstieg. Das Reaktionsgemisch wurde anschliessend langsam auf Raumtemperatur gebracht, 2 Stunden gerührt, mit 2N HCl angesäuert und zweimal mit 250 ml Essigester extrahiert. Die vereinigten organischen Phasen wurden über $MgSO_4$ getrocknet und eingedampft. Der Rückstand wurde in 300 ml Dioxan gelöst, und die Lösung wurde unter Eiskühlung mit 150 ml 2N NaOH versetzt. Das Reaktionsgemisch wurde über Nacht gerührt, mit 4N HCl angesäuert und dreimal mit 300 ml Essigester extrahiert. Die vereinigten organischen Phasen wurden über $MgSO_4$ getrocknet und eingedampft. Der Rückstand wurde in 500 ml Diethylether/Hexan (4:1) aufgenommen, und die Suspension wurde über Nacht gerührt und dann filtriert. Der Filterrückstand wurde am Hochvakuum getrocknet, wobei 28.6 g (92.4%) (rac)-2-Benzamido-8-hydroxy-1,2,3,4-tetrahydro-naphthalin-2-carbonsäure erhalten wurden. Smp. 234-236° (Zers.).

Beispiel 1.5

Zu einer Suspension von 18.0 g (57.8 mMol) (rac)-2-Benzamido-8-hydroxy-1,2,3,4-tetrahydro-naphthalin-2-carbonsäure in 150 ml Dichlormethan wurden unter Kühlen portionenweise 12.53 g (60.7 mMol) N,N-Dicyclohexylcarbodiimid zugegeben. Das Gemisch wurde während 2 Stunden bei Raumtemperatur gerührt und dann filtriert, worauf der Filterrückstand mit Dichlormethan gewaschen wurde. Das Filtrat wurde eingedampft, und der Rückstand wurde am Hochvakuum getrocknet und in 150 ml absolutem Dioxan gelöst. Die Lösung wurde unter Eiskühlung und Argon portionenweise mit 2.80 g Natriumhydrid-Suspension (55%) versetzt, worauf 30 Minuten bei Raumtemperatur gerührt und dann 16.5 ml Dimethylsulfat zugegeben wurden. Die Suspension wurde 1 Stunde bei 80° gerührt, abgekühlt und dann auf eine Mischung von Eis, 10%iger Natriumhydrogenphosphat-Lösung und Essigester gegossen. Die wässrige Phase wurde mit Essigester extrahiert, und die vereinigten organischen Phasen wurden über $MgSO_4$ getrocknet und eingeengt. Der Rückstand wurde an 500 g Kieselgel mit Essigester/Hexan (1:4) chromatographiert, wobei nach Kristallisation aus Diethyläther/Hexan (1:2) und Trocknen am Hochvakuum 13.6 g (76.5%) (rac)-3,4-Dihydro-8-methoxy-2'-phenyl-spiro[naphthalin-2(1H),4'(5'H)-oxazol]-5'-on erhalten wurden. Smp. 108-109°.

Beispiel 1.6

Ein Gemisch von 12.6 g (41 mMol) (rac)-3,4-Dihydro-8-methoxy-2'-phenyl-spiro[naphthalin-2(1H),4'(5'H)-oxazol]-5'-on und 15.15 g (61.5 mMol) L-phenylalanin-cyclohexylamid in 120 ml N-Methylpyrrolidon wurde während

18 Stunden bei 60° gerührt, dann abgekühlt und anschliessend auf ein Gemisch von 300 ml Wasser und 500 ml Essigester gegossen. Die organische Phase wurde zweimal mit 250 ml 0.5N HCl extrahiert, und die vereinigten wässrigen Phasen wurden mit Essigester extrahiert. Die vereinigten organischen Phasen wurden mit gesättigter Kochsalzlösung gewaschen, über $MgSO_4$ getrocknet und eingedampft. Der Rückstand wurde während 2 Stunden in 300 ml Essigester suspendiert, worauf die Suspension filtriert und der Rückstand mit Essigester gewaschen, aus Essigester/Hexan (6:1) umkristallisiert und getrocknet wurde. Man erhielt 10.44 g (46%) $N^2$-[(S)-2-Benzamido-8-methoxy-1,2,3,4-tetrahydro-naphthalin-2-carbonyl]-L-phenylalanin-cyclohexylamid. Smp. 186-188°. $[\alpha]_D$ = +12.0° (c=0.2, Methanol).

Das Filtrat wurde eingeengt, und der Rückstand wurde an 1 kg Kieselgel mit Diethyläther/Isopropanol (99.5: 0.5) chromatographiert, worauf nach Umkristallisation aus Essigester/Hexan (1:1) und Trocknen am Hochvakuum 10.25 g (45.2%) $N^2$-[(R)-2-Benzamido-8-methoxy-1,2,3,4-tetrahydronaphthalin-2-carbonyl]-L-phenylalanin-cyclohexylamid erhalten wurden. Smp. 191-192°. $[\alpha]_D$ = -21.0° (c=0.2, Methanol).

Beispiel 1.7.a

Zu einer Suspension von 7.5 g (13.54 mMol) $N^2$-[(S)-2-Benzamido-8-methoxy-1,2,3,4-tetrahydro-naphthalin-2-carbonyl]-L-phenylalanin-cyclohexylamid in 35 ml Methanol wurden unter Argon und Eiskühlung 7.1 ml (80.74 mMol) Trifluormethansulfonsäure zugetropft. Das Gemisch wurde im Bombenrohr während 4 Stunden bei 80° gerührt, dann abgekühlt und anschliessend auf ein Volumen von ~20 ml eingeengt. Unter Rühren wurden 50 ml Dichlormethan zugegeben, die Suspension wurde filtriert, und der Filterrückstand wurde mit Dichlormethan gewaschen und getrocknet, wobei 4.48 g (83%) L-Phenylalanin-cyclohexylamid-trifluormethansulfonat isoliert wurden. Das Filtrat wurde mit Wasser gewaschen, über $MgSO_4$ getrocknet und eingeengt. Der Rückstand wurde an 350 g Kieselgel mit Essigester/Hexan (2:3) chromatographiert, worauf 4.0 g (87%) (S)-2-Benzamido-8-methoxy-1,2,3,4-tetrahydro-naphthalin-2-carbonsäuremethylester erhalten wurden. $[\alpha]_D$ = +144,0° ($CHCl_3$, c=0.2). IR(KBr): 3366w(br.), 3062w, 2998w, 2948w, 1739s, 1647s, 1586m, 1527s, 1468s, 1437m, 1292m, 1259s, 1099m, 1047m, 774w, 713m. (D. Obrecht, Helv. Chim. Acta 1992, 75, 1666).

Beispiel 1.7.b

In Analogie zu Beispiel 1.7.a wurden aus 8.79 g (15.87 mMol) $N^2$-[(R)-2-Benzamido-8-methoxy-1,2,3,4-tetrahydro-naphthalin-2-carbonyl]-L-phenylalanin-cyclohexylamid 4.78 g (88%) (R)-2-Benzamido-8-methoxy-1,2,3,4-tetrahydro-naphthalin-2-carbonsäure-methylester erhalten. $[\alpha]_D$ = -142.5° (c=0.2, $CHCl_3$).

Beispiel 1.8.a

Zu einer Lösung von 1.95 g (5.75 mMol) (S)-2-Benzamido-8-methoxy-1,2,3,4-tetrahydro-naphthalin-2-carbonsäure-methylester in 15 ml Dichlormethan wurden unter Argon und Eiskühlung 28.7 ml Bortribromid-Lösung (1M in Dichlormethan) zugetropft. Das Reaktionsgemisch wurde 30 Minuten bei 0° und 3 Stunden bei Raumtemperatur gerührt und dann auf eine Mischung von Eis, gesättigter Ammoniumchlorid-Lösung und Essigester gegossen. Die organische Phase wurde abgetrennt, mit gesättigter Kochsalzlösung extrahiert, über $MgSO_4$ getrocknet und eingeengt. Der Rückstand wurde am Hochvakuum getrocknet, in Diethylether/Hexan (1:4) suspendiert und abfiltriert. Der Filterrückstand wurde getrocknet, wobei 1.75 g (97.8%) (S)-2-Benzamido-8-hydroxy-1,2,3,4-tetrahydro-naphthalin-2-carbonsäure erhalten wurden. $[\alpha]_D$ = +84.3° (c=0.3, Methanol). IR(KBr): 3362m(br.), 3064w, 3022w, 2976w, 2938w, 2620w, 1718s, 1644s, 1588m, 1523s, 1487m, 1467s, 1330m, 1278s, 1082w, 716m.

Beispiel 1.8.b

In Analogie zu Beispiel 1.8.a wurden aus 2.71 g (7.98 mMol) (R)-2-Benzamido-8-methoxy-1,2,3,4-tetrahydro-naphthalin-2-carbonsäure-methylester 2.42 g (97.4%) (R)-2-Benzamido-8-hydroxy-1,2,3,4-tetrahydro-naphthalin-2-carbonsäure erhalten. $[\alpha]_D$ = -85.7° (c=0.3, Methanol).

Beispiel 2.1.a

Eine Lösung von 1.75 g (5.62 mMol) (S)-2-Benzamido-8-hydroxy-1,2,3,4-tetrahydro-naphthalin-2-carbonsäure in 12 ml 25%iger Salzsäure und 6 ml Dioxan wurde im Bombenrohr 8 Stunden auf 110° erhitzt, dann abgekühlt und anschliessend zur Trockene eingedampft. Der Rückstand wurde über Nacht am Hochvakuum über Sicapent getrocknet und dann unter Rühren in 20 ml Diäthyläther suspendiert, worauf die Suspension filtriert wurde. Der Filterrückstand wurde mit Diethylether gewaschen und am Hochvakuum getrocknet und dann in 3 ml Methanol und 3 ml 15%iger

methanolischer Salzsäure gelöst. Die Lösung wurde unter Argon und Eiskühlung mit 0.42 ml (3.89 mMol) Oxalylchlorid versetzt, worauf das Gemisch im Bombenrohr 20 Stunden bei 50° gerührt, dann abgekühlt und anschliessend auf eine Mischung von gesättigter Natriumhydrogencarbonat-Lösung und Chloroform gegossen wurde. Die wässrige Phase wurde mit Chloroform/Methanol (6:1) extrahiert. Die vereinigten organischen Phasen wurden über $MgSO_4$ getrocknet, filtriert und eingeengt. Der Rückstand wurde an 150 g Kieselgel mit Chloroform/Methanol (6:1) chromatographiert, wobei nach Umkristallisation aus Essigester/Hexan 1.05 g (84.4%) (S)-2-Amino-8-hydroxy-1,2,3,4-tetrahydro-naphthalin-2-carbonsäure-methylester erhalten wurden. Smp. 183-185°. $[\alpha]_D$ = -22.7° (c=0.15, Methanol).

Beispiel 2.1.b

In Analogie zu Beispiel 2.1.a wurden aus 2.40 g (7.71 mMol) (R)-2-Benzamido-8-hydroxy-1,2,3,4-tetrahydro-naphthalin-2-carbonsäure 1.60 g (95.3%) (R)-2-Amino-8-hydroxy-1,2,3,4-tetrahydro-naphthalin-2-carbonsäuremethylester erhalten. Smp. 184-185°.

Beispiel 2.2.a

Eine Suspension von 9.2 g (29.55 mMol) (S)-2-Benzamido-8-hydroxy-1,2,3,4-tetrahydro-naphthalin-2-carbonsäure in 45 ml Dioxan und 62 ml 25%iger Salzsäure wurde im Bombenrohr 24 Stunden auf 100° erhitzt, dann abgekühlt, auf die Hälfte des Volumens eingeengt und mit Essigester extrahiert. Die organische Phase wurde mit Wasser gewaschen, worauf die vereinigten wässrigen Phasen auf ein Volumen von 200 ml reduziert und mit konzentrierter Ammoniaklösung auf pH 7 gestellt wurden. Die erhaltene Lösung wurde über MCI-Gel (CHP20P; 75-150 μ) filtriert (Wasser, Wasser/Methanol (95:5) → Wasser/Methanol (9:1)), worauf nach Trocknen über Sicapent 5.30 g (86.6%) (S)-2-Amino-8-hydroxy-1,2,3,4-tetrahydronaphthalin-2-carbonsäure erhalten wurden. Smp. >270° (Zers.). $[\alpha]_D$ = +7.0° (c=0.2, 0,1N HCl).

Beispiel 2.2.b

In Analogie zu Beispiel 2.2.a wurden aus 4.50 g (14.45 mMol) (R)-2-Benzamido-8-hydroxy-1,2,3,4-tetrahydro-naphthalin-2-carbonsäure 2.49 g (83%) (R)-2-Amino-8-hydroxy-1,2,3,4-tetrahydro-naphthalin-2-carbonsäure erhalten. Smp. >272° (Zers.). $[\alpha]_D$ = -5.0° (c=0.2, 0,1N HCl).

Beispiel 2.3.a

Zu einer Lösung von 780 mg (3.53 mMol) (S)-2-Amino-8-hydroxy-1,2,3,4-tetrahydro-naphthalin-2-carbonsäure-methylester in 10 ml DMF wurde unter Eiskühlung und Argon eine Lösung von 924 mg (4.24 mMol) Di-tert.Butyldicarbonat in 1.5 ml DMF zugegeben. Das Reaktionsgemisch wurde 30 Minuten bei 0° und 18 Stunden bei Raumtemperatur gerührt und dann auf eine Mischung von Wasser und Essigester gegossen. Die organische Phase wurde abgetrennt, mit Wasser extrahiert, über $MgSO_4$ getrocknet und eingeengt. Der Rückstand wurde an 100 g Kieselgel mit Essigester/Hexan (1:2) chromatographiert, worauf nach Umkristallisation aus Diethylether/Hexan und Trocknen 1.0 g (88.1%) (S)-2-(tert.Butoxycarbonylamino)-8-hydroxy-1,2,3,4-tetrahydro-naphthalin-2-carbonsäure-methylester erhalten wurden. Smp. 186-187°. $[\alpha]_D$ = +95.0° (c=0.2, Chloroform).

Beispiel 2.3.b

In Analogie zu Beispiel 2.3.a wurden aus 1.46 g (6.60 mMol) (R)-2-Amino-8-hydroxy-1,2,3,4-tetrahydro-naphthalin-2-carbonsäure-methylester 1.80 g (85%) (R)-2-(tert.Butoxycarbonylamino)-8-hydroxy-1,2,3,4-tetrahydronaphthalin-2-carbonsäure-methylester erhalten. Smp. 186-187°. $[\alpha]_D$ = -93.5° (c=0.2, Chloroform).

Beispiel 2.4.a

Eine Suspension von 1.1 g (4.27 mMol) (S)-2-Amino-8-hydroxy-1,2,3,4-tetrahydro-naphthalin-2-carbonsäure-methylester in 22 ml Dioxan wurde bei Raumtemperatur mit 2.12 g (8.54 mMol) N-(Benzyloxycarbonyloxy)-succinimid und 717 mg (8.54 mMol) Natriumhydrogencarbonat versetzt. Das Reaktionsgemisch wurde 4 Stunden bei Raumtemperatur gerührt und dann auf eine Mischung von gesättigter Natriumchlorid-Lösung und Essigester gegossen. Die organische Phase wurde abgetrennt, über $MgSO_4$ getrocknet und eingeengt. Der Rückstand wurde an 200 g Kieselgel mit Chloroform/Methanol (98:2→95:5) chromatographiert, worauf nach Trocknen 1.30 g (86.9%) (S)-2-Benzyloxycarbonylamino-8-hydroxy-1,2,3,4-tetrahydronaphthalin-2-carbonsäure-methylester erhalten wurden. Smp. 58-68° (Sintern). $[\alpha]_D$ = +85.0° (c=0.2, Chloroform).

Beispiel 2.5.a

Eine Lösung von 800 mg (2.26 mMol) (S)-2-Benzyloxycarbonylamino-8-hydroxy-1,2,3,4-tetrahydro-naphthalin-2-carbonsäure-methylester in 10 ml THF/Methanol/Wasser (3:1:1) wurde unter Eiskühlung mit 695 mg Lithiumhydroxid•1 $H_2O$ versetzt. Das Reaktionsgemisch wurde 1 Stunde bei 0° und 3 Stunden bei Raumtemperatur gerührt und dann auf eine Mischung von Eis, 0.5N Salzsäurelösung und Chloroform gegossen. Die Wasserphase wurde abgetrennt und mit Choroform erschöpfend extrahiert. Die vereinigten organischen Fraktionen wurden über Natriumsulfat getrocknet und eingeengt. Nach Trocknen des Rückstands erhielt man 555 mg (72%) (S)-2-Benzyloxycarbonyl-8-hydroxy-1,2,3,4-tetrahydro-naphthalin-2-carbonsäure als beiges Pulver. Smp. 68-75° (Sintern). $[\alpha]_D$ = +83.5° (c= 0.2, Chloroform).

Beispiel 2.6.a

Zu einer Suspension von 100 mg (0.48 mMol) (S)-2-Amino-8-hydroxy-1,2,3,4-tetrahydro-naphthalin-2-carbonsäure in 2 ml Dioxan/Wasser (1:1) wurden 143 mg (0.576 mMol) N-(Benzyloxycarbonyl)-succinimid und 80.6 mg (0.96 mMol) Natriumhydrogencarbonat zugegeben. Das Reaktionsgemisch wurde über Nacht bei Raumtemperatur gerührt, danach nochmals mit 40 mg (0.336 mMol) Natriumhydrogencarbonat und 83.6 mg (0.48 mMol) N-(Benzyloxycarbonyl)-succinimid versetzt, 4 Stunden bei Raumtemperatur gerührt und dann auf eine Mischung von gesättigter Natriumchlorid-Lösung und Essigester gegossen. Die organische Phase wurde abgetrennt, über $MgSO_4$ getrocknet und eingeengt. Der Rückstand wurde an 20 g Kieselgel mit Chloroform/Methanol/Wasser (6:3:0.5) chromatographiert, worauf nach Trocknen 66 mg (38.7%) (S)-2-Benzyloxycarbonylamino-8-hydroxy-1,2,3,4-tetrahydro-naphthalin-2-carbonsäure als beiges Pulver erhalten wurden. Smp. 65-75° (Sintern).

Beispiel 2.7.a

Eine Lösung von 620 mg (1.82 mMol) (S)-Benzyloxycarbonylamino-8-hydroxy-1,2,3,4-tetrahydro-naphthalin-2-carbonsäure in 6 ml N,N-Dimethylformamid wurde bei 0° mit 521 mg (2.73 mMol) N-Ethyl-N'-(3-dimethylaminopropyl)-carbodiimid-hydrochlorid, 489 mg (3.64 mMol) 1-N-Hydroxylbenzotriazol, 502 µl (2.91 mMol) Aethyldiisopropylamin und 627 µl (4.37 mMol) Trimethylsilyläthanol versetzt. Das Reaktionsgemisch wurde 1 Stunde bei 0° gerührt, dann mit 50 mg N,N-Dimethylaminopyridin und weiteren 627 µl (4.37 mMol) Trimethylsilyläthanol versetzt, hierauf 24 Stunden bei Raumtemperatur gerührt und anschliessend auf eine Mischung von Wasser und Dichlormethan gegossen. Die wässrige Phase wurde mit 0.1N Salzsäure angesäuert und mit Dichlormethan erschöpfend extrahiert. Die vereinigten organischen Phasen wurden über $MgSO_4$ getrocknet und eingeengt. Der Rückstand wurde an 100 g Kieselgel mit Hexan/Essigester (4:1→1:1) chromatographiert, worauf nach Trocknen 449 mg (56%) (S)-2-Benzyloxycarbonylamino-8-hydroxy-1,2,3,4-tetrahydronaphthalin-2-carbonsäure-trimethylsilyläthylester erhalten wurden. Smp. 141-144°. $[\alpha]_D$ = +68.0° (c=0.2, Chloroform).

Beispiel 2.8.a

Zu einer Suspension von 513 mg (2.68 mMol) (S)-2-Amino-8-hydroxy-1,2,3,4-tetrahydro-naphthalin-2-carbonsäure in 10 ml Dichlormethan wurden unter Argon und Eiskühlung 1.2 ml (9.50 mMol) Trimethylchlorsilan zugegeben. Das Reaktionsgemisch wurde 30 Minuten am Rückfluss erhitzt, abgekühlt, bei 0° mit 1.7 ml (9.92 mMol) Ethyldiisopropylamin versetzt, anschliessend 15 Minuten bei Raumtemperatur und 2 Stunden unter Rückfluss gerührt, dann auf 0° gekühlt und schliesslich mit einer Lösung von 650 mg (2.98 mMol) Di-tert.butyl-dicarbonat in 0.6 ml Dichlormethan versetzt. Das Reaktionsgemisch wurde 40 Stunden bei Raumtemperatur und 7 Stunden unter Rückfluss gerührt, dann abgekühlt und anschliessend auf eine Mischung von 40 ml gesättigter Natriumhydrogencarbonat-Lösung und Diethylether gegossen. Die organische Phase wurde abgetrennt und zweimal mit 30 ml gesättigter Natriumhydrogencarbonat-Lösung extrahiert. Die vereinigten Wasserphasen wurden unter Eiskühlung auf pH 4 gestellt und dreimal mit 100 ml Essigester extrahiert. Die vereinigten organischen Phasen wurden über $Na_2SO_4$ getrocknet und eingeengt, worauf nach Trocknen am Hochvakuum 820 mg (99.6%) (S)-2-(tert.Butoxycarbonylamino)-8-hydroxy-1,2,3,4-tetrahydro-naphthalin-2-carbonsäure als amorpher Feststoff erhalten wurden. $[\alpha]_D$ = +66.0° (c=1.0, Chloroform). IR(KBr): 3403m(br.), 3033w, 2979w, 2933w, 1717s, 1695s, 1589w, 1500w, 1467m, 1395m, 1368m, 1278m, 1164m, 1063w, 776w.

Beispiel 2.9.b

Eine Lösung von 465 mg (1.45 mMol) (R)-2-(tert.Butoxycarbonylamino)-8-hydroxy-1,2,3,4-tetrahydro-naphthalin-2-carbonsäure-methylester in 4.5 ml Tetrahydrofuran/Methanol/Wasser (3:1:1) wurde unter Eiskühlung mit 647

mg Lithiumhydroxid•1$H_2O$ versetzt. Das Reaktionsgemisch wurde 30 Minuten bei 0° und 2 Stunden bei Raumtemperatur gerührt und dann auf eine Mischung von Chloroform/Methanol (6:1) und 0.5N Salzsäure/Eis gegossen. Die organische Phase wurde abgetrennt, über $MgSO_4$ getrocknet und eingeengt, worauf nach Trocknen am Hochvakuum 410 mg (92%) (R)-2-(tert.Butoxycarbonylamino)-8-hydroxy-1,2,3,4-tetrahydro-naphthalin-2-carbonsäure als amorpher Feststoff erhalten wurden. $[\alpha]_D$ = -65.0° (c=0.5, Chloroform).

Beispiel 2.10.a

Eine Suspension von 770 mg (250 mMol) (S)-2-(tert.Butoxycarbonylamino)-8-hydroxy-1,2,3,4-tetrahydro-naphthalin-2-carbonsäure in 5 ml Toluol wurde mit 1.80 ml (7.52 mMol) N,N-Dimethylformamid-di-tert.butylacetal versetzt. Das Reaktionsgemisch wurde während 6 Stunden bei 70° gerührt und dann abgekühlt, worauf nochmals 0.6 ml (2.50 mMol) N,N-Dimethylformamid-di-tert.butylacetal zugegeben und 3 Stunden bei 70° gerührt wurde. Das Reaktionsgemisch wurde auf eine Mischung von Eiswasser und Essigester gegossen, worauf gesättigte Kochsalzlösung zugegeben wurde. Die Wasserphase wurde abgetrennt und mit Essigester extrahiert. Die vereinigten organischen Phasen wurden über $Na_2SO_4$ getrocknet und eingeengt. Der Rückstand wurde an 170 g Kieselgel mit Essigester/Hexan (1:4) chromatographiert, worauf nach Trocknen 665 mg (73%) (S)-2-(tert.Butoxycarbonylamino)-8-hydroxy-1,2,3,4-tetrahydronaphthalin-2-carbonsäure-tert.butylester als amorpher Feststoff erhalten wurden. $[\alpha]_D$ = +65.5° (c=0.2, Chloroform). IR(KBr): 3406m(br.), 2978m, 2932w, 1721s, 1695s, 1590m, 1497m, 1467m, 1394m, 1368s, 1303m, 1254m, 1162s, 1086m, 784w.

Beispiel 2.11.a

Zu einer Lösung von 800 mg (2.60 mMol) (S)-2-(tert.Butoxycarbonylamino)-8-hydroxy-1,2,3,4-tetrahydro-naphthalin-2-carbonsäure in 6 ml N,N-Dimethylformamid wurden unter Eiskühlung 426 µl (2.86 mMol) 1,8-Diazabicyclo [5.4.2.0]undec-7-en und 340 µl (2.86 mMol) Benzylbromid zugegeben. Das Reaktionsgemisch wurde während 18 Stunden bei Raumtemperatur gerührt und dann auf Eiswasser gegossen, worauf mit Essigsäureäthylester erschöpfend extrahiert wurde. Die vereinigten organischen Phasen wurden mit gesättigter Kochsalzlösung gewaschen, über Magnesiumsulfat getrocknet und eingeengt. Der Rückstand wurde an 120 g Kieselgel mit Hexan/Essigsäureethylester (7:3) chromatographiert, worauf nach Trocknen am Hochvakuum 829 mg (80.5%) (S)-(tert.Butoxycarbonylamino)-8-hydroxy-1,2,3,4-tetrahydro-naphthalin-2-carbonsäure-benzylester als weisser Schaum erhalten wurden. Smp. 51-65° (Sintern). $[\alpha]_D$ = +64.5° (c=0.2, Chloroform).

Beispiel 2.12.b

Eine Lösung von 440 mg (1.43 mMol) (R)-2-(tert.Butoxycarbonylamino)-8-hydroxy-1,2,3,4-tetrahydro-naphthalin-2-carbonsäure in 8 ml Dichlormethan wurde mit 140 µl Phenyldiazomethan versetzt. Das Reaktionsgemisch wurde 1 Stunde bei Raumtemperatur gerührt und dann eingeengt. Der Rückstand wurde an 50 g Kieselgel mit Chloroform chromatographiert, worauf 384 mg (70%) (R)-2-(tert.Butoxycarbonylamino)-8-hydroxy-1,2,3,4-tetrahydro-naphthalin-2-carbonsäure-benzylester als amorpher Feststoff erhalten wurden. Smp. 56-60°. $[\alpha]_D$ = -62.7° (c=1, Chloroform).

Beispiel 3.1.a

Zu einer Lösung von 200 mg (0.62 mMol) (S)-2-(tert.Butoxycarbonylamino)-8-hydroxy-1,2,3,4-tetrahydro-naphthalin-2-carbonsäure-methylester in 4 ml Trifluoräthanol wurden unter Argon und Eiskühlung portionenweise 110 mg (0.62 mMol) N-Bromsuccinimid zugegeben. Das Reaktionsgemisch wurde je 1 Stunde bei 0° und bei Raumtemperatur gerührt und dann auf eine Mischung von gesättigter Natriumhydrogencarbonat-Lösung und Chloroform gegossen. Die organische Phase wurde abgetrennt, mit Kochsalzlösung gewaschen, über $MgSO_4$ getrocknet und eingeengt. Der Rückstand wurde an 60 g Kieselgel mit Essigester/Hexan (1:9→4:6) chromatographiert, worauf nach Trocknen am Hochvakuum 220 mg (88.6%) (S)-5-Brom-2-(tert.butoxycarbonylamino)-8-hydroxy-1,2,3,4-tetrahydronaphthalin-2-carbonsäure-methylester erhalten wurden. Smp. >180° (Zers.). MS: 400 ($M^{+\bullet}$, <1), 343(2), 284, 282 (100), 144(52), 57(96).

Beispiel 4.1.a

Eine Lösung von 1.0 g (311 mMol) (S)-2-(tert.Butoxycarbonylamino)-8-hydroxy-1,2,3,4-tetrahydro-naphthalin-2-carbonsäure-methylester in 9 ml Pyridin wurde unter Eiskühlung und Argon mit 180 mg Natriumhydrid-Dispersion (55%) versetzt. Das Gemisch wurde 30 Minuten bei 0° gerührt, mit 966 mg (3.73 mMol) Kupferbromid-dimethylsulfid-Komplex und mit 3.06 g (9.33 mMol) 2-Iodbenzoesäure-benzylester versetzt, auf Raumtemperatur gebracht, 2 Stunden bei 120° gerührt, abgekühlt und auf eine Mischung von Eis, 0.5N Salzsäure und Essigester gegossen. Die organische Phase wurde abgetrennt, mit 0.5N Salzsäure und gesättigter Natriumchloridlösung gewaschen, über $MgSO_4$ getrocknet und eingeengt. Der Rückstand wurde an 140 g Kieselgel mit Essigester/Hexan (1:3) chromatographiert, worauf nach Trocknen am Hochvakuum 1.47 g (87%) (S)-8-(2-Benzyloxycarbonyl-phenoxy)-2-(tert.butoxycarbonylamino)-1,2,3,4-tetrahydro-naphthalin-2-carbonsäure-methylester als amorpher Festkörper erhalten wurden. $[\alpha]_D$ = +47.0° (c=0.2, Chloroform). IR(KBr): 3392w(br.), 3065w, 3035w, 2976w, 2949w, 1740s, 1714s, 1604w, 1484m, 1455m, 1367w, 1295m, 1250s, 1165m, 1081m, 777w, 698w.

Beispiel 4.1.b

In Analogie zu Beispiel 4.1.a wurden aus 250 mg (0.78 mMol) (R)-2-(tert.Butoxycarbonylamino)-8-hydroxy-1,2,3,4-tetrahydro-naphthalin-2-carbonsäure-methylester in 2 ml Pyridin mit 40 mg Natriumhydrid-Dispersion, 226 mg (1.09 mMol) Kupferbromid-dimethylsulfid-Komplex und 510 mg (1.56 mMol) 2-Iodbenzoesäure-benzylester 350 mg (84.4%) (R)-8-(2-Benzyloxycarbonyl-phenoxy)-2-(tert.butoxycarbonylamino)-1,2,3,4-tetrahydro-naphthalin-2-carbonsäure-methylester als amorpher Festkörper erhalten. $[\alpha]_D$ = -53.0° (c=0.2, Chloroform).

Beispiel 4.2.a

In Analogie zu Beispiel 4.1.a wurden 500 mg (1.55 mMol) (S)-2-(tert.Butoxycarbonylamino)-8-hydroxy-1,2,3,4-tetrahydro-naphthalin-2-carbonsäure-methylester in 1.5 ml Pyridin mit 124 mg Natriumhydrid-Dispersion, 876 mg (4.26 mMol) Kupferbromid-dimethylsulfid-Komplex und 1.36 g (4.57 mMol) 3-Bromthiophen-2-carbonsäure-benzylester während 3 Stunden bei 130° umgesetzt. Nach Chromatographie an 450 g Kieselgel mit Essigester/Hexan (9:1→4:1) erhielt man nach Trocknen am Hochvakuum 388 mg (46%) (S)-3-(7-tert.Butoxycarbonylamino-7-methoxycarbonyl-5,6,7,8-tetrahydro-naphthalin-1-yloxy)-thiophen-2-carbonsäure-benzylester als amorphen Feststoff. $[\alpha]_D$ = +56.0° (c=0.2, Chloroform). IR(KBr): 3392w(br.), 3965w, 2950w, 1740m, 1709s, 1537m, 1459m, 1426m, 1394m, 1273m, 1218s, 1065m, 776w.

Beispiel 4.2.b

In Analogie zu Beispiel 4.1.a wurden 100 mg (0.311 mMol) (R)-2-(tert.Butoxycarbonylamino)-8-hydroxy-1,2,3,4-tetrahydro-naphthalin-2-carbonsäure-methylester in 0.5 ml Pyridin mit 25 mg Natriumhydrid-Dispersion, 178 mg (0.86 mMol) Kupferbromid-dimethylsulfid-Komplex und 273 mg (0.92 mMol) 3-Bromthiophen-2-carbonsäure-benzylester umgesetzt. Nach Chromatographie und Trocknen am Hochvakuum erhielt man 88 mg (52.6%) (R)-3-(7-tert.Butoxycarbonylamino-7-methoxycarbonyl-5,6,7,8-tetrahydro-naphthalin-1-yloxy)-thiophen-2-carbonsäure-benzylester als amorphen Festkörper. $[\alpha]_D$ = -49.0° (c=0.2, Chloroform). MS (FAB): 560.4 ($M^{+\bullet}$ + $Na^+$, 40), 538.4 ($M^{+\cdot}$ + $H^+$, 25), 438.4(100).

Beispiel 4.3.a

Analog zu Beispiel 4.1.a wurden 520 mg (1.43 mMol) (S)-2-(tert.Butoxycarbonylamino)-8-hydroxy-1,2,3,4-tetrahydro-naphthalin-2-carbonsäure-tert.butylester in 2 ml Pyridin mit 75 mg Natriumhydrid-Dispersion (55%), 413 mg (2.00 mMol) Kupferbromid-dimethylsulfid-Komplex und 825 mg (2.86 mMol) 2-Iodbenzoesäur-prop-2-enylester umgesetzt. Nach Chromatographie an 70 g Kieselgel mit Essigester/Hexan (1:4) und Trocknen erhielt man 645 mg (86.1%) (S)-2-(tert.Butoxycarbonylamino)-8-[2-(prop-2-enyloxycarbonyl)-phenoxy]-1,2,3,4-tetrahydro-naphthalin-2-carbonsäure-tert.butylester als amorphen Festkörper. $[\alpha]_D$ = +32.0° (c=0.1, Chloroform). IR(KBr): 3390w(br.), 3080w, 2977m, 2932w, 1735s, 1716s, 1604m, 1510m, 1484m, 1455m, 1366m, 1299m, 1249s, 1081m, 848m, 779m.

Beispiel 4.4.a

In Analogie zu Beispiel 4.1.a wurden 383 mg (1.19 mMol) (S)-2-(tert.Butoxycarbonylamino)-8-hydroxy-1,2,3,4-tetrahydro-naphthalin-2-carbonsäure-methylester in 1 ml Pyridin während 1 Stunde bei 110° mit 94 mg (1.87 mMol) Natriumhydrid-Dispersion (55%), 409 mg (1.99 mMol) Kupferbromid-dimethylsulfid-Komplex und 1.17 g (3.48 mMol) 2-Brom-5-nitrobenzoesäure-benzylester umgesetzt, worauf nach Chromatographie an 200 g Kieselgel mit Essigester/Hexan (1:4→3:7) und Trocknen am Hochvakuum 585 mg (85%) (S)-2-(tert.Butoxycarbonylamino)-8-(2-benzyloxycarbonyl-4-nitro-phenoxy)-1,2,3,4-tetrahydro-naphthalin-2-carbonsäuremethylester als amorpher Feststoff erhalten wurden. Smp. 70-80°. MS(ISP): 599.4 ($M^{+\bullet}$ + $Na^+$, 25), 594.4 ($M^{+\bullet}$ + $NH_4^+$ , 30), 577.4 ($M^{+\bullet}$ + $H^+$, 25), 474.4 (100). $[\alpha]_D$ = +52.7° (c=0.15, Chloroform).

Beispiel 4.5.a

In Analogie zu Beispiel 4.1.a wurden 280 mg (0.634 mMol) (S)-2-Benzyloxycarbonylamino-8-hydroxy-1,2,3,4-tetrahydro-naphthalin-2-carbonsäuretrimethylsilylethylester in 1 ml Pyridin während 1.25 Stunden bei 120° mit 34 mg (0.66 mMol) Natriumhydrid-Dispersion (55%), 208 mg (1.014 mMol) Kupferbromid-dimethylsulfid-Komplex und 474 mg (1.84 mMol) 2-Bromnicotinsäure-tert.butylester umgesetzt, worauf nach Aufarbeitung und Chromatographie an 18 g Kieselgel mit Essigester/Hexan (1:4) und Trocknen am Hochvakuum 344 mg (88%) (S)-3-(7-Benzyloxycarbonylamino-7-trimethylsilylaethoxycarbonyl-5,6,7,8-tetrahydro-naphthalin-1-yloxy)nicotinsäure-tert.butylester als amorpher Festkörper erhalten wurden. $[\alpha]_D$ = +56.0° (c=0.2, Chloroform). MS(FAB): 619.3 ($M^{+\cdot}$ + $H^+$, 60), 535.2 (10), 91.1 (100).

Beispiel 4.6.a

In Analogie zu Beispiel 4.1.a wurden 720 mg (1.80 mMol) (S)-(tert.Butoxycarbonylamino)-8-hydroxy-1,2,3,4-tetrahydro-naphthalin-2-carbonsäure-benzylester in 2.5 ml Pyridin mit 85 mg Natriumhydrid-Dispersion, 520 mg (2.49 mMol) Kupferbromid-dimethylsulfid-Komplex und 1.25 g (5.22 mMol) 2-Brom-nicotinsäure-prop-2-enylester umgesetzt. Nach Aufarbeitung und Chromatographie an 500 g Kieselgel mit Hexan/Essigsäureethylester (4:1) erhielt man nach Trocknen am Hochvakuum 853 mg (84%) (S)-2-(7-Benzyloxycarbonyl-7-tert.butoxycarbonylamino-5,6,7,8-tetrahydronaphthalin-1-yloxy)-nicotinsäure-prop-2-enylester. MS: 558 ($M^{+\bullet}$, 1), 441 (15), 323 (27), 91 (100), 57 (80), 41 (52).

Beispiel 4.7.a

In Analogie zu Beispiel 4.1.a wurden 270 mg (0.678 mMol) (S)-5-Brom-2-(tert.butoxycarbonylamino)-8-hydroxy-1,2,3,4-tetrahydro-naphthalin-2-carbonsäure-methylester, 34.5 mg Natriumhydrid-Dispersion (55%), 194 mg (0.943 mMol) Kupferbromid-dimethylsulfid-Komplex und 553 mg (1.68 mMol) 2-Jodbenzoesäure-benzylester in 1 ml Pyridin während 4 Stunden auf 120° erhitzt. Nach Aufarbeitung analog zu Beispiel 4.1.a wurde das Rohprodukt an 150 g Kieselgel mit Hexan/Essigsäureethylester (9:1→1:1) chromatographiert, worauf nach Trocknen am Hochvakuum 294.3 mg (71.6%) (S)-8-(2-Benzyloxycarbonyl-phenoxy)-5-brom-2-tert.butoxycarbonylamino-1,2,3,4-tetrahydro-naphthalin-2-carbonsäure-methylester als weisser amorpher Festkörper erhalten wurden. Smp. 66-74° (Sintern). $[\alpha]_D$ = +25.5° (c=0.2, Chloroform). MS(ISP): 680.3 ($M^{+\bullet}$ + $Na^+$, 50), 675.4 ($M^{+\bullet}$ + $NH_4^+$, 65), 658.3 ($M^{+\bullet}$ + $H^+$, 45), 558.2 (100).

Beispiel 4.8.a

In Analogie zu Beispiel 4.1.a wurden 2.5 g (7.78 mMol) (S)-2-(tert.Butoxycarbonylamino)-8-hydroxy-1,2,3,4-tetrahydronaphthalin-2-carbonsäure-methylester in 5 ml Pyridin mit 370 mg Natriumhydrid-Dispersion, 2.23 g (10.9 mMol) Kupferbromid-dimethylsulfid-Komplex und 4.48 g (15.56 mMol) 2-Iodbenzoesäure-prop-2-enylester während 2 Stunden bei 130° umgesetzt. Nach Chromatographie an 500 g Kieselgel mit Essigester/Hexan (1.9→1:1) erhielt man 2.98 g (80%) (S)-2-(tert.Butoxycarbonylamino)-8-[2-(prop-2-enyloxycarbonyl)-phenoxy]-1,2,3,4-tetrahydronaphthalin-2-carbonsäure-methylester als zähes Oel. $[\alpha]_D$ = +57.0° (c = 0.4, Chloroform). IR (Film): 3386w(br.), 3075w, 2977m, 2950w, 1738s, 1717s, 1604m, 1581m, 1511m, 1484m, 1455m, 1366m, 1247s(br.), 1166m, 777m.

Beispiel 5.1.a

Zu einer Lösung von 591 mg (1.13 mMol) (S)-2-(tert.Butoxycarbonylamino)-8-[2-(prop-2-enyloxycarbonyl)-phenoxy]-1,2,3,4-tetrahydronaphthalin-2-carbonsäure-tert.butylester in 6 ml Dichlormethan wurden unter Eiskühlung und Argon 6 ml Trifluoressigsäure zugegeben. Das Reaktionsgemisch wurde 3 Stunden bei 0° gerührt, worauf das Lösungsmittel abdestilliert wurde. Der Rückstand wurde am Hochvakuum getrocknet und in 4 ml Chloroform gelöst, wonach bei 0° 0.36 ml (2.82 mMol) Trimethylchlorsilan zugegeben wurden. Das Reaktionsgemisch wurde 15 Minuten bei Raumtemperatur gerührt, mit 0.62 ml (4.13 mMol) Aethyldiisopropylamin versetzt, 1 Stunde bei 50° gerührt, abgekühlt, mit 419 mg (1.24 mMol) Fluoren-9-ylmethyl-succinimidyl-carbonat versetzt, während 18 Stunden bei Raumtemperatur und während 40 Stunden bei 50° gerührt, abgekühlt und auf eine Mischung von Essigester und 1M $NaH_2PO_4$-Lösung gegossen. Die organische Phase wurde abgetrennt, mit gesättigter Kochsalzlösung gewaschen, über $MgSO_4$ getrocknet und eingeengt, worauf der Rückstand am Hochvakuum getrocknet und in 6 ml Essigester gelöst wurde. Die Lösung wurde mit 228 mg (1.24 mMol) Pentafluorphenol versetzt, wonach unter Eiskühlung 0.192 ml (1.24 mMol) N,N-Diisopropylcarbodiimid zugegeben wurden. Das Reaktionsgemisch wurde über Nacht bei Raumtemperatur gerührt und dann auf ein Gemisch von Wasser und Dichlormethan gegossen. Die organische Phase wurde abgetrennt, über $MgSO_4$ getrocknet und eingeengt. DerRückstand wurde an 300 g Kieselgel mit Diethylether/Hexan

(1:4) chromatographiert, worauf nach Trocknen am Hochvakuum 450 mg (46%) (S)-2-(Fluoren-9-ylmethoxycarbonyl)-8-[2(prop-2-enyloxycarbonyl)-phenoxy]-1,2,3,4-tetrahydro-naphthalin-2-carbonsäurepentafluorphenylester als amorpher Festkörper erhalten wurden. $[\alpha]_D$ = -16.0° (c=0.1, Chloroform). IR(KBr): 3371w(br.), 3075w, 2949w, 1788m, 1716s, 1521s, 1541m, 1268m, 1242s, 1165w, 1135w, 1078m, 1047m, 1000s, 740w.

Beispiel 6.1.a

Ein Gemisch von 80 mg (0.13 mMol) (S)-8-(2-Benzyloxycarbonylphenoxy)-5-bromo-2-(tert.butoxycarbonylamino)-1,2,3,4-tetrahydronaphthalin-2-carbonsäure-methylester, 43 mg (0.143 mMol) 3,4-Dimethoxyphenyl-trimethylstannan und 6.9 mg (6 µMol) Tetrakis-(triphenylphosphin)-palladium in 0.4 ml Dioxan wurde während 18 Stunden bei 80° gerührt, abgekühlt, mit weiteren 8.6 mg (30 µMol) 3,4-Dimethoxyphenyl-trimethylstannan versetzt und nochmals während 4 Stunden bei 80° gerührt, erneut abgekühlt und über Celit filtriert. Der Filterrückstand wurde mit Dioxan gewaschen, und das Filtrat wurde eingeengt, worauf der Rückstand in Essigsäureäthylester aufgenommen wurde. Die erhaltene Lösung wurde mit gesättigter Kochsalzlösung gewaschen, über Magnesiumsulfat getrocknet und eingeengt. Der Rückstand wurde an 50 g Kieselgel mit Hexan/Essigsäureäthylester (9:1→1:1) chromatographiert, worauf nach Trocknen am Hochvakuum 34.0 mg (39.2%) (S)-8-(2-Benzyloxycarbonyl-phenoxy)-5-(3,4-dimethoxyphenyl)-2-(tert. butoxycarbonylamino)-1,2,3,4-tetrahydro-naphthalin-2-carbonsäure-methylester als beiges Pulver erhalten wurden. Smp. 64-74° (Sintern). $[\alpha]_D$ = -13.0° (c=0.2, Chloroform). MS(FAB): 668.4 ($M^{+\bullet}$ + $H^+$, 5), 568.3 (40), 217.0 (55), 109.1 (35), 91.2(100).

Beispiel 6.2.a

Ein Gemisch von 90.0 mg (0.147 mMol) (S)-8-(2-Benzyloxycarbonylphenoxy)-5-brom-2-(tert.butoxycarbonylamino)-1,2,3,4-tetrahydro-naphthalin-2-carbonsäure-methylester, 19.2 mg (0.295 mMol) Kaliumcyanid, 6.5 mg (11.8 µMol) 1,1'-Bis-(diphenylphosphino)-ferrocen und 3.0 mg (2.9 µMol) bis-(Dibenzylidenaceton)-di-palladium ($Pd_2dba_3$) in 0.2 ml N,N-Dimethylacetamid wurde während 15 Stunden unter Argon bei 80° gerührt, abgekühlt und über Celit filtriert, worauf das Filtrat eingedampft wurde. Der Rückstand wurde an 30 g Kieselgel mit Hexan/Essigsäureethylester (9:1→1:1) chromatographiert, wobei zunächst 71 mg (78.9%) Ausgangsmaterial zurückgewonnen wurden; anschliessend wurden ca. 7.0 mg (8.6%) roher (S)-8-(2-Benzyloxycarbonyl-phenoxy)-2-(tert.butoxycarbonylamino)-5-cyano-1,2,3,4-tetrahydro-naphthalin-2-carbonsäure-methylester erhalten, welcher mittels präparativer HPLC-Chromatographie (Säule: Vydac 250-10 RP-18; Programm: 60% Acetonitril → 100% Acetonitril in 20 Minuten) gereinigt wurde. Es wurden 5.5 mg (6.7% bezogen auf eingesetztes Ausgangsprodukt; 31.6% bezogen auf umgesetztes Ausgangsprodukt) reines Produkt erhalten. MS: 556 ($M^{+\bullet}$, <1), 439 (10), 397 (20), 91 (100), 57 (24).

Beispiel 7.1.1.a

Zu einer Lösung von 540 mg (1.02 mMol) (S)-8-(2-Benzyloxycarbonyl-phenoxy)-2-tert.butoxycarbonylamino-1,2,3,4-tetrahydro-naphthalin-2-carbonsäure-methylester in 2 ml Dichlormethan wurden unter Argon und Eiskühlung 2 ml Trifluoressigsäure zugetropft. Das Reaktionsgemisch wurde 1.5 Stunden bei 0° gerührt, worauf das Lösungsmittel am Hochvakuum abdestilliert wurde. Der Rückstand wurde gut getrocknet und mit 290 mg (1.53 mMol) L-Boc-Alanin, 293 mg (1.53 mMol) N-Aethyl-N'-(3-dimethylaminopropyl)-carbodiimid-hydrochlorid und 310 mg (2.30 mMol) 1-Hydroxybenzotriazol versetzt. Das Gemisch wurde in 3 ml DMF gelöst, und die Lösung wurde unter Eiskühlung und Argon mit 0.52 ml (3.06 mMol) Aethyldiisopropylamin versetzt, 30 Minuten bei 0° und 18 Stunden bei Raumtemperatur gerührt und dann auf eine Mischung von Eiswasser und Essigester gegossen. Die organische Phase wurde abgetrennt, mit Wasser, gesättigter Natriumhydrogencarbonatlösung und Kochsalzlösung gewaschen, über $MgSO_4$ getrocknet und eingedampft. Der Rückstand wurde an 60 g Kieselgel mit Essigester/Hexan (2:3) chromatographiert, worauf 570 mg (92.7%) (S)-8-(2-Benzyloxycarbonyl-phenoxy)-2-[N-(tert.butoxycarbonyl-L-alanyl)]-1,2,3,4-tetrahydro-naphthalin-2-carbonsäuremethylester als amorpher Festkörper erhalten wurden. $[\alpha]_D$ = +24.0° (c=0.1, Chloroform). MS(FAB): 603 ($M^{+\bullet}$ + $H^+$, 25), 503 (100), 432 (20), 414 (40), 372 (20), 324 (40), 264 (50), 247 (40).

Beispiel 7.1.1.b

In Analogie zu Beispiel 7.1.1.a wurden aus 500 mg (0.94 mMol) (R)-8-(2-Benzyloxycarbonyl-phenoxy)-2-tert. butoxycarbonylamino-1,2,3,4-tetrahydronaphthalin-2-carbonsäure-methylester 450 mg (79.4%) (R)-8-(2-Benzyloxycarbonyl-phenoxy)-2-[N-(tert.butoxycarbonyl-D-alanyl)]-1,2,3,4-tetrahydronaphthalin-2-carbonsäure-methylester als amorpher Festkörper erhalten. $[\alpha]_D$ = -23.5 (c= 0.2, Chloroform). MS(FAB): 603 ($M^{+\bullet}$ + $H^+$, 25), 503 (100).

Beispiel 7.1.2.a

Zu einer Lösung von 550 mg (0.913 mMol) (S)-8-(2-Benzyloxycarbonylphenoxy)-2-[N-(tert.butoxycarbonylamino-L-alanyl)]-1,2,3,4-tetrahydronaphthalin-2-carbonsäure-methylester in 2 ml Dichlormethan wurden unter Argon und Eiskühlung 2 ml Trifluoressigsäure zugegeben. Das Reaktionsgemisch wurde 1 Stunde bei 0° gerührt, worauf das Lösungsmittel am Hochvakuum entfernt wurde. Der Rückstand wurde getrocknet, mit 260 mg (1.37 mMol) Boc-L-Alanin, 263 mg (1.37 mMol) N-Aethyl-N'-(3-dimethylaminopropyl)carbodiimid-hydrochlorid und 370 mg 1-Hydroxybenzotriazol versetzt, worauf das Gemisch in 3 ml N,N-Dimethylformamid gelöst wurde und die Lösung bei 0° mit 0,47 ml (2.74 mMol) N-Ethyldiisopropylamin versetzt wurde. Das Reaktionsgemisch wurde 30 Minuten bei 0° und 1 Stunde bei Raumtemperatur gerührt und dann auf eine Mischung von Eiswasser und Essigester gegossen. Die organische Phase wurde abgetrennt, mit Wasser, gesättigter Natriumhydrogencarbonatlösung und Kochsalzlösung gewaschen, über $MgSO_4$ getrocknet und eingeengt. Der Rückstand wurde an 50 g Kieselgel mit Essigester/Hexan (3:2) chromatographiert, worauf nach Trocknen am Hochvakuum 550 mg (89.4%) (S)-8-(2-Benzyloxycarbonyl-phenoxy)-2-[N-(tert. butoxycarbonyl-L-alanyl)-L-alanyl]-1,2,3,4-tetrahydro-naphthalin-2-carbonsäure-methylester als amorpher Festkörper erhalten wurden. $[\alpha]_D$ = +8.0° (c=0.1, Chloroform). MS(ISP): 696.2 ($M^{+\bullet}$ + $Na^+$, 50), 674.2 ($M^{+\bullet}$ + $H^+$, 100), 618 (30), 574.1 (65), 432.1 (75).

Beispiel 7.1.2.b

In Analogie zu Beispiel 7.1.2.a wurden 400 mg (0.66 mMol) (R)-8-(2-Benzyloxycarbonyl-phenoxy)-2-[N-(tert. butoxycarbonyl-D-alanyl)]-1,2,3,4-tetrahydro-naphthalin-2-carbonsäure-methylester mit Boc-D-Alanin umgesetzt. Nach Chromatographie an 50 g Kieselgel mit Essigester/Hexan (1:1) erhielt man 390 mg (87.6%) (R)-8-(2-Benzyloxycarbonyl-phenoxy)-2-[N-[(tert.butoxycarbonylamino-D-alanyl)-D-alanyl]]-1,2,3,4-tetrahydro-naphthalin-2-carbonsäure-methylester als amorphen Festkörper. $[\alpha]_D$ = -10.0° (c=0.2, Chloroform). MS(FAB): 674 ($M^{+\bullet}$ + H, 20), 574 (20), 422 (100), 414 (30), 372 (50), 342 (50), 324 (50), 282 (50), 264 (50), 247 (40).

Beispiel 7.1.3.a

Zu einer vorhydrierten Suspension von 150 mg Palladium/Kohle (10%) in 10 ml Trifluorethanol wurde bei Raumtemperatur eine Lösung von 505 mg (0.75 mMol) (S)-8-(2-Benzyloxycarbonyl-phenoxy)-2-[N-[(tert.butoxycarbonyl-L-alanyl)-L-alanyl]]-1,2,3,4-tetrahydro-naphthalin-2-carbonsäuremethylester in 2 ml Trifluoräthanol zugegeben. Das Gemisch wurde bei Atmosphärendruck während 4 Stunden hydriert und über Celite filtriert, worauf das Lösungsmittel abdestilliert wurde. Der Rückstand wurde am Hochvakuum getrocknet und in Dichlormethan gelöst, worauf die Lösung mit 207 mg (1.12 mMol) Pentafluorphenol versetzt wurde. Zum Reaktionsgemisch wurden unter Eiskühlung und Argon portionenweise 216 mg (1.12 mMol) N-Aethyl-N'-(3-dimethylaminopropyl)-carbodiimid-hydrochlorid zugegeben, worauf 30 Minuten bei 0° und 6 Stunden bei Raumtemperatur gerührt wurde. Das Reaktionsgemisch wurde auf eine Mischung von Eiswasser, 0.1N Salzsäurelösung und Essigester gegossen. Die organische Phase wurde abgetrennt, mit gesättigter Kochsalzlösung gewaschen, über $MgSO_4$ getrocknet und eingedampft. Der Rückstand wurde am Hochvakuum getrocknet und in 1.5 ml Dichlormethan gelöst, worauf die Lösung unter Argon und Eiskühlung mit 1.5 ml Trifluoressigsäure versetzt, 1,5 Stunden bei 0° gerührt und dann am Hochvakuum eingedampft wurde. Der Rückstand wurde getrocknet und in Diethylether/Hexan (1:3) suspendiert, worauf die Suspension 1 Stunde gerührt und filtriert wurde. Der Rückstand wurde mit Hexan gewaschen, worauf nach Trocknen am Hochvakuum 580 mg (100%) (S)-2-[N-(L-Alanyl-L-alanyl)]-1,2,3,4-tetrahydro-8-[2-(pentafluorphenoxycarbonyl)phenoxy]-naphthalin-2-carbonsäure-methylester-trifluoracetat als amorpher Festkörper erhalten wurden. MS(ISP): 672 ($M^{+\bullet}$ + $Na^+$, 25), 650 ($M^{+\bullet}$ + $H^+$, 100), 507.8 (90), 448 (30), 406 (25).

Beispiel 7.1.3.b

In Analogie zu Beispiel 7.1.3.a wurden aus 120 mg (0.206 mMol) (R)-8-[2-(Benzyloxycarbonyl-phenoxy)]-2-[N-[(tert.butoxycarbonyl-D-alanyl)-D-alanyl]]-1,2,3,4-tetrahydro-2-naphthalincarbonsäure-methylester 155 mg (98.5%) (R)-2-[N-(D-Alanyl-D-alanyl)]-1,2,3,4-tetrahydro-8-[2-(pentafluorphenoxycarbonyl)phenoxy]-2-naphthalincarbonsäure-methylester-trifluoracetat erhalten. MS(FAB): 650 ($M^{+\bullet}$ + H, 100), 508 (30), 448 (20), 324 (20), 307 (60), 264 (50), 247 (50), 160 (40), 143 (40), 115 (70).

Beispiel 7.1.4.a

Zu einem Gemisch von 1 l Dioxan und 10 ml Pyridin wurde innerhalb von 17 Stunden bei 80° und unter Argon eine Lösung von 573 mg (0.75 mmol) (S)-2-[N-(L-Alanyl-L-alanyl)]-1,2,3,4-tetrahydro-8-[2-(pentafluorophenoxycarbonyl)phenoxy]-2-naphthalin-carbonsäuremethylester-trifluoracetat in 20 ml Dioxan zugetropft. Das Reaktionsgemisch wurde noch 3 Stunden bei 80° gerührt und dann abgekühlt, worauf das Lösungsmittel entfernt wurde. Der Rückstand wurde in Chloroform und 0.5N Salzsäure aufgenommen, worauf die organische Phase abgetrennt, über $MgSO_4$ getrocknet und eingeengt wurde. Der Rückstand wurde an 30 g Kieselgel mit Chloroform/Methanol (6:1) chromatographiert, worauf 285 mg (81.6%) (12S,15S,18S)-12,15-Dimethyl-10,13,16-trioxo-10,11,12,13,14,15,16,17,18,19,20,21-dodecahydro-1,18-etheno-dibenz[b,n][1,5,8,11]oxatriazacyclopentadecin-18-carbonsäuremethylester als amorpher Feskörper erhalten wurden. $[\alpha]_D$= -9.0° (c= 0.1, Chloroform). MS(FAB): 466 ($M^{+\bullet}$ + $H^+$, 100), 406 (30), 264 (45), 247 (50), 232 (40), 214 (40), 197 (30), 181 (35), 126 (55), 121 (40), 105 (40).

Beispiel 7.1.4.b

In Analogie zu Beispiel 7.1.4.a wurden aus 160 mg (0.21 mMol) (R)-2-[N-(D-Alanyl-D-alanyl)]-1,2,3,4-tetrahydro-8-[-2-(pentafluorophenoxycarbonyl)-phenoxy]-2-naphthalincarbonsäure-methylester-trifluoroacetat 76.5 mg (78.2%) (12R,15R,18R)-12,15-Dimethyl-10,13,16-trioxo10,11,12,13,14,15,16,17,18,19,20,21-dodecahydro-1,18-etheno-dibenz[b,n]oxatriazacyclopentadecin-18-carbonsäure-methylester als amorpher Festkörper erhalten. $[\alpha]_D$= +9.0° (c= 0.1, Chloroform). MS(FAB): 466 ($M^{+\bullet}$ + $H^+$, 100).

Beispiel 7.1.5.a

Zu einer Lösung von 340 mg (0.75 mMol) (12S,15S,18S)-12,15-Dimethyl-10,13,16-trioxo-10,11,12,13,14,15,16,17,18,19,20,21-dodecahydro-1,18-etheno-dibenz[b,n][1,5,8,11]oxatriazacyclopentadecin-18-carbonsäure-methylester in einem Gemisch von 3 ml Tetrahydrofuran, 1 ml Methanol und 1 ml Wasser wurden unter Eiskühlung 300 mg Lithiumhydroxyd-Monohydrat zugegeben. Das Reaktionsgemisch wurde 30 Minuten bei 0° und 1.5 Stunden bei Raumtemperatur gerührt und dann auf eine Mischung von Wasser und Chloroform/Methanol (4:1) gegossen. Die Wasserphase wurde abgetrennt und mit Chloroform/-Methanol (4:1) erschöpfend extrahiert. Die vereinigten organischen Phasen wurden über $MgSO_4$ getrocknet und eingeengt. Der Rückstand wurde aus Diaethylether/Hexan (1:1) kristallisiert, worauf nach Trocknen am Hochvakuum 270 mg (79.7%) (12S,15S,18S)-12,15-Dimethyl-10,13,16-trioxo-10,11,12,13,14,15,16,17,18,19,20,21-dodecahydro-1,18-etheno-dibenz[b,n]oxatriazacyclopentadecin-18-carbonsäure erhalten wurden. $[\alpha]_D$= -13.5° (c= 0.1, Methanol). MS(FAB): 451 ($M^{+\bullet}$, 30), 450 ($M^{+\bullet}$ - H, 100).

Beispiel 7.1.5.b

In Analogie zu Beispiel 7.1.5.a wurden aus 520 mg (1.12 mMol) (12R,15R,18R)-12,15-Dimethyl-10,13,16-trioxo-10,11,12,13,14,15,16,17,18,19,20,21-dodecahydro-1,18-etheno-dibenz[b,n]oxatriazacyclopentadecin-18-carbonsäure-methylester 435 mg (85%) (12R,15R,18R)-12,15-Dimethyl-10,13,16-trioxo-10,11,12,13,14,15,16,17,18,19,20,21-dodecahydro-1,18-etheno-dibenz[b,n]oxatriazacyclopentadecin-18-carbonsäure erhalten. $[\alpha]_D$= +14.0° (c= 0.1, Methanol). MS(FAB): 451 ($M^{+\bullet}$, 30), 450 ($M^{+\bullet}$ - H, 100).

Beispiel 7.2.1.a

Eine Lösung von 355 mg (0.66 mMol) 3-[(S)-7-(-tert.-Butoxycarbonylamino)-7-methoxycarbonyl-5,6,7,8-tetrahydro-naphthalin-1-yloxy]-thiophen-2-carbonsäure-benzylester im 1 ml Dichlormethan wurde analog zu Beispiel 7.1.1.a bei 0° mit 1 ml Trifluoressigsäure umgesetzt, worauf das Produkt mit 187 mg (0.99 mMol) Boc-L-alanin, 189 mg (0.99 mMol) N-Aethyl-N'-(3-dimethylaminopropyl)-carbodiimid-hydrochlorid, 225 mg (1.45 mMol) 1-Hydroxybenzotriazol und 339 µl (1.98 mMol) Ethyldiisopropylamin in 3.5 ml N,N-Dimethylformamid umgesetzt wurde. Nach Chromatographie an 110 g Kieselgel mit Essigester/Hexan (3:7 → 4:6) erhielt man nach Trocknen am Hochvakuum 336 mg (83.8%) 3-[(S)-7-[N-(tert.-Butoxycarbonyl-L-alanyl)]-7-methoxycarbonyl-5,6,7,8-tetrahydro-naphthalin-1-yloxy]-thiophen-2-carbonsäure-benzylester als amorphen Festkörper. $[\alpha]_D$= + 19.0° (c = 0.2, Chloroform). MS(FAB): 609 ($M^{+\bullet}$ + $H^+$, 70), 509.1 (90), 217.1 (100), 109 (40), 91.0 (95).

Beispiel 7.2.2.a

Eine Lösung von 300 mg (0.493 mMol) 3-[(S)-7-[N-(tert.-Butoxycarbonyl-L-alanyl)]-7-methoxycarbonyl-5,6,7,8-tetrahydro-naphthalin-1-yloxy]-thiophen-2-carbonsäure-benzylester in 1 ml Dichlormethan wurde in Analogie zu Beispiel 7.1.2.a bei 0° mit 1 ml Trifluoressigsäure umgesetzt, worauf das Produkt mit 123 mg (0.65 mMol) Boc-L-Alanin, 124.6 mg (0.65 mMol) N-Ethyl-N'-(3-dimethylaminopropyl)-carbodiimid-hydrochlorid, 148 mg (0.955 mMol) 1-Hydroxybenzotriazol und 222 µl (1.302 mMol) N-Aethyldiisopropylamin in 3 ml N,N-Dimethylformamid umgesetzt wurde. Nach Chromatographie an 110 g Kieselgel mit Chloroform/Methanol (98:2) und Trocknen am Hochvakuum

erhielt man 300 mg (92%) 3-[(S)-7-[N-[(tert.-butoxycarbonyl-L-alanyl)-L-alanyl]]-7-methoxycarbonyl-5,6,7,8-tetrahydronaphthalin-1-yloxy]-thiophen-2-carbonsäure-benzylester als amorphen Festkörper. $[\alpha]_D$= + 8.0° (c= 0.2, Chloroform). MS(FAB): 680.5 ($M^{+\bullet}$ + $H^+$, 60), 580.4 (50), 420.3 (20), 378.1 (30), 348.1 (30), 330.1 (20), 270.0 (20), 217 (30), 91 (100).

Beispiel 7.2.3.a

In Analogie zu Beispiel 7.1.3.a wurden 284 mg (0.43 mMol) 3-[(S)-7-[N-[(tert.-butoxycarbonyl-L-alanyl)-L-alanyl]]-7-methoxycarbonyl-5,6,7,8-tetrahydro-naphthalin-1-yloxy]-thiophen-2-carbonsäure-benzylester in 5 ml Methanol/Wasser (9:1) mit 100 mg Palladium/Kohle (10%) während 48 Stunden bei 2 bar hydriert. Das Produkt wurde zunächst mit 79.2 mg (0.43 mMol) Pentafluorphenol und 82.4 mg (0.43 mMol) N-Aethyl-N'-(3-dimethylaminopropyl)-carbodiimid-hydrochlorid in 2 ml Methylenchlorid umgesetzt, und das Produkt wurde nach Trocknen am Hochvakuum in Analogie zu Beispiel 7.1.3.a mit 2 ml Methylenchlorid und 2 ml Trifluoressigsäure behandelt, worauf nach Fällen mit Diaethylaether/Hexan und Trocknen am Hochvakuum 240 mg (72.5%) 3-[(S)-7[N-(L-alanyl-L-alanyl)]-7-methoxycarbonyl-5,6,7,8-tetrahydro-naphthalin-1-yloxy]-thiophen-2-carbonsäure-pentafluorphenylester-trifluoracetat als amorpher Festkörper erhalten wurden. MS(ISP): 678.3 ($M^{+\bullet}$ (freie Base) + $Na^+$, 50), 656.4 ($M^{+\bullet}$ (freie Base) + $H^+$, 70), 514.3 (100), 454.3 (35), 412.4 (25).

Beispiel 7.2.4.a

In Analogie zu Beispiel 7.1.4.a wurden 120 mg (0.156 mMol) 3-[(S)-7-[N-(L-alanyl-L-alanyl)]-7-methoxycarbonyl-5,6,7,8-tetrahydro-naphthalin-1-yloxy]-thiophen-2-carbonsäure-pentafluorphenylester-trifluoracetat in 10 ml Dioxan innerhalb von 18 Stunden bei 80° einem Gemisch von 120 ml Dioxan und 2 ml Pyridin zugetropft. Nach Chromatographie des Produkts an 12 g Kieselgel mit Chloroform/Methanol (95:5) und Trocknen am Hochvakuum wurden 50 mg (68%) (11S,14S,17S)-11,14-Dimethyl-9,12,15-trioxo-9,10,11,12,13,14,15,16,17,18,19,20-dodecahydro-1,17-etheno-thieno[3,2-b]benz[n][1,5,8,11]oxatriazacyclopentadecin-17-carbonsäure-methylester als amorpher Festkörper erhalten. $[\alpha]_D$= -57.3° (c= 0.15, Chloroform). MS(ISP) 492.2 ($M^{+\bullet}$ + $Na^+$, 25) 472.3 ($M^{+\bullet}$ + $H^+$, 75), 412.6 (100).

Beispiel 7.2.5.a

In Analogie zu Beispiel 7.1.5.a wurden 35 mg (76.5 μMol) (11S,14S,17S)-11,14-Dimethyl-9,12,15-trioxo-9,10,11,12,13,14,15,16,17,18,19,20-dodecahydro-1,17-etheno-thieno[3,2-b]benz[n][1,5,8,11]oxa-triazacyclo-pentadecin-17-carbonsäure-methylester in 1.5 ml Tetrahydrofuran/Methanol/Wasser (3:1:1) mit 34 mg Lithiumhydroxid-Monohydrat umgesetzt, worauf nach Fällung aus Diaethylether/Hexan (1:1) und Trocknen am Hochvakuum 29.6 mg (87%) (11S,14S,17S)-11,14-Dimethyl-9,12,15-trioxo-9,10,11,12,13,14,15,16,17,18,19,20-dodecahydro-1,17-etheno-thieno [3,2-b]benz[n][1,5,8,11]oxatriazacyclopentadecin-17-carbonsäure als weisser amorpher Feststoff erhalten wurden. MS (FAB): 457.4 ($M^{+\bullet}$, 20), 456.4 ($M^{+\bullet}$ - H, 100). IR(KBr): 3380s(br), 3107w, 3087w, 2977w, 2928w, 1724m, 1643s, 1583w, 1536s, 1460m, 1420w, 1388m, 1236m, 1120w, 1019w, 775w.

Beispiel 7.3.1.a

Eine Lösung von 540 mg (0.94 mMol) (S)-tert.-Butoxycarbonylamino-8-(2-benzyloxycarbonyl-4-nitro-phenoxy)-1,2,3,4-tetrahydro-naphthalin-2-carbonsäure-methylester in 2 ml Dichlormethan wurde analog zu Beispiel 7.1.1.a bei 0° mit 2 ml Trifluoressigsäure umgesetzt, und das erhaltene Produkt wurde mit 266 mg (1.41 mMol) Boc-L-alanin, 270 mg (1.41 mMol) N-Ethyl-N'-(3-dimethylamino-propyl)carbodiimid-hydrochlorid, 279 mg (2.1 mMol) 1-Hydroxybenzotriazol und 482 ml (2.82 mMol) Aethyldiisopropylamin in 5 ml N,N-Dimethylformamid umgesetzt. Nach Chromatographie an 80 g Kieselgel mit Hexan/Essigester (1:1 → 2:3) erhielt man 468 mg (77%) (S)-8-(2-Benzyloxycarbonyl-4-nitrophenoxy)-2-[N-(tert.-butoxycarbonyl-L-alanyl)]-1,2,3,4-tetrahydro-naphthalin-2-carbonsäure-methylester als amorphen Schaum. MS(FAB): 648.1 ($M^{+\bullet}$ + $H^+$, 40), 548.1 (50), 217 (80), 109 (40), 91(100).

Beispiel 7.3.2.a

Eine Lösung von 575 mg (0.88 mMol) (S)-8-(2-Benzyloxycarbonyl-4-nitrophenoxy)-2-[N-(tert.-butoxycarbonyl-L-alanyl)]-1,2,3,4-tetrahydronaphthalin-2-carbonsäure-methylester in 1 ml Dichlormethan wurde analog zu Beispiel 7.1.1.a bei 0° mit 1.5 ml Trifluoressigsäure umgesetzt, und das Produkt wurde mit 287 mg (1.33 mMol) Boc-L-Prolin, 255 mg (1.33 mMol) N-Aethyl-N'-(3-dimethylaminopropyl)-carbodiimid-hydrochlorid, 270 mg (1.99 mMol) 1-Hydroxybenzotriazol und 455 μl (2.66 mMol) Aethyldiisopropylamin in 8 ml N,N-Dimethylformamid umgesetzt. Nach Chromatograhie and 100 g Kieselgel mit Chloroform erhielt man 572 mg (86.5%) (S)-8-(2-Benzyloxycarbonyl-4-nitrophenoxy)-2-[N-[(tert.-butoxycarbonyl-L-prolyl)-L-alanyl]]-1,2,3,4-tetrahydro-naphthalin-2-carbonsäure-methylester als amorphen Schaum. MS(ISP): 745.5 ($M^{+\bullet}$ + $H^+$, 70), 689.4 (20), 645.4 (100), 477.4 (85).

Beispiel 7.3.3.a

a) Eine Suspension von 540 mg (0.78 mMol) (S)-8-(2-Benzyloxycarbonyl-4-nitrophenoxy)-2-[N-(tert.-butoxycarbonyl-L-prolyl)-L-alanyl]]-1,2,3,4-tetrahydro-naphthalin-2-carbonsäure-methylester und 200 mg Palladium-Kohle (10%) in 18 ml Ethanol wurde bei Raumtemperatur während 2 Stunden unter Atmosphärendruck hydriert und dann über Celite filtriert. Der Filterrückstand wurde mit Aethanol gewaschen, und das Filtrat wurde eingedampft. Nach Trocknen des Rückstands am Hochvakuum erhielt man 440 mg (97%) (S)-8-(4-Amino-2-carboxyphenoxy)-2-[N-[(tert.-butoxycarbonyl-L-prolyl)-L-alanyl]]-1,2,3,4-tetrahydro-naphthalin-2-carbonsäuremethylester als amorphen Festkörper. Smp. > 135°. $[\alpha]_D$ = -13.0° (MeOH, c = 0.1). MS(ISP): 625.3 ($M^{+\bullet}$ + $H^+$, 10), 525.5 (100).

b) Zu einer Lösung von 340 mg (0.544 mMol) des obigen Produktes in 3 ml Pyridin wurden bei Raumtemperatur 308 µl (3.26 mMol) Essigsäureanhydrid zugegeben. Das Gemisch wurde 2 Stunden gerührt und dann eingeengt. Der Rückstand wurde in Methylenchlorid aufgenommen, die Lösung wurde zweimal mit $H_2O$ gewaschen, die Wasserphasen wurde mit Methylenchlorid extrahiert, und die vereinigten organischen Phasen wurden über $MgSO_4$ getrocknet und eingeengt. Der Rückstand wurde am Hochvakuum getrocknet. Nach Kristallisation aus Diaethylether/Hexan (2:1), Waschen und Trocknen am Hochvakuum erhielt man 320 mg (88%) (S)-8-(4-Acetylamino-2-carboxyphenoxy)-2-[N-[(tert.-butoxycarbonyl-L-prolyl)-L-alanyl]-1,2,3,4-tetrahydro-naphthalin-2-carbonsäure-methylester als amorphes Produkt $[\alpha]_D$= -55.0° ($CHCl_3$, c = 0.2). MS (ISP): 689.6 ($M^{+\bullet}$ +$Na^+$, 50), 684.6 ($M^{+\bullet}$ + $NH_4^+$, 60), 667.5 ($M^{+\bullet}$ + $H^+$, 50), 567.2 (100).

c) Zu einer Lösung von 323 mg (0.48 mMol) des obigen Produktes und 133 mg (0.72 mMol) Pentafluorphenol in 4 ml Dichlormethan wurden bei 0° portionenweise 138 mg (0.72 mMol) N-Ethyl-N'-(3-dimethylaminopropyl)-carbodiimid-hydrochlorid zugegeben, worauf das Gemisch 2 Stunden bei 0° gerührt und dann auf Phosphat-Puffer (pH 6.8) gegeben wurde. Die Wasserphase wurde mit Dichlormethan extrahiert, und die vereinigten organischen Phasen wurden mit gesättigter Natriumchlorid-Lösung extrahiert, über $MgSO_4$ getrocknet und eingeengt. Der Rückstand wurde am Hochvakuum getrocknet und dann in 2 ml Dichlormethan gelöst, worauf bei 0° 1.5 ml Trifluoressigsäure zugegeben wurden. Das Gemisch wurde 2.5 Stunden bei 0° gerührt und dann eingeengt. Der Rückstand wurde am Hochvakuum getrocknet und in Diethylaether suspendiert, abfiltriert, mit Diaethylaether gewaschen und am Hochvakuum getrocknet, worauf 327 mg (80%) (S)-8-(4-Acetylamino-2-pentafluorphenoxycarbonylphenoxy)-2-[N-(L-prolyl-L-alanyl)]-1,2,3,4-tetrahydro-naphthalin-2-carbonsäure-methylestertrifluoroacetat erhalten wurden. Smp. 136-146° (Sintern). $[\alpha]_D$= -23.0° ($CHCl_3$, c = 0.2). MS (ISP): 733.4 ($M^{+\bullet}$ + $H^+$, 100).

Beispiel 7.3.4.a

Eine Lösung von 227 mg (0.268 mMol) (S)-8-(4-Acetylamino-2-pentafluorphenoxycarbonylphenoxy)-2-[N-(L-prolyl-L-alanyl)]-1,2,3,4-tetrahydro-naphthalin-2-carbonsäure-methylester-trifluoracetat in 20 ml Dioxan wurde innerhalb von 18 Stunden unter Argon bei 80° zu einem Gemisch von 300 ml Dioxan und 4.5 ml Pyridin zugetropft. Das Reaktionsgemisch wurde 2 Stunden bei 80° gerührt, abgekühlt, eingeengt und auf eine Mischung von 0,1 N HCl-Lösung und Dichlormethan gegossen. Die organische Phase wurde abgetrennt, über Magnesiumsulfat getrocknet und eingeengt. Der Rückstand wurde an 100 g Kieselgel mit Chloroform/Methanol (98:2 → 9:1) vorgereinigt, worauf nach Reinigung mittels präparativer HPLC (Säule: Vydac $C_{18}$ 100 250-10; Programm: 25% Acetonitril → 60% Acetonitril), Lyophilisierung und Trocknen 102.4 mg (70%) (14aS,17S,20S)-8-Acetylamino-17-methyl-10,15,18-trioxo-11,12,13,14,14a,15,16,17,18,19,20,21,22,23-tetradecahydro-1,20-etheno-10H-pyrrolo[1,2-e]dibenz]b,n][1,5,8,11] oxatriazacyclopentadecin-20-carbonsäuremethylester als leicht gelblicher amorpher Festkörper erhalten wurden. Smp. 180-200° (Sintern). $[\alpha]_D$ = -11.6° ($CHCl_3$, c = 0.06). MS (ISP): 571.5 ($M^{+\bullet}$ +$Na^+$, 30), 566.6 ($M^{+\bullet}$ + $NH_4^+$, 100), 549.6 ($M^{+\bullet}$ + $H^+$, 95). Daneben erhielt man 13.0 mg (4.4%) dimeres Produkt Cyclo[L-alanyl-[(S)-2-(7-amino-7-methoxycarbonyl-5,6,7,8-tetrahydronaphthalin-1-yloxy)-benzoyl]-L-prolyl-L-alanyl]-[(S)-2-(7-amino-7-methoxycarbonyl-5,6,7,8-tetrahydro-naphthalin-1-yloxy)-benzoyl]-L-prolyl-]; Smp. (Zers.) > 280°.

Beispiel 7.3.5.a

Zu einer Lösung von 20 mg (0.036 mMol) (14aS,17S,20S)-8-Acetylamino-17-methyl-10,15,18-trioxo-11,12,13,14a,15,16,17,18,19,20,21,22,23-tetradecahydro-1,20-etheno-10H-pyrrolo[1,2-e]dibenz[b,n][1,5,8,11]oxatriazacyclopentadecin-20-carbonsäuremethylester in 1 ml Tetrahydrofuran/Methanol/Wasser (3:1:1) wurden unter Eiskühlung 16 mg Lithiumhydroxyd-monohydrat zugegeben. Das Reaktionsgemisch wurde 2 Stunden bei Raumtemperatur gerührt und dann auf eine Mischung von Eis, 0.5N HCl und Chloroform gegossen. Die Wasserphase wurde abgetrennt und mit Chloroform extrahiert. Die vereinigten organischen Phasen wurden über Natriumsulfat getrocknet und eingedampft. Der Rückstand wurde in Diaethylaether/Hexan (1:1) suspendiert, abfiltriert und am Hochvakuum getrocknet, worauf 18 mg (92%) (14aS,17S,20S)-8-Acetylamino-17-methyl-10,15,18-trioxo-11,12,13,14,14a,15,16,17,18,19,20,21,22,23- tetradecahydro-1,20-etheno-10H-pyrrolo[1,2-e]dibenz[b,n][1,5,8,11]oxatriazacyclopentadecin-20-carbonsäure als weisses Pulver erhalten wurden. Smp. 70-110° (Sintern). $[\alpha]_D$= -32.0° (Methanol, c = 0.15). MS (ISP): 533.2 ($M^{+\cdot}$ + $H^+$, 100).

Beispiel 7.4.1.1.a

In Analogie zu Beispiel 7.1.1.a wurden 816 mg (1.46 mMol) (S)-2-(7-Benzyloxycarbonyl-7-tert.-butoxycarbonylamino-5,6,7,8-tetrahydronaphthalin-1-yloxy)-nicotinsäure-prop-2-enylester zunächst bei 0° mit 1.5 ml Trifluoressigsäure und 2 ml Dichlormethan behandelt. Das Produkt wurde anschliessend mit 414 mg (2.19 mMol) Boc-L-Alanin, 444 mg (3.28 mMol) 1-Hydroxybenzotriazol, 428 mg (2.19 mMol) N-Aethyl-N'-(3-dimethylaminopropyl)-carbodiimid hydrochlorid und 750 µl (4.38 mMol) Aethyldiisopropylamin in 7 ml N,N-Dimethylformamid umgesetzt. Nach Aufarbeitung gemäss Beispiel 7.1.1.a und Chromatgraphie an 130 g Kieselgel mit Hexan/Essigsäureethylester (1:1) und Trocknen am Hochvakuum erhielt man 792 mg (86%) 2-[(S)-7-Benzyloxycarbonyl-7-[N-[(tert.-butoxycarbonyl-L-alanyl)]-5,6,7,8-tetrahydro-naphthalin-1-yloxy]-nicotinsäure-prop-2-enylester als amorphen Festkörper. Smp. 55-68°. $[\alpha]_D$ +23.0° (Chloroform, c = 0.2). MS(ISP): 647.6 ($M^{+\bullet}$ + $NH_4^+$, 29), 630.6 ( $M^{+\bullet}$ + $H^+$, 100).

Beispiel 7.4.1.2.a

In Analogie zu Beispiel 7.1.1.a wurden 321 mg (0.51 mMol) 2-[(S)-7-Benzyloxycarbonyl-7-[N-[(tert.-butoxycarbonyl-L-alanyl)]-5,6,7,8-tetrahydronaphthalin-1-yloxy]-nicotinsäure-prop-2-enylester in 1.5 ml Dichloraethan bei 0° mit 1.0 ml Trifluoressigsäure behandelt. Das Produkt wurde am Hochvakuum getrocknet und mit 340 mg (0.77 mMol) Boc-Glu(OMe)OH, 155 mg (1.15 mMol) 1-Hydroxybenzotriazol, 147 mg (0.77 mMol) N-Aethyl-N'-(3-dimethylaminopropyl)-carbodiimid-hydrochlorid und 290 µl (1.69 mMol) Aethyl-diisopropylamin in 4 ml N,N-Dimethylformamid umgesetzt. Nach Chromatographie an 120 g Kieselgel mit Hexan/Essigsäureethylester (1:1 → 2:3) und nach Trocknen am Hochvakuum erhielt man 293 mg (99%) 2-[(S)-7-Benzyloxycarbonyl-7-[N-[(tert.-butoxycarbonyl-L-$O^6$-methylglutamyl)-L-alanyl]]-5,6,7,8-tetrahydronaphthalin-1-yloxy]-nicotinsäure-prop-2-enylester. Smp 55-75° $[\alpha]_D$ = +24° (Chloroform, c = 0.2). MS(ISP): 795.5 ($M^{+\bullet}$ + $Na^+$, 25), 793.6 ($M^{+\bullet+}$ + $H^+$, 100).

Beispiel 7.4.1.3.a

Zu 5 ml eines Gemischs von Dimethylsulfoxid, Tetrahydrofuran und 0.5N Salzsäure (2:2:1) wurde solange N-Methylanilin zugegeben, bis das pH der Lösung 7 erreichte. Die Lösung wurde 15 Minuten mit Stickstoff durchgespült, dann mit 287 mg (0.37 mMol) 2-[(S)-7-Benzyloxycarbonyl-7-[N-[(tert.-butoxycarbonyl-L-$O^6$-methylglutamyl)-L-alanyl]]-5,6,7,8-tetrahydronaphthalin-1-yloxy]-nicotinsäure-prop-2-enylester und 74.5 mg (0.065 mMol) Tetrakis (triphenylphosphin) palladium versetzt und während 24 Stunden gerührt. Nach Einengen wurde der Rückstand auf eine Mischung von Dichlormethan, Eis und Wasser gegossen, wonach das pH mit 1N HCl-Lösung auf 3 gestellt wurde. Die wässrige Phase wurde abgetrennt und zweimal mit Dichlormethan extrahiert. Die vereinigten organischen Phasen wurden über Magnesium-sulfat getrocknet und eingeengt. Der Rückstand wurde an 50 g Kieselgel mit Chloroform/Methanol (95:5 → 4:1) chromatographiert, worauf nach Fällen des Rückstands aus Diaethylether/Hexan, Filtration und Trocknen 229 mg (84%) 2-[(S)-7-Benzyloxycarbonyl-7-[N-[(tert-butoxycarbonyl-L-$O^6$-methylglutamyl)-L-alanyl]]-5,6,7,8-tetrahydronaphthalin-1-yloxy]-nicotinsäure erhalten wurden. Smp. (Zers.) > 145°. $[\alpha]_D$= +17.0° (Chloroform, c = 0.1). In Analogie zu Beispiel 7.1.4.a wurden 256 mg (0.35 mMol) dieser Säure und 96 mg (0.52 mMol) Pentafluorphenol in 4 ml Dichlormethan mit 100 mg (0.52 mMol) N-Ethyl-N'-(3-dimethylaminopropyl)-carbodiimid hydrochlorid umgesetzt, worauf das Produkt bei 0° mit 1.5 ml Trifluoressigsäure und 2 ml Dichlormethan umgesetzt wurde. Nach Fällen aus Diaethylaether/Hexan (1:1), Filtration und Trocknen erhielt man 315 mg (90%) 2-[(S)-7-Benzyloxycarbonyl-7-[N-[(L-$O^6$-methylglutamyl)-L-alanyl]]-5,6,7,8-tetrahydro-naphthalin-1-yloxy]-nicotinsäure-pentafluorphenylester-trifluoracetat als weisses Pulver. Smp. 95-99°. $[\alpha]_D$= +17.0° (Chloroform, c = 0.2). MS(ISP): 799.3 ($M^{+\bullet}$ + $H^+$, 100).

Beispiel 7.4.1.4.a

Zu einer Lösung von 30 mg 4-(N,N-Dimethylamino)-pyridin und 3 ml Pyridin in 250 ml N,N-Dimethylformamid wurde bei Raumtemperatur innerhalb von 18 Stunden eine Lösung von 187 mg (0.2 mMol) 2-[(S)-7-Benzyloxycarbonyl-

7-[N-[(L-$O^6$-methylglutamyl)-L-alanyl]]-5,6,7,8-tetrahydro-naphthalin-1-yloxy]-nicotinsäure-pentafluorphenylester-trifluoracetat in 12 ml N,N-Dimethylformamid zugegeben. Das Reaktionsgemisch wurde während 2 Stunden nachgerührt und dann eingeengt, worauf der Rückstand in einer Mischung von Eis, 0.1 N HCl und Dichlormethan aufgenommen wurde. Die Wasserphase wurde abgetrennt und mit Dichlormethan extrahiert, worauf die vereinigten organischen Phasen über Magnesiumsulfat getrocknet und eingeengt wurden. Der Rückstand wurde in Acetonitril suspendiert, die Suspension wurde filtriert, der Filterrückstand (40 mg dimeres Nebenprodukt) wurde verworfen, und das Filtrat wurde eingedampft. Der Rückstand wurde an 30 g Kieselgel mit Chloroform/Methanol (99:1 → 98:2) vorgereinigt, und das erhaltene Produkt wurde mittels präparativer HPLC (Säule: Vydac 250-10, RP18, 30% Acetonitril → 100% Acetonitril in 20 Minuten) chromatographiert, worauf nach Lyophilisieren und Trocknen 72 mg (50%). (12S,15S,18S)-12-(2-Methoxycarbonyl-aethyl)-15-methyl-10,13,16-trioxo-10,11,12,13,14,15,16,17,18,19,20,21-dodecahydro-1,18-etheno-pyrido[2,3-b]-benz[n][1,5,8,11]-oxatriazacyclopentadecin-18-carbonsäure-benzylester als weisser Feststoff erhalten wurden. Smp. 120-123° $[\alpha]_D$= -44.6° (Chloroform, c = 0.15). MS(ISP): 632.4 ($M^{+\bullet}$ + $NH_4^+$, 15), 615.3 ($M^{+\bullet}$ + $H^+$, 20), 531.4 (70), 431.4 (100).

Beispiel 7.4.1.5.a

Eine Suspension von 47.0 mg (76 μMol) (12S,15S,18S)-12-(2-Methoxycarbonyl-ethyl)-15-methyl-10,13,16-trioxo-10,11,12,13,14,15,16,17,18,19,20,21-dodecahydro-1,18-etheno-pyrido[2,3-b]benz[n][1,5,8,11]oxatriazocyclopentadecin-18-carbonsäure-benzylester und 10 mg Palladium-Kohle (10%) in 5 ml Trifluoraethanol wurde unter Normaldruck während 3 Stunden bei Raumtemperatur hydriert und dann über Celite filtriert, worauf der Filterrückstand mit Trifluoraethanol gewaschen und das Filtrat eingeengt wurde. Der Rückstand wurde in Chloroform und gesättigter Kochsalz-Lösung aufgenommen, worauf das pH mit 0.1N HCl auf 1 gestellt und die wässrige Phase abgetrennt und erschöpfend mit Chloroform extrahiert wurde. Die vereinigten Chloroform-Phasen wurden über $Na_2SO_4$ getrocknet und eingeengt. Der Rückstand wurde in Diaethylaether suspendiert, die Suspension wurde filtriert, und der Filterrückstand wurde am Hochvakuum getrocknet, wobei 28.6 mg (71%) (12S,15S,18S)-12-(2-Methoxycarbonyl-aethyl)-15-methyl-10,13,16-trioxo-10,11,12,13,14,15,16,17,18,19,20,21-dodecahydro-1,18-etheno-pyrido[2,3-b]benz[n][1,5,8,11]oxatriazacyclopentadecin-18-carbonsäure als weisses Pulver erhalten wurden. Smp. (Zers.) > 120°. IR(Kbr) 3378 m (br.), 3060w, 2945w, 173sc, 1658s, 1588w, 1539m, 1422m, 1224m, 1175m. MS (ISP): 542.2 ($M^{+\bullet}$ + $NH_4^+$, 25), 525.3 ($M^{+\bullet}$ + $H^+$, 100).

Beispiel 7.4.2.1.a

Eine Lösung von 298 mg (0.48 mMol) 3-[(S)-7-Benzyloxycarbonylamino-7-trimethylsilylethoxycarbonyl-5,6,7,8-tetrahydro-naphthalin-1-yloxy]nicotinsäure-tert.-butylester in 5 ml Aethanol wurde mit 80 mg Palladium-Kohle (10%) 1 Stunde bei Raumtemperatur unter Wasserstoffatmosphäre hydriert, worauf über Hyflo filtriert und das Filtrat eingeengt wurde. Der Rückstand wurde am Hochvakuum getrocknet und an 50 g Kieselgel mit Chloroform/Methanol (95:5) chromatographiert, worauf 190 mg (81.7%) 3-[(S)-7-Amino-7-trimethylsilylaethoxycarbonyl-5,6,7,8-tetrahydro-naphthalin-1-yloxy]-nicotinsäure-tert.-butylester erhalten wurden. MS(ISP): 485.5 ($M^{+\bullet}$ + $H^+$, 50), 429 (15), 401.3 (100).

In Analogie zu Beispiel 7.1.1.a wurden 170 mg (0.35 mMol) 3-[(S)-7-Amino-7-trimethylsilylethoxycarbonyl-5,6,7,8-tetrahydro-naphthalin-1-yloxy]-nicotinsäure-tert.-butylester, 140 mg (0.63 mMol) Z-L-Alanin, 127 mg (0.94 mMol) 1-Hydroxybenzotriazol, 120 mg (0.63 mMol) N-Ethyl-N'-(3-dimethylaminopropyl)-carbodiimid-hydrochlorid und 120 μl (0.7 mMol) Ethyldiisopropylamin in 2 ml N,N-Dimethylformamid ungesetzt. Nach Aufarbeitung gemäss Beispiel 7.1.1.a und Chromatographie an 60 g Kieselgel mit Hexan/Essigsäureethylester (1:1) erhielt man 145 mg (60%) 2-[(S)-7-[N-(Benzyloxycarbonyl-L-alanyl)]-7-(2-trimethylsilylaethoxy-carbonyl)-5,6,7,8-tetrahydro-naphthalin-1-yloxy]-nicotinsäure-tert.-butylester als amorphen Schaum. Smp 57-67°. MS(ISP): 712.4 ($M^{+\bullet}$ + $Na^+$, 30) 690.5 ($M^{+\bullet}$ + $H^+$, 100), 606.4 (20). $[\alpha]_D$= +49.5° ($CHCl_3$, c = 0.2).

Beispiel 7.4.2.2.a

Analog zu Beispiel 7.4.2.1.a wurden 166 mg (0.204 mMol) 2-[(S)-7-[N-(Benzyloxycarbonyl-L-alanyl)]-7-(2-trimethylsilylaethoxycarbonyl)-5,6,7,8-tetrahydro-naphthalin-1-yloxy]-nicotinsäure-tert.-butylester in 4 ml Methanol mit 34 mg Palladium-Kohle hydriert. In Analogie zu Beispiel 7.1.1.a wurden 160 mg (0.24 mMol) des getrockneten Produktes mit 106 mg (0.36 mMol) Z-Glu(OMe)OH, 73 mg (0.54 mMol) 1-Hydroxybenzotriazol, 69 mg (0.36 mMol) N-Aethyl-N'-(3-dimethylaminopropyl)-carbodiimidhydrochlorid und 82 μl (0.48 mMol) Ethyldiisopropylamin umgesetzt. Nach Aufarbeitung und Chromatographie an 30 g Kieselgel mit Chloroform/Methanol (9:1) und nach Trocknen am Hochvakuum erhielt man 138 mg (80,7%) 2-[(S)-7-[N-[(Benzyloxycarbonyl-L-$O^6$-methylglutamyl)-L-alanyl]]-7-(2-trimethylsilylaethoxycarbonyl)-5,6,7,8-tetrahydro-naphthalin-1-yloxy]-nicotinsäure-tert.-butylester als amorphen Feststoff. Smp. >60°. $[\alpha]_D$= +30.5° (Chloroform, c = 0.2). MS (ISP): 833.5, ($M^{+\bullet}$ + $H^+$, 100).

Beispiel 7.4.2.3.a

Zu einer Lösung von 120 mg (0.144 mMol) 2-[(S)-7-[N-[(Benzyloxycarbonyl-L-$O^6$-methylglutamyl)-L-alanyl]]-7-(2-trimethylsilylaethoxycarbonyl)-5,6,7,8-tetrahydro-naphthalin-1-yloxy]-nicotinsäure-tert.-butylester in 1 ml Dichlormethan wurden unter Argon und Eiskühlung 0.5 ml Trifluoressigsäure zugetropft. Das Gemisch wurde 1 Stunde bei 0° gerührt und dann zu Trockene eingedampft. Der Rückstand wurde am Hochvakuum getrocknet und in 1 ml Dimethylformamid gelöst, worauf 79.5 mg (0.432 mMol) Pentafluorphenol und 83.0 mg (0.432 mMol) N-Ethyl-N'-(3-dimethylaminopropyl)carbodiimid-hydrochlorid zugegeben wurden. Das Gemisch wurde 18 Stunden bei Raumtemperatur gerührt, worauf das Lösungsmittel am Hochvakuum abgezogen wurde. Der Rückstand wurde am Hochvakuum getrocknet und in 4 ml N,N-Dimethylformamid gelöst, und die Lösung wurde innerhalb von 18 Stunden bei Raumtemperatur unter Wasserstoff bei Atmosphärendruck zu einer Suspension von 50 mg N,N-Dimethylaminopyridin, 1 ml Pyridin, 50 mg Palladium-Kohle und 100 ml N,N-Dimethylformamid zugegeben. Das Gemisch wurde noch 1 Stunde nachgerührt und dann eingeengt. Der Rückstand wurde in Chloroform und Wasser aufgenommen, und die organische Phase wurde abgetrennt, über $NaSO_4$ getrocknet und eingedampft. Der Rückstand wurde am Hochvakuum getrocknet und anschliessend in 2N HCl und Dioxan bei Raumtemperatur gerührt, worauf Chloroform zugegeben wurde und die organische Phase abgetrennt, über Magnesiumsulfat getrocknet und eingeengt wurde. Der Rückstand wurde aus Diethylaether gefällt, abfiltriert und mit Diethylether gewaschen, worauf 38.5 mg (51%) (12S,15S,18S)-12-(2-Methoxycarbonyl-aethyl)-15-methyl-10,13,16-trioxo-10,11,12,13,14,15,16,17,18,19,20,21-dodecahydro-1,18-etheno-pyrido [2,3-b]-benz[n][1,5,8,11]oxatriazacyclo-pentadecin-18-carbonsäure als weisses Pulver erhalten wurden. MS(ISP): 542.2 ($M^{+\bullet}$ + $NH_4^+$, 25), 525.3 ($M^{+\bullet}$ + $H^+$, 100). Identisch mit Produkt aus Beispiel 7.4.1.5.a.

Beispiel 7.5.1.a

In Analogie zu Beispiel 7.1.1.a wurden 309 mg (0.49 mMol) 2-[(S)-7-Benzyloxycarbonyl-7-[N-(tert.-butoxycarbonyl-L-alanyl)]-5,6,7,8-tetrahydronaphthalin-1-yloxyl]-nicotinsäure-prop-2-enylester in 1,5 ml Dichlormethan bei 0° mit 1.0 ml Trifluoressigsäure umgesetzt, worauf das Produkt mit 183 mg (0.74 mMol) Boc-Asp(OMe)OH, 149 mg (1.10 mMol) 1-Hydroxybenzotriazol, 141 mg (0.74 mMol) N-Ethyl-N'-(3-dimethylaminopropyl)-carbodiimid-hydrochlorid und 210 µl (1.23 mMol) Aethyldiisopropylamin in 4 ml N,N-Dimethylformamid umgesetzt wurde. Nach Chromatographie an 90 g Kieselgel mit Hexan/Essigsäureaethylester (1:1 $\rightarrow$ 2:3) und Trocknen am Hochvakuum wurden 360 mg (96.5%) 2-[(S)-7-Benzyloxycarbonyl-7-[N-[(tert.-butoxycarbonyl-L-$O^5$-methylaspartyl)-L-alanyl]]-5,6,7,8-tetrahydronaphthalin-1-yloxy]-nicotinsäure-prop-2-enylester als amorpher Feststoff erhalten. Smp. 55-73°. $[\alpha]_D$= + 34.0° (Chloroform, c = 0.2). MS(ISP): 776.6 ($M^{+\bullet}$ + $NH_4^+$, 40), 759.5 ($M^{+\bullet}$ + $H^+$, 100).

Beispiel 7.5.2.a

In Analogie zu Beispiel 7.4.1.3.a wurden 330 mg (0.43 mMol) 2-[(S)-7-Benzyloxycarbonyl-7-[N-[(tert.-butoxycarbonyl-L-$O^5$-methylaspartyl)-L-alanyl]]-5,6,7,8-tetrahydro-naphthalin-1-yloxy]-nicotinsäure-prop-2-enylester mit 74.5 mg (0.65 mMol) Tetrakis (triphenylphosphin) palladium umgesetzt. Nach Chromatographie an 50 g Kieselgel mit Chloroform/Methanol (95:5 $\rightarrow$ 4:1), Fällen des Rückstandes mit Diaethylaether/Hexan (1:1), Filtration und Trocknen erhielt man 231 mg (74%) 2-[(S)-7-Benzyloxycarbonyl-7-[N-[(tert.-butoxycarbonyl-L-$O^5$-methylaspartyl)-L-alanyl]]-5,6,7,8-tetrahydronaphthalin-1-yloxy]-nicotinsäure als weisses Pulver. Smp.116-120°. $[\alpha]_D$= + 32.0° (Chloroform, c = 0.1).

In Analogie zu Beispiel 7.1.3.a wurden 220 mg (0.31 mMol) dieser Säure mit 84 mg (0.46 mMol) Pentafluorphenol und 88 mg (0.46 mMol) N'-Aethyl-N-(3-dimethyl-aminopropyl)-carbodiimid-hydrochlorid in 2 ml Dichlormethan umgesetzt, worauf das Produkt bei 0° mit 1.5 ml Trifluoressigsäure in 2 ml Dichlormethan behandelt wurde. Nach Fällen aus Diethylaether/Hexan (1:1), Filtration und Trocknen wurden 255 mg (92%) 2-[(S)-7-Benzyloxycarbonyl-7-[N-(L-$O^5$-methylaspartyl-L-alanyl)]-5,6,7,8-tetrahydro-naphthalin-1-yloxy]-nicotinsäure-pentafluorphenylester-trifluoracetat als weisses Pulver erhalten. Smp. 171-174°. $[\alpha]_D$= +13.0° (Chloroform, c = 0.2). MS(ISP): 785.3 ($M^{+\bullet}$ + $H^+$, freies Amin, 100).

Beispiel 7.5.3.a

In Analogie zu Beispiel 7.4.1.4.a wurden 190 mg (0.211 mMol) 2-[(S)-7-Benzyloxycarbonyl-7-[N-(L-$O^5$-methylaspartyl-L-alanyl)]-5,6,7,8-tetrahydronaphthalin-1-yloxy]-nicotinsäure-pentafluorphenylester-trifluoracetat in 20 ml N,N-Dimethylformamid innerhalb von 22 Stunden zu einer Lösung von 300 ml N,N-Dimethylformamid, 2.5 ml Pyridin und 80 mg 4-(N,N-Dimethylamino)pyridin zugegeben. Nach Chromatographie an 50 g Kieselgel mit Chloroform/Methanol (99:1 $\rightarrow$ 95:5) erhielt man 43 mg (17%) dimeres Nebenprodukt als weisses Pulver (MS(ISP): 1202.5 ($M^{+\bullet}$ + $H^+$, 100), Smp. (Zers.) > 180°) und 80 mg (~67%) rohes Hauptprodukt, welches mittels präparativer HPLC-Chromatographie (Säule; Vydac 250-10, RP 18, Programm: 30% Acetonitril → 100% Acetonitril in 20 Minuten) gereinigt wurde. Nach Lyophilisieren und Trocknen wurden 60 mg (47%) (12S,16S,19S)-16-Methyl-10,13,17,23-tetraoxo-10,11,12,13,14,15,16,17,18,19,20, 21,22,23-tetradecahydro-1,19-etheno-12,15-metheno-pyrido[2,3-b]benz[o][1,5,9,12]-oxatriazacyclohexadecin-19-carbonsäure-benzylester als weisses Pulver erhalten. Smp. (Zers.) >200°. $[\alpha]_D$= -17.0° (Chloroform, c = 0.2). MS(FAB): 569.3 ($M^{+\bullet}$ + $H^+$, 60), 217 (60), 109 (30), 91 (100).

Beispiel 7.5.4.a

Eine Suspension von 50 mg (88 µMol) (12S,16S,19S)-16-Methyl-10,13,17,23-tetraoxo-10,11,12,13,14,15,16,17,18,19,20,21,22,23-tetradecahydro-1,19-etheno-12,15-metheno-pyrido-[2,3-b]benz[o][1,5,9,12]oxatriazacyclohexadecin-19-carbonsäure-benzylester und 18 mg Palladiumkohle (10%) in Trifluoraethanol wurde während 2 Stunden bei Raumtemperatur unter Normaldruck hydriert und dann über Celite filtriert, worauf der Filterrückstand mit Trifluoraethanol gewaschen und das Filtrat eingeengt wurde. Der Rückstand wurde in Chloroform suspendiert, die Suspension wurde filtriert, und der Filterrückstand wurde zuerst mit Chloroform und dann mit Diaethylether/Essigsäureethylester (1:1) gewaschen und anschliessend am Hochvakuum getrocknet, wobei 41 mg (97%) (12S,16S,19S)-16-Methyl-10,13,17,23-tetraoxo-10,11,12,13,14,15,16,17,18,19,20 21,22,23-tetradecahydro-1,19-etheno-12,15-metheno-pyrido[2,3-b]benz[o][1,5,9,12]oxatriazacyclohexadecin-19-carbonsäure erhalten wurden. Smp. (Zers.) >255°. $[\alpha]_D$ = -4.0° (Methanol, c = 0.15). MS(ISN): 477.3 ($M^{+\bullet}$ - $H^-$, 100).

Beispiel 7.6.1.a

Eine Lösung von 800 mg (1.66 mMol) (S)-2-(tert.Butoxycarbonylamino)-8-[2-(prop-2-enyloxycarbonyl)-phenoxy]-1,2,3,4-tetrahydronaphthalin-2-carbonsäure-methylester in 3.5 ml Dichlormethan wurde in Analogie zu Beispiel 7.1.1.a bei 0° mit 6 ml Trifluoressigsäure umgesetzt, worauf das Produkt mit 660 mg (2.49 mMol) Boc-L-phenylalanin, 477 mg (2.49 mMol) N-Aethyl-N'-(3-dimethylaminopropyl)-carbodiimid-hydrochlorid, 673 mg (4.98 mMol) 1-Hydroxybenzotriazol und 710 µl (4.15 mMol) Ethyldiisopropylamin in 5 ml N,N-Dimethylformamid umgesetzt wurde. Nach Chromatographie an 150 g Kieselgel mit Essigester/Hexan (7:3→1:1) und nach Trocknen am Hochvakuum erhielt man 826 mg (79%) (S)-2-N-(tert.-Butoxycarbonyl-L-phenylalanyl)-8-[2-(prop-2-enyloxycarbonyl)-phenoxy]-1,2,3,4-tetrahydronaphthalin-2-carbonsäure-methylester als amorphen Festkörper. $[\alpha]_D$ = +52.6° (c = 0.3, Chloroform). Smp. 65-75°. MS (ISP): 646.5 ($M^{+\bullet}$+$NH_4^+$ , 40), 629.5 ($M^{+\bullet}$+$H^+$, 50), 315.8 (100).

Beispiel 7.6.2.a

In Analogie zu Beispiel 7.1.2.a wurden zu einer Lösung von 556 mg (0.88 mMol) (S)-2-N-(tert.Butoxycarbonyl-L-phenylalanyl)-8-[2-(prop-2-enyloxycarbonyl)-phenoxy]-1,2,3,4-tetrahydro-naphthalin-2-carbonsäuremethylester in 6 ml Dichlormethan 4 ml Trifluoressigsäure zugegeben, und der nach dem ersten Schritt erhaltene Rückstand wurde mit 488 mg (1.32 mMol) Z-L-Tyrosin-tert.butylether, 252.5 mg (1.32 mMol) N-Aethyl-N'-(3-dimethylaminopropyl)-carbodiimid-hydrochlorid, 270 mg (2.0 mMol) 1-Hydroxybenzotriazol und 456 µl (2.67 mMol) Ethyldiisopropylamin in 6 ml N,N-Dimethylformamid umgesetzt. Nach Chromatographie an 200 g Kieselgel mit Essigester/Hexan 3:7→1:1) wurden 683 mg (86.7%) (S)-2-N-[(tert.Butoxycarbonyl-L-(O-tert.butyl)tyrosyl)-L-phenylalanyl)-8-[2-(prop-2-enyloxycarbonyl)-phenoxy]-1,2,3,4-tetrahydro-naphthalin-2-carbonsäuremethylester als amorpher Festkörper erhalten. $[\alpha]_D$ = +34.7° (c = 0.3, Chloroform). Smp. 80-90° (Sintern). MS (ISP): 882.7 ($M^{+\cdot}$+$H^+$, 80), 470.0 (100), 461.2 (70).

Beispiel 7.6.3.a

In Analogie zu Beispiel 7.4.1.3.a wurde zu 6 ml eines Gemischs von Dimethylsulfoxid, Tetrahydrofuran und 0.5N Salzsäure (2:2:1) N-Methylanilin solange zugegeben, bis das pH der Lösung 7 erreichte, worauf die Lösung mit 212 mg (0.24 mMol) (S)-2-N-[(tert.Butoxycarbonyl-L-(O-tert.butyl)tyrosyl)-L-phenylalanyl]-8-[2-(prop-2-enyloxycarbonyl)phenoxy)-1,2,3,4-tetrahydro-naphthalin-2-carbonsäure-methylester und 41 mg (0.036 mMol) Tetrakis(triphenylphosphin)palladium versetzt wurde. Der Rückstand wurde an 30 g Kieselgel mit Chloroform/Methanol (95:5→92:8) chromatographiert, worauf nach Trocknung am Hochvakuum 188 mg (93%) (S)-2-N-[(tert.Butoxycarbonyl-L-(O-tert.butyl) tyrosyl)-L-phenylalanyl]-8-[2-carboxyphenoxy]-1,2,3,4-tetrahydro-naphthalin-2-carbonsäure-methylester als amorpher Festkörper erhalten wurden. $[\alpha]_D$ = +18.3° (c = 0.3, Chloroform). Smp. (Zers.) >110°. MS (FAB): 842.2 ($M^{+\bullet}$+$H^+$, 40), 708.2 (10), 342.0 (60), 324 (30), 282.0 (40), 216.9 (40), 120.1 (40), 91.1 (100).

Beispiel 7.6.4.a

In Analogie zum zweiten Teil von Beispiel 7.1.3.a wurden 164 mg (0.195 mMol) (S)-2-N-[(tert.Butoxycarbonyl-L-(O-tert.butyl)tyrosyl)-L-phenylalanyl]-8-[2-carboxy-phenoxy]-1,2,3,4-tetrahydro-naphthalin-2-carbonsäure-methylester in 3 ml Dichlormethan mit 56 mg (0.29 mMol) N-Aethyl-N'-(3-dimethylaminopropyl)carbodiimid-hydrochlorid und 53.8 mg (0.29 mMol) Pentafluorphenol umgesetzt. Nach Chromatographie an 45 g Kieselgel mit Chloroform und nach Trocknung am Hochvakuum erhielt man 175 mg (89%) (S)-2-N-[(tert.Butoxycarbonyl-L-(O-tert.butyl)tyrosyl)-L-phenylalanyl]-8-(2-pentafluorphenyloxycarbonylphenoxy)-1,2,3,4-tetrahydro-naphthalin-2-carbonsäure-methylester als amorphen Festkörper. $[\alpha]_D$ = +21.0° (c = 0.2, Chloroform). Smp. 90-98° (Sintern). MS (ISP): 1008.6 ($M^{+\bullet}$ +$H^+$, 30), 532.8 (100).

Beispiel 7.6.5.a

Zu einer Lösung von 90 ml Dioxan, 1.8 ml Ethanol, 40 mg (0.27 mMol) 4-Pyrrolidinopyridin und 319 mg Palladium-Kohle (10%) wurden unter Argon bei 90° innerhalb von 2 Stunden eine Lösung von 92 mg (0.091 mMol) (S)-2-N-[(tert.Butoxycarbonyl-L-(O-tert.butyl)tyrosyl)-L-phenylalanyl]-8-(2-pentafluorphenyloxycarbonyl-phenoxy)-1,2,3,4-tetrahydro-naphthalin-2-carbonsäure-methylester und 8.1 ml Cyclohexen zugetropft. Das Reaktionsgemisch wurde noch 30 Minuten bei 90° gerührt, abgekühlt und über Celite filtriert. Das Filtrat wurde eingeengt, und der Rückstand wurde in Eiswasser und Dichlormethan aufgenommen. Danach wurde das pH mittels 1N Salzsäure auf 3 gestellt, worauf die wässrige Phase abgetrennt und danach mit Dichlormethan extrahiert wurde und die vereinigten organischen Phasen mit gesättigter Natriumcarbonat-Lösung und gesättigter Kochsalzlösung gewaschen, über Natriumsulfat getrocknet und eingeengt wurden. Der Rückstand wurde an 15 g Kieselgel mit Essigester/Hexan (3:7→3:2) chromatographiert, worauf nach Trocknung am Hochvakuum 38 mg (60%) (12S,15S,18S)-15-Benzyl-12-(4-tert.butoxybenzyl)-10,13,16-trioxo-10,11,12,13,14,15,16,17,18,19,20,21-dodecahydro-1,18-etheno-dibenz[b,n]-[1,5,8,11]-oxatriazocyclopentadecin-18-carbonsäure-methyester als weisses Pulver erhalten wurden.MS (ISP): 707.5 ($M^{+\bullet}$+$NH_4^+$ , 40), 690.4 ($M^{+\bullet}$+$H^+$, 100). IR (KBr): 3416s(br.), 3030w, 2977w, 2950w, 1738m, 1655s, 1537m, 1508s, 1461m, 1237s, 1162w.

Beispiel 7.6.6.a

Zu einer Lösung von 34 mg (0.05 mMol) (12S,15S,18S)-15-Benzyl-12-(4-tert.butoxybenzyl)-10,13,16-trioxo-10,11,12,13,14,15,16,17,18,19,20,21-dodecahydro-1,18-etheno-dibenz[b,n][1,5,8,11]-oxatriazocyclopentadecin-18-carbonsäure-methyester in 1.5 ml Tetrahydrofuran, Methanol und Wasser (3:1:1) wurden 32 mg Lithiumhydroxyd-Monohydrat zugegeben, wonach in Analogie zu Beispiel 7.1.5.a vorgegangen wurde. Das Rohprodukt wurde 2 x mit Diethylether/Hexan (1:1) gewaschen und am Hochvakuum getrocknet, worauf 29 mg (89%) (12S,15S,18S)-15-Benzyl-18-(4-tert.butoxybenzyl)-10,13,16-trioxo-10,11,12,13,14,15,16,17,18,19,20,21-dodecahydro-1,18-etheno-dibenz[b,n][1,5,8,11]oxatriazacyclopentadecin-18-carbonsäure als weisses Pulver erhalten wurden. Smp. (Zers.) >170°. MS (ISP): 676.4 ($M^{+\bullet}$+$H^+$, 75), 409.2 (25), 388 (100).

Beispiel 8.1.a

Zu einer Lösung von 21.0 mg (0.034 mMol) (12S,15S,18S)-12,15-Dimethyl-10,13,16-trioxo-10,11,12,13,14,15,16,17,18,19,20,21-dodecahydro-1,18-etheno-dibenz[b,n]oxatriazacyclopentadecin-18-carbonxäure, 45 mg (0.044 mMol) L-Alanyl-L-alanyl-L-alanyl-L-alanyl-L-alanyl-L-alanyl-(2-amino-2-methylpropionyl)-L-alanyl-L-alanin-(4-jodo-phenyl)-amid-trifluoracetat [C.E. Spiegler, Synthesis and conformational studies of peptides containing novel $\alpha,\alpha$-disubstituted amino acids, Dissertation, Zürich (Universität) 1993], 98 mg (0.0513 mMol) N-Ethyl-N'-(3-dimethylaminopropyl)-carbodiimidhydrochlorid und 102.7 mg (0.075 mMol) 1-Hydroxybenzotriazol in 0.5 ml N,N-Dimethylformamid wurden bei 0° unter Argon 17.6 $\mu$l (0.102 mMol) N-Aethyldiisopropylamin zugegeben. Das Reaktionsgemisch wurde 30 Minuten bei 0° und 4 Stunden bei Raumtemperatur gerührt und dann auf eine Mischung von Eis, 0.5N HCl und Chloroform/Methanol (9:1) gegossen. Die wässrige Phase wurde abgetrennt und erschöpfend mit Chloroform/Methanol (9:1) extrahiert. Die vereinigten organischen Phasen wurden über $Na_2SO_4$ getrocknet und eingedampft. Der Rückstand wurde an 10 g Kieselgel mit Chloroform/Methanol (6:1) vorgereinigt. Das erhaltene Produkt (ca 45 mg) wurde mittels präparativer HPLC (10 Nucleosil 7$C_{18}$ 250/1/2, Macherey Nagel) chromatographiert, worauf nach Lyophilisieren und Trocken am Hochvakuum 40 mg (91.8%) [(12S,15S,18S)-12,15-Dimethyl-10,13,16-trioxo-10,11,12,13,14,15,16,17,18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid erhalten wurden. MS (ISP): 1307.6 ($M^{+\bullet}$ + $H^+$, 5), 673 (100).

Beispiel 8.1.b

In Analogie zu Beispiel 8.1.a wurden aus 21.7 mg (0.048 mMol) (12R,15R,18R)-12,15-Dimethyl-10,13,16-trioxo-10,11,12,13,14,15,16,17,18, 19,20,21-dodecahydro-1,18-etheno-dibenz[b,n]-oxatriazacyclopentadecin-18-carbonsäure, 71.0 mg (0.072 mMol) L-Alanyl-L-alanyl-L-alanyl-L-alanyl-L-alanyl-L-alanyl-(2-amino-2-methyl-propionyl)-L-alanyl-L-alanin-(4-jodophenyl)-amid-trifluoracetat, 13.8 mg (0.072 mMol) N-Aethyl-N'-(3-dimethylaminopropyl)-carbodiimid-hydrochlorid und 14.3 mg (0.105 mMol) 1-Hydroxybenzotriazol in 0,5 ml N,N-Dimethylformamid nach Lyophilisieren und Trocknen 55 mg (87.7%) [(12R,15R,18R)-12,15-Dimethyl-10,13,16-trioxo-10,11,12,13,14,15,16,-17,18,19,20,21-dodecahydro-1,18-etheno-dibenz[b,n][1,5,8,11]oxatriaza-cyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid erhalten. MS (ISP): 1307.6 ($M^{+\bullet}$ + $H^+$, 10), 673 (100).

Beispiel 8.2.b

a) 5 g Aminomethylpolystyrolharz (Novabiochem, CH-4448 Läufelfingen, Produkt Nr. 01-64-0010) wurden in 50 ml DMF vorgequollen und nacheinander mit 6.8 g Fmoc-2,4-dimethoxy-4'-(carboxymethyloxy)-benzhydrylamin (Bachem AG, CH-4416 Bubendorf, Q 1660), 4.75 g HBTU und 2.13 ml DIPEA versetzt und 18 Stunden bei 20° geschüttelt. Das entstandene Fmoc-Aminoharz wurde abfiltriert, mit DMF, Isopropanol und Diethylether gründlich gewaschen und im Vakuum getrocknet: Ausbeute: 7.9 g.

b) 6 g dieses Fmoc-Aminoharzes (Beladung: ca. 4 mMol) wurden in einen Peptidsynthesizer Labortec SP 650 eingefüllt und nachfolgendem Synthesezyklus unterworfen:

| Stufe | Reagenz | Zeit |
|---|---|---|
| 1 | DMF | 2 x 1 Min. |
| 2 | 20% Piperidin/DMF | 1 x 7 Min. |
| 3 | DMF | 5 x 1 Min. |
| 4 | 2,5 Aeq. Fmoc- bzw. Z.Aminosäure/DMF + 2,5 Aeq. 1-Benzotriazol-1-yl-tetramethyluroniumhexafluorphosphat [HBTU] oder tetrafluorborat [TBTU] + 2,5 Aeq. Diisopropylaethylamin | 1 x 90 Min |
| 5 | DMF | 3 x 1 Min. |
| 6 | Isopropylalkohol | 2 x 1 Min. |

In jeder Stufe wurden ca. 50 ml Lösungsmittel benutzt. Fmoc-Leu-OH, Fmoc-Lys(Boc)-OH, Fmoc-Gln(Trt)-OH- Fmoc-Ala-OH; Fmoc-Ile-OH, Fmoc-Glu(OBut)-OH, Fmoc-Ala-OH, Fmoc-Lys(Boc)-OH und Fmoc-Leu-OH wurden nach obigem Protokoll gekuppelt. Nach Abschluss der Synthese wurde die Fmoc-Schutzgruppe mit 20% Piperidin/DMF, wie in Stufe 2 des Synthezyklus beschrieben, abgespalten, und das Peptidharz wurde im Vakuum getrocknet. Es wurden 9.3 g N-[Leu-Lys(Boc)-Ala-Glu(OBut)-Ile-Ala-Gln(Trt)-Lys-(Boc)-Leu]-Aminoharz erhalten.

d) 300 mg N-[Leu-Lys(Boc)-Ala-Glu(OBut)-Ile-Ala-Gln(Trt)-Lys-(Boc)-Leu]-Aminoharz wurden in 2 ml DMF vorgequollen und nacheinander mit 32 mg (12S,15S,18S)-12,15-Dimethyl-10,13,16-trioxo-10,11,12,13,14,15,16,17,18,19,20,21-dodecahydro-1,18-etheno-dibenz[b,n]oxatriazacyclopentadecin-18-carbonsäure, 32 mg TBTU und 18 µl DIPEA versetzt. Das Reaktionsgemisch wurde 1 Stunde geschüttelt, und das Harz wurde abfiltriert, mit DMF, Isopropanol und Diethyläther gewaschen und in 50 ml einer Lösung bestehend aus 82.5% TFA/5% Phenol/5%Thioanisol/2.5% 1,2-Dimercaptoethanol/5% $H_2O$ [Reagenz K] suspendiert und 2 Stunden bei 20° geschüttelt. Dann wurde das Harz abfiltriert und mit Trifluoressigsäure gewaschen, und die vereinigten Filtrate wurden im Vakuum eingeengt. Der Rückstand wurde mit Diaethyläther digeriert, in verd. Trifluoressigsäure gelöst und mittels HPLC (an einer LiChrospher-Säule RP-18 10 µm (2.5 x 25 cm) im Gradientensystem 0.1% Trifluoressigsäure-Aethanol gereinigt. Nach Lyophilisierung wurden 24.9 mg [(12R,15R,18R)-12,15-Dimethyl-10,13,16-trioxo-10,11,12,13,14,15,-16,17,18,19,20,21-dodecahydro-1,18-etheno-dibenz[b,n][1,5,8,11]-oxatriaza-cyclopentadecin-18-ylcarbonyl]-L-leucyl-L-lysyl-L-alanyl-L-glutamyl-L-isoleucyl-L-alanyl-L-glutaminyl-L-lysyl-L-leucinamid-trifluoracetat (1:2) erhalten. MS (ISP) 1446.0.

Beispiel 8.2.a

In analoger Weise wie in Beispiel 8.2.b beschrieben, jedoch unter Verwendung von (12R,15R,18R)-12,15-Dimethyl-10,13,16-trioxo-10,11,12,13,14,15,16,17,18,19,20,21-dodecahydro-1,18-etheno-dibenz[b,n]-oxatriazacyclopentadecin-18-carbonsäure wurde [(12S,15S,18S)-12,15-Dimethyl-10,13,16-trioxo-10,11,12,13,-14,15,16,17,18,-

19,20,21-dodecahydro-1,18-etheno-dibenz[b,n]1,5,8,11]oxatriaza-cyclopentadecin-18-ylcarbonyl]-L-leucyl-L-lysyl-L-alanyl-L-glutamyl-L-isoleucyl-L-alanyl-L-glutaminyl-L-lysyl-L-leucinamid-trifluoracetat (1:2) synthetisiert. Ausbeute: 26 mg Lyophilisat. MS(FAB) 1445.8 $MH^+$, 100).

Beispiel 8.3.a

In Analogie zu Beispiel 8.1.a wurden 16.0 mg (0.03 mMol) (14aS,17S,20S)-8-Acetylamino-17-methyl-10,15,18-trioxo-11,12,13,14, 14a,15,16,17,18,19,20,21,22,23-tetradecahydro-1,20-etheno-10H-pyrrolo[1,2-e]dibenz [b,n][1,5,8,11]-oxatriazacyclopentadecin-20-carbonsäure mit 44.0 mg (0.045 mMol) L-Alanyl-L-alanyl-L-alanyl-L-alanyl-L-alanyl-L-alanyl-(2-amino-2-methyl-propionyl)-L-alanyl-L-alanin-(-4-jodophenyl)-amidtrifluoracetat, 8.6 mg (0.045 mMol) N-Aethyl-N'-(3-dimethylaminopropyl)-carbodiimid-hydrochlorid, 9.0 mg (0.068 mMol) 1-Hydroxybenzotriazol und 15 µl (0.09 mMol) Aethyldiisopropylamin in 1.5 ml N,N-Dimethylformamid umgesetzt. Das erhaltene Produkt wurde an 6 g Kieselgel mit Chloroform/Methanol (95:5 → 9:1) vorgereinigt. Nach präparativer HPLC-Chromatographie (Säule: Vydac 250-10, Programm: 50% Acetonitril → 85% Acetonitril in 20 Minuten), Lyophilisieren und Trocknen erhielt man 34.8 mg (84%) [(14aS,17S,20S)-8-Acetylamino-17-methyl-10,15,18-trioxo-11,12,13,14,14a, 15,16,17,18,19,20,21,22,23-tetradecahydro-1,20-etheno-10H-pyrrolo[1,2-e]dibenz[b,n][1,5,8,11]-oxatriazacyclopentadecin-20-ylcarbonyl]-L-alanyl-L-alanyl-L-alanyl-L-alanyl-L-alanyl-L-alanyl-(2-amino-2-methylpropionyl)-L-alanyl-L-alanin-(4-iodophenyl)-amid als weissen Feststoff. Smp. (Zers.) >215°, $[\alpha]_D = + 11.0°$ (Chloroform, c = 0.1), MS (ISP): 1411.4 ($M^{+\bullet}$ + $Na^+$, 10), 1389.4 ($M^{+\bullet}$, $H^+$, 10), 714.4 (100).

Beispiel 8.4.a.

In Analogie zu Beispiel 8.1.a. wurden 13.0 mg (24 µMol) (12S,15S,18S)-12-(2-Methoxycarbonyl-aethyl)-15-methyl-10,13,16-trioxo-10,11,12,13,14,15,16,17,18,19, 20,21-dodecahydro-1,18-etheno-pyrido[2,3-b]benz[n] [1,5,8,11]oxatriazacyclo-pentadecin-18-carbonsäure, 7.1 mg (37 µMol) N-Ethyl-N'-(3-dimethyl aminopropyl)-carbodiimid, 7.5 mg (56.0 µMol) 1-Hydroxybenzo-triazol, 36.5 mg (37.0 µMol) L-Alanyl-L-alanyl-L-alanyl-L-alanyl-L-alanyl-L-alanyl-(2-amino-2-methyl-propionyl)-L-alanyl-L-alanin-(4-jodophenyl)-amid-trifluoracetat und 12.3 µl (72.0 µMol) N-Ethyldiisopropylamin in 1.5 ml N,N-Dimethylformamid umgesetzt. Der Rückstand wurde in heissem Acetonitril suspendiert, abfiltriert, mit Acetonitril gewaschen und am Hochvakuum getrocknet, wobei insgesamt 24.4 mg (71%) [(12S, 15S,18S)-12-(2-Methoxycarbonyl-ethyl)-15-methyl-10,13,16-trioxo-10,11,12,13,14,15,16,17,18,19,20,21-dodecahydro-1,18-etheno-pyrido[2,3-b]benz-[n][1,5,8,11]oxatriazacyclo-pentadecin-18-ylcarbonyl]-L-alanyl-L-alanyl-L-alanyl-L-alanyl-L-alanyl-L-alanyl-(2-amino-2-methylpropionyl)-L-alanyl-L-alanin-(4-jodophenyl)-amid als weisses Pulver erhalten wurden. Smp. (Zers.) >310°. MS(ISP): 1401.5 ($M^{+\bullet}$ + $Na^+$, 10), 1996.6 ($M^{+\bullet}$ + $NH_4^+$, 10), 1380.6 ($M^{+\bullet}$, + $H^+$, 10), 718.2 (20), 465.4 (30), 289.4 (65), 267.4 (100).

Beispiel 8.5.a

In Analogie zu Beispiel 8.1.a wurden 15.0 mg (26.0 µMol) (12S,16S,19S)-16-Methyl-10,13,17,23-tetraoxo-10,11,12,13,14,15,16,17,18,19,20,21,22,23-tetradecahydro-1,19-etheno-12,15-metheno-pyrido[2,3-b]benz[o] [1,5,9,12]-oxatriazacyclohexadecin-19-carbonsäure, 7.5 mg (39.0 µMol) N-Aethyl-N'-(3-dimethylaminopropyl)-carbodiimid-hydrochlorid, 7.9 mg (59.0 µMol) 1-Hydroxybenzotriazol, 38.5 mg (39.0 µMol) L-Alanyl-L-alanyl-L-alanyl-L-alanyl-L-alanyl-L-alanyl-(2-amino-2-methyl-propionyl)-L-alanyl-L-alanin-(4-jodophenyl)-amid-trifluoracetat und 13.6 mg (80.0 µMol) Aethyldiisopropylamin in 1.5 ml N,N-Dimethylformamid umgesetzt. Der Rückstand wurde in Chloroform und Wasser aufgenommen, die Wasserphase wurde erschöpfend mit Chloroform extrahiert, und die vereinigten organischen Phasen wurden über Natriumsulfat getrocknet, filtriert und eingeengt. Der feste Rückstand wurde in Acetonitril suspendiert und abfiltriert, worauf nach Trocknen 15.0 mg (43.0%) [(12S,16S,19S)-16-Methyl-10,13,17,23-tetraoxo-10,11,12,13,14,16,17,18,19,20,21,22,23-tetradecahydro-1,19-etheno-12,15-metheno-pyrido[2,3-b]benz[o][1,5,9,12]-oxatriazacyclohexadecin-19-ylcarbonyl]-L-alanyl-L-alanyl-L-alanyl-L-alanyl-L-alanyl-L-alanyl-(2-amino-2-methyl-propionyl)-L-alanyl-L-alanin-(4-jod-phenyl)-amid als weisses Pulver erhalten wurden. Smp. (Zers.) >240°. MS(FAB): 1333.3 ($M^{+\bullet}$ + $H^+$, 60), 1114.4 (70), 1043.3 (40), 972.3 (50), 887.3 (20), 816.3 (20), 745.2 (30), 674.2 (30), 532.0 (60), 433.0 (25), 324.9 (50), 216.9 (90), 91.2 (100).

Beispiel 8.6.a

Ausgehend von einer Lösung von 9.0 mg (13.0 µMol) (12S,15S,18S)-15-Benzyl-18-(4-tert.butoxybenzyl)-10,13,16-trioxo-10,11,12,13,14,15,16,17,18,19, 20,21-dodecahydro-1,18-etheno-dibenz[b,n][1,5,8,11]oxatriazacyclopentadecin-18-carbonsäure, 19.7 mg (19,9 µMol) L-Alanyl-L-alanyl-L-alanyl-L-alanyl-L-alanyl-L-alanyl-(2-amino-2-methylpropionyl)-L-alanyl-L-alanin-(4-jodophenyl)amid-trifluoracetat, 3.8 mg (19.9 µMol) N-Aethyl-N'-(3-dimethyl-

aminopropyl)-carbodiimid-hydrochlorid, 3.5 mg (26.0 µMol) 1-Hydroxybenzotriazol und 4.4 µl (26.0 µMol) Ethyldiisopropylamin in 0.3 ml N,N-Dimethylformamid wurde in Analogie zu Beispiel 8.1.a vorgegangen. Das Rohprodukt wurde an 4 g Kieselgel mit Chloroform/Methanol (95:5) chromatographiert, und das als Festkörper erhaltene Produkt wurde in Diaethylaether suspendiert, abfiltriert und am Hochvakuum getrocknet, worauf 18 mg (89.5%) [(12S,15S,18S)-15-Benzyl-18-(4-tert.butoxybenzyl)-10,13,16-trioxo-10,11,12,13,14,15,16,17,18,19,20,21-dodecahydro-1,18-ethenodibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-jodophenyl)amid erhalten wurden. Smp. (Zers.) >230°. MS (ISP): 1547.8 ($M^{+\bullet}$ + $NH_4^+$ , 100), 1530.6 ($M^{+\bullet}$ +$H^+$, 20), 766 (30).

Beispiel 9.1.1.a

Zu einer Lösung von 48.2 mg (63.8 µMol) (S)-2-(Fluoren-9-ylmethoxycarbonyl)-8-[2-(prop-2-enyloxycarbonyl)-phenoxy]-1,2,3,4-tetrahydronaphthalin-2-carbonsäure-pentafluorphenylester und 60.0 mg (60.8 µMol) L-Alanyl-L-alanyl-L-alanyl-L-alanyl-L-alanyl-L-alanyl-(2-amino-2-methylpropionyl)-L-alanyl-L-alanin-(4-iodophenyl)-amid-trifluoracetat in 1 ml N,N-Dimethylformamid wurden 10.7 mg Natriumbicarbonat zugegeben. Das Reaktionsgemisch wurde während 4 Stunden bei Raumtemperatur gerührt und dann auf eine Mischung von Wasser und Essigsäureaethylester gegossen. Die organische Phase wurde abgetrennt, mit gesättigten Natriumbicarbonat und Kochsalzlösungen gewaschen, über Natriumsulfat getrocknet und eingedampft. Der Rückstand wurde aus Diaethylaether/Hexan gefällt und am Hochvakuum getrocknet, worauf 56.2 mg (61%) [(S)-[2-(Fluoren-9-ylmethoxycarbonylamino)-8-[2-[(prop-2-enyloxycarbonyl)-phenoxy]]-1,2,3,4-tetrahydro-naphthalin-2-ylcarbonyl]-L-alanyl-L-alanyl-L-alanyl-L-alanyl-L-alanyl-L-alanyl-(2-amino-2-methylpropionyl)-L-alanyl-L-alanin (4-iodophenyl)-amid als weisses Pulver erhalten wurden. MS (ISP): 682.3 ($M^{+\bullet}$ + $Na^+$, 25), 660.4 ($M^+$ + $H^+$, 100), 441.4(35). IR(KBr): 3325m(br), 3065w, 2983w, 2938w, 1662s, 1534s, 1453m, 1382w, 1300w, 1243m, 1081w.

Beispiel 9.1.2.a

Eine Lösung von 32 mg (22.2 µMol) [(S)-[2-(Fluoren-9-ylmethoxycarbonylamino)-8-[2-[(prop-2-enyloxy-carbonyl)-phenoxy]]-1,2,3,4-tetrahydronaphthalin-2-ylcarbonyl]-L-alanyl-L-alanyl-L-alanyl-L-alanyl-L-alanyl-L-alanyl-(2-amino-2-methylpropionyl)-L-alanyl-L-alanin-(4-iodophenyl)-amid in 1 ml 20% Piperidin/N,N-Dimethylformamid wurde während 20 Stunden bei Raumtemperatur gerührt und dann eingedampft. Der Rückstand wurde aus Diethylether gefällt, abfiltriert, mit Diethylether gewaschen und am Hochvakuum getrocknet. Das erhaltene rohe Produkt (27 mg) wurde in 0.5 ml N,N-Dimethylformamid gelöst, worauf die Lösung mit 6.4 mg (33.3 µMol) N-Aethyl-N'-(3-dimethylaminopropyl)-carbodiimid-hydrochlorid, 6.6 mg (48.8 µMol) 1-Hydroxybenzotriazol, 6.3 mg (33.3 µMol) Boc-L-Alanin und 11.3 µl Ethyldiisopropylamin versetzt, während 17 Stunden bei Raumtemperatur unter Argon gerührt und dann auf eine Mischung von Wasser und Chloroform gegossen wurde. Die organische Phase wurde abgetrennt, mit gesättigter Natriumbicarbonatlösung extrahiert, über $Na_2SO_4$ getrocknet und eingeengt. Der Rückstand wurde mit Hexan gefällt, abfiltriert und am Hochvakuum getrocknet. Das rohe Produkt ( 30 mg) wurde mit präparativer HPLC-Chromatographie (Säule: Vydac $C_{18}$, Programm: 50% Acetonitril $\rightarrow$ 100% Acetonitril in 30 Minuten) chromatographiert, worauf nach Lyophilisieren und Trocknen, 26.2 mg (90%) [(S)-[2-[N-(tert.-Butoxycarbonyl-L-alanyl)]-8-[2-[(prop-2-enyloxycarbonyl)-phenoxy]]-1,2,3,4-tetrahydro-naphthalin-2-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid erhalten wurden. MS(ISP): 1410.6 ($M^{+\bullet}$ + $Na^+$, 15), 1393.4 ($M^{+\bullet}$ + $H^+$, 10), 716.6 (100), 538 (20). IR(KBr): 3324 m(br), 3065w, 2982w, 2937w, 1663s, 1534s, 1455w, 1382w, 1302w, 1247w, 1165w.

Beispiel 9.1.3.a

Eine Lösung von 24.0 mg (17.2 µMol) [(S)-[2[N-(tert.-Butoxycarbonyl-L-alanyl)]-8-[2-[(prop-2-enyloxycarbonyl)-phenoxy]]-1,2,3,4-tetrahydronaphthalin-2-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid in 0.5 ml Dichlormethan wurde unter Eiskühlung mit 0.5 ml Trifluoressigsäure versetzt, worauf das Gemisch 28 Stunden bei Raumtemperatur gerührt und dann eingeengt wurde. Der Rückstand wurde aus Diethylether/Hexan (1:1) gefällt, abfiltriert, am Hochvakuum getrocknet und in 0.5 ml N,N-Dimethylformamid gelöst. Die Lösung wurde bei Raumtemperatur unter Argon mit 4.95 mg (25.8 µMol) N-Aethyl-N'-(3-dimethylaminopropyl)-carbodiimid-hydrochlorid, 5.1 mg (37.8 µMol) 1-Hydroxybenzotriazol, 4.5 mg (25.8 µMol) Alloc-L-Alanin und 8.8 µl (51.6 µMol) N-Aethyldiisopropylamin versetzt, worauf der Gemisch während 6 Stunden bei Raumtemperatur gerührt wurde. Nach Aufarbeitung und präparativer HPLC-Chromatographie in Analogie zu Beispiel 9.1.2.a erhielt man 19.8 mg (80.2%) [(S)-[2-[N-[[(Prop-2-enyloxycarbonyl)-L-alanyl]-L-alanyl]]-8-[2-[(prop-2-enyloxycarbonyl)-phenoxy]]-1,2,3,4-tetrahydronaphthalin-2-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid als weisses Pulver. MS(ISP): 1448.6 ($M^{+\bullet}$

+ $H^+$, 10), 744.0 (100). IR(KBr): 3320m (br), 3065w, 2984w, 2938w, 1661s, 1537s, 1455m, 1382w, 1302w, 1243m, 1166w.

Beispiel 9.1.4.a

6.8 mg Tetrakis(triphenylphosphin)palladium wurden zu einem Gemisch von 110 µl N-Methyl-anilin und 600 µl Dimethylsulfoxid gegeben. Dieses Gemisch wurde bei Raumtemperatur gerührt und mit einer Lösung von 17.8 mg (12.3 µMol) [(S)-[2-[N-[[(Prop-2-enyloxycarbonyl)-L-alanyl]-L-alanyl]]-8-[2-[(prop-2-enyloxycarbonyl)-phenoxy]]-1,2,3,4-tetrahydronaphthalin-2-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid in 1.5 ml Tetrahydrofuran/Dimethylsulfoxid/0.5N HCl (2:2:1) versetzt worauf das Reaktionsgemisch 20 Stunden bei Raumtemperatur gerührt und dann eingeengt wurde. Der Rückstand wurde mittels präparativer HPLC-Chromatographie (Säule: Vydac $C_{18}$; Programm: 30% Acetonitril → 100% Acetonitril in 30 Minuten) gereinigt, worauf nach Lyophilisieren und Trocknen 17.0 mg (96%) [(S)[-[2-[N-(L-Alanyl-L-alanyl)]-8-(2-carboxyphenoxy)-5,6,7,8-tetrahydro-naphthalin-2-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid als weisses Pulver erhalten wurden. MS(ISP): 1324.4 ($M^{+\cdot}$ + $H^+$, 40), 663.0 (75), 553.6 (100), 518 (30), 455 (25). IR(KBr): 3318 m (br), 3060w, 2985w, 2938w, 1666s, 1532s, 1456m, 1383w, 1243m, 1202m.

Beispiel 9.1.5.a

Zu einer Suspension von 4.4 mg (3.1 µMol) [(S)-[2-[N-(L-Alanyl-L-alanyl)]-8-(2-carboxy-phenoxy)-5,6,7,8-tetrahydro-naphthalin-2-ylcarbonyl]-L-alanyl-L-alanyl-L-alanyl-L-alanyl-L-alanyl-L-alanyl-(2-amino-2-methylpropionyl)-L-alanyl-L-alanin-(4-jodophenyl)-amid und 1.3 mg Natriumcarbonat in 10 ml N,N-Dimethylformamid wurden bei Raumtemperatur und unter Argon 2.3 µl Diphenylphosphorylazid zugegeben, worauf das Reaktionsgemisch 20 Stunden gerührt und dann eingeengt wurde. Der Rückstand wurde in Acetonitril aufgenommen und mittels präparativer HPLC-Chromatographie (Säule: Vydac $C_{18}$; Programm: 30% Acetonitril → 100% Acetonitril in 30 Minuten) gereinigt, worauf nach Lyophilisieren und Trocknen 2.7 mg (67%) [(12S,15S,18S)-12,15-Dimethyl-10,13,16-trioxo-10,11,12,13,14,15,16,17,18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methylpropionyl)-L-alanyl-L-alanin-(4-iodophenyl)-amid als weisses Pulver erhalten wurden. Identisch mit Produkt aus Beispiel 8.1.a.

Beispiel 9.2.a.

Zu einem Gemisch von 450 mg (ca. 0.11 mMol) das gemäss Absatz d) von Beispiel 8.2.b. erhaltenen Peptidharzes in 2.3 ml N,N-Dimethylformamid wurden 41 mg (0.12 mMol) (S)-2-(Fluoren-9-ylmethoxycarbonyl)-8-[2-(prop-2-enyloxycarbonyl)-phenoxy]-1,2,3,4-tetrahydro-naphthalin-2-carbonsäure-pentafluorphenylester zugegeben. Das Reaktionsgemisch wurde während 18 Stunden in einem Peptidsynthesizer Labortec SP 650 geschüttelt, dreimal mit 10 ml N,N-Dimethylformamid gewaschen und während 20 Stunden mit 10 ml 20%-iger Piperidinlösung in N,N-Dimethylformamid behandelt. Zum Harz wurden 137 mg (0.44 mMol) Fmoc-L-Alanin, 91 mg (0.40 mMol) N-Ethyl-N'-(3-dimethylaminopropyl)-carbodiimid-hydrochlorid, 4.9 mg (0.04 mMol) N-Hydroxypyridin-2-on und 2.5 ml N,N-Dimethylformamid gegeben, worauf 2 Stunden geschüttelt wurde. Danach wurde das Harz abfiltriert und der Kopplungsschritt mit Fmoc-L-Alanin nochmals wiederholt, mit dem Unterschied, dass 18 Stunden geschüttelt wurde. Das Harz wurde mit N,N-Dimethylformamid gewaschen, worauf die Fmoc-Gruppe durch dreimalige Behandlung mit 20%-iger Piperidinlösung in N,N-Dimethylformamid während je 45 Minuten abgespalten und das Harz analog wie oben beschrieben gewaschen wurde. Zum Harz wurden anschliessend 76 mg (0.44 mMol) Alloc-L-Alanin, 91 mg (0.40 mMol) N-Ethyl-N'-(3-dimethylaminopropyl)carbodiimidhydrochlorid, 4.9 mg (0.04 mMol) N-Hydroxypyridin-2-on und 2.5 ml N,N-Dimethylformamid gegeben, worauf 20 Stunden geschüttelt und mit N,N-Dimethylformamid gewaschen wurde. Die Kopplung mit Alloc-L-Alanin wurde analog zur Kopplung mit Fmoc-L-Alanin wiederholt, worauf das Harz mit N,N-Dimethylformamid gewaschen und getrocknet wurde. Zum getrockneten Harz wurden 3.5 ml einer Lösung von Dimethylsulfoxid, Tetrahydrofuran und 0.5 N Salzsäure (2:2:1) und 350 µl N-Methylanilin zugegeben, worauf das Gemisch mit Argon sauerstoffrei gemacht und mit 19.0 mg (16.5 µMol) Tetrakis-(Triphenylphosphin)palladium versetzt wurde. Das Reaktionsgemisch wurde während 22 Stunden unter Argon geschüttelt und dann mit N,N-Dimethylformamid gewaschen. Diese Prozedur wurde nochmals wiederholt. Auschliessend wurden zum Harz 125 mg (0.33 mMol) 1-Benzotriazol-1-yl-tetramethyluronium-hexafluorophosphat (HBTU), 94 µl (0.55 mMol) Ethyldiisopropylamin und 1.5 ml N,N-Dimethylformamid gegeben, worauf das Gemisch 2 Stunden geschüttelt, filtriert und nacheinander mit N,N-Dimethylformamid, Isopropanol und Diaethylaether gewaschen und getrocknet wurde. Das Peptid-Harz wurde anschliessend durch 2-stündige Behandlung mit einer Lösung von Trifluoressigsäure/Wasser (95:5) gespalten, worauf filtriert und das Filtrat eingedampft wurde. Der Rückstand wurde mit Diethylaether gefällt und mittels präparativer HPLC-Chromatographie

(Säule: Lichrosorb Select B, 10 µm (250 x 10 mm); Programm: 30% Acetonitril → 50% Acetonitril in 20 Minuten; Retentionszeit des Produktes 11.8 Minuten) gereinigt, worauf 8.5 mg reines (12S,15S,18S)-12,15-Dimethyl-10,13,16-trioxo-10,11,12,13,14,15,16,17,18,19,20,21-dodecahydro-1,18-etheno-dibenz[b,n][1,5,8,11]oxatriazacyclopentadecin-18-ylcarbonyl]-L-leucyl-L-lysyl-L-alanyl-L-glutamyl-L-isoleucyl-L-alanyl-L-glutaminyl-L-lysyl-L-leucinamid erhalten wurden. MS (FAB): 1445.5 ($M^{+\bullet}$) (Identisch mit Produkt aus Beispiel 8.2.a.).

Beispiel 10.1.a

Zu einer Lösung von 10.0 mg (7,2 µMol) [(12S,15S,18S)-12-(2-Methoxycarbonyl-aethyl)-15-methyl-10,13,16-trioxo-10,11,12,13,14,15,16,17,18, 19,20,21-dodecahydro-1,18-etheno-pyrido[2,3-b]benz[n][1,5,8,11]oxatriazacyclopentadecin-18-carbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid in 1 ml Tetrahydrofuran/Methanol/Wasser (3:1:1) wurden unter Eiskühlung ca 5 mg LiOH• 1 $H_2O$ zugegeben. Das Reaktionsgemisch wurde 1 Stunde gerührt und dann eingeengt. Der Rückstand wurde in Acetonitril/Wasser (1:1) aufgenommen, das pH wurde mit 1N HCl auf ca. 4 gestellt, worauf lyophilisiert wurde. Der Rückstand wurde mittels HPLC-Chromatographie (Säule: Select B 250-10; Programm: 5% Acetonitril → 100% Acetonitril in 20 Minuten) gereinigt, worauf nach Lyophilisieren und Trocknen 9.1 mg (94.3%) [(12S,15S,18S)-12-(2-Carboxy-ethyl)-15-methyl-10,13,16-trioxo-10,11,12,13,14,15,16,17,18,19,20,21-dodecahydro-1,18-etheno-pyrido [2,3-b]benz[n][1,5,8,11]oxatriazacyclopentadecin-18-ylcarbonyl]-L-alanyl-L-alanyl-L-alanyl-L-alanyl-L-alanyl-L-alanyl-(2-amino-2-methylpropionyl)-L-alanyl-L-alanin-(4-iodophenyl)-amid als weisser fester Rückstand erhalten wurden. MS(ISN): 681.4 [$(M^{+\bullet} - 2H)^{2-\cdot}$ 0.5, 100].

Beispiel 10.2.a.

Zu einer Lösung von 10.0 mg (7.50 µMol) [(12S,16S,19S)-16-Methyl-10,13,17,23-tetraoxo-10,11,12,13,14,15,16,17,18,19,20,21,22,23-tetradecahydro-1,19-etheno-12,15-metheno-pyrido[2,3-b]benz[o][1,5,9,12]oxatriazacyclohexadecin-19-ylcarbonyl]-L-alanyl-L-alanyl-L-alanyl-L-alanyl-L-alanyl-L-alanyl-(2-amino-2-methyl-propionyl)-L-alanyl-L-alanyl-(4-iodophenyl)amid in 0.5 ml Tetrahydrofuran, Methanol und Wasser (3:1:1) wurden unter Eiskühlung 5 mg Lithiumhydroxid-monohydrat zugegeben, und das Reaktionsgemisch wurde 2 Stunden bei 0° und 15 Minuten bei Raumtemperatur gerührt. Danach wurde das pH der Lösung mittels 1 N Salzsäurelösung auf ca 3 gestellt, die Lösung wurde eingeengt, und der Rückstand wurde mittels präparativer HPLC (Säule: Select B 250-10, Programm: 10% Acetonitril → 95% Acetonitril in 20 Minuten) gereinigt. Nach Lyophilisieren und Trocknen wurden 9.5 mg (93.3%) [(12S,15S,18S)-12-(2-Carboxymethyl)-15-methyl-10,13,16-trioxo-10,11,12,13,14,15,16,17,18,19,20,21-dodecahydro-1,18-etheno-pyrido[2,3-b]benz[n][1,5,8]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanyl-(4-iodophenyl)amid als weisses amorphes Pulver erhalten. MS (ISP): 1351.4 ($M^{+\bullet}$ + $H^+$, 20), 704.4 (40), 695.4 (100), 487.4(20), 465.4 (20).

Beispiel 10.3.a

Zu einer Lösung von 15.0 mg (9.8 µMol) [(12S,15S,18S)-15-Benzyl-18-(4-tert.butoxybenzyl)-10,13,16-trioxo-10,11,12,13,14,15,16,17,18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)amid in 0.8 ml Dichlormethan wurden unter Argon bei 0° 0.2 ml Trifluoressigsäure zugegeben. Das Reaktionsgemisch wurde 2 Stunden bei 0° gerührt, worauf das Lösungsmittel am Hochvakuum abdestilliert wurde. Der feste Rückstand wurde 2 x mit Diethylaether gewaschen, worauf 13.0 mg (89.9%) [(12S,15S,18S)-15-Benzyl-18-(4-hydroxybenzyl)-10,13,16-trioxo-10,11,12,13,14, 15,16,17,18,19,20,21-dodecahydro-1,18-etheno-dibenz[b,n][1,5,8,11]oxatriazacyclopentadecin 18-ylcarbonyl]-L-alanyl-L-alanyl-L-alanyl-L-alanyl-alanyl-L-alanyl-(2-amino-2-methyl-propionyl)-L-alanyl-L-alanin-(4-iodophenyl)amid als weisses Pulver erhalten wurden. Smp. (Zers.) >215°. MS (ISP): 1513.6 ($M^{+\cdot}$ + $K^+$, 30), 1497.5 ($M^{+\bullet}$ + $Na^+$, 100). IR (KBr): 3311m(br.), 3070w, 2990w, 2945w, 1691s, 1587w, 1532s, 1456w, 1305w, 1241w.

## Patentansprüche

1. Tetrahydronaphthalinderivate der allgemeinen Formeln

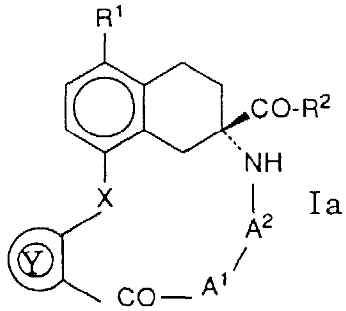

Ia und

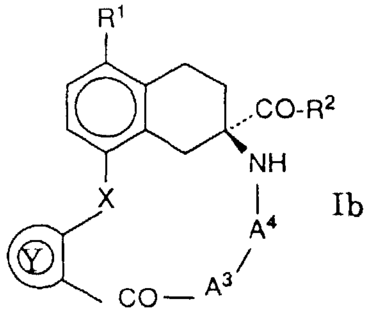

Ib

worin

| | |
|---|---|
| $R^1$ | Wasserstoff, Brom, Cyano, Formyl, Hydroxy, niederes Alkyl, niederes Alkenyl, niederes Alkinyl, niederes Alkoxy, Aryloxy, niederes Aralkoxy oder Aryl; |
| $R^2$ | eine gegebenenfalls geschützte Kette von bis zu 20 Aminosäureresten, deren C-terminale Aminosäure als freie oder geschützte Säure oder in Form eines Amids vorliegen kann; |
| $A^1$, $A^2$, $A^3$ und $A^4$ | je Reste von α-Aminosäuren, deren α-C-Atom, wenn es unsymmetrisch ist, in $A^1$ und $A^2$ in der L-Konfiguration und in $A^3$ und $A^4$ in der D-Konfiguration vorliegt; |
| X | Sauerstoff oder Schwefel; und |
| Y | zusammen mit den beiden C-Atomen einen gegebenenfalls substituierten Benzol-, Furan-, Thiophen-, Pyridin- oder Pyrimidinring bedeuten; |

sowie Salze davon.

**2.** Verbindungen nach Anspruch 1, worin $R^1$ in der Bedeutungsmöglichkeit "Aryl" durch niederes Alkyl oder niederes Alkoxy mono- oder disubstituiertes oder durch niederes Alkylendioxy substituiertes Phenyl bedeutet.

**3.** Verbindungen nach Anspruch 1 oder 2, worin $R^2$ 9 bis 15 Aminosäurereste enthält.

**4.** Verbindungen nach Anspruch 3, worin $R^2$ 9, 12 oder 15 Aminosäurereste enthält.

**5.** Verbindungen nach einem der Ansprüche 1 bis 4, worin $R^2$ Reste von α-Aminosäuren enthält, deren α-C-Atom, wenn es asymmetrisch ist, in der L-Konfiguration vorliegt.

**6.** Verbindungen nach Anspruch 5, worin $R^2$ Reste von L-Alanin, L-Glutamin, L-Glutaminsäure, L-Isoleucin, L-Leucin, L-Lysin oder 2-Amino-2-methylpropionsäure enthält.

**7.** Verbindungen nach einem der Ansprüche 1 bis 6, worin $A^1$ bzw. $A^3$ einen Rest von L- bzw. D-Alanin, Asparagin, Asparaginsäure, Glutamin, Glutaminsäure oder Lysin oder von 2-Amino-2-methylpropionsäure und $A^2$ bzw. $A^4$ einen Rest von L- bzw. D-Alanin, Asparagin, Asparaginsäure, Glutamin, Glutaminsäure oder Lysin bedeuten.

**8.** Verbindungen nach Anspruch 7, worin $A^1$ bzw. $A^3$ L- bzw. D-Alanyl oder Lysyl und $A^2$ bzw. $A^4$ L- bzw. D-Alanyl bedeuten.

**9.** Verbindungen nach einem der Ansprüche 1 bis 8, worin der durch Y bezeichnete Benzol-, Furan-, Thiophen-, Pyridin- oder Pyrimidinring unsubstituiert ist oder durch Nitro, Amino, niederes Alkanoylamino, Hydroxy, niederes Alkoxy, niederes Alkanoyloxy, niederes Alkyl, Fluor, Cyano, Carboxy oder Formyl mono- oder disubstituiert ist oder mit einem Benzolring zu einem bicyclischen System kondensiert ist.

**10.** [(12S,15S,18S)-12,15-Dimethyl-10,13,16-trioxo-10,11,12,13,14,15, 16,17, 18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid;

[(12R,15R,18R)-12,15-Dimethyl-10,13,16-trioxo-10,11,12,13,14,15,16, 17,18,19,20,21-dodecahydro-1,18-etheno-dibenz[b,n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid;
[(12S,15S,18S)-12,15-Dimethyl-10,13,16-trioxo-10,11,12,13,-14,15,16,17,18, 19,20,21-dodecahydro-1,18-etheno-dibenz[b,n][1,5,8,11]oxatriaza-cyclopentadecin-18-ylcarbonyl]-L-leucyl-L-lysyl-L-alanyl-L-glutamyl-L-isoleucyl-L-alanyl-L-glutaminyl-L-lysyl-L-leucinamid trifluoracetat;
[(12R,15R,18R)-12,15-Dimethyl-10,13,16-trioxo-10,11,12,13,14,15,16, 17,18,19,20,21-dodecahydro-1,18-etheno-dibenz[b,n][1,5,8,11]-oxatriazacyclopentadecin-18-ylcarbonyl]-L-leucyl-L-lysyl-L-alanyl-L-glutamyl-L-isoleucyl-L-alanyl-L-glutaminyl-L-lysyl-L-leucinamid trifluoracetat;
[(14aS,17S,20S)-8-Acetylamino-17-methyl-10,15,18-trioxo-11,12,13,14,14a, 15,16,17, 18,19, 20,21,22,23-tetradecahydro-1,20-etheno-10H-pyrrolo[1,2-e]-dibenz[b,n][1,5,8,11]-oxatriazacyclopentadecin-20-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid;
[(12S,15S,18S)-12-(2-Methoxycarbonyl-aethyl)-15-methyl-10,13,16-trioxo-10,11,12,13,14,15,16,17,18,19,20,21-dodecahydro-1,18-etheno-pyrido[2,3-b]benz-[n][1,5,8,11]oxatriaza-cyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid;
[(12S,15S,18S)-12-(2-Carboxy-aethyl)-15-methyl-10,13,16-trioxo-10,11,12,13,14,15,16,17,18,19,20,21-dodecahydro-1,18-etheno-pyrido[2,3-b]-benz[n][1,5,8,11]oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanin-(4-iodophenyl)-amid; und
[(12S,15S,18S)-12-Carboxymethyl-15-methyl-10,13,16,trioxo-10,11,12,13,14,15,16,17,18,19,20,21-dodecahydro-1,18-etheno-pyrido[2,3-b]-benzo[n][1,5,8,11]-oxatriazacyclopentadecin-18-ylcarbonyl]-L-alanyl-L-alanyl-L-alanyl-L-alanyl-L-alanyl-L-alanyl-(2-amino-2-methyl-propionyl)-L-alanyl-L-alanyl-(4-iodophenyl)-amid.

**11.** Verfahren zur Herstellung von Verbindungen nach einem der Ansprüche 1 bis 10, dadurch gekennzeichnet, dass man

a) eine Verbindung der allgemeinen Formel

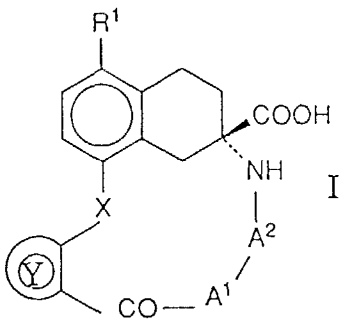

IIa bzw.

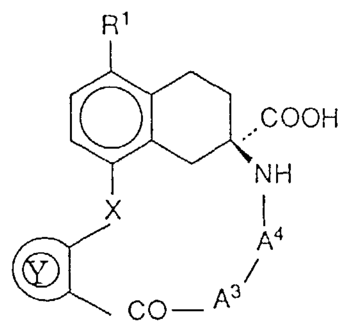

IIb

worin $R^1$, $A^1$, $A^2$, $A^3$, $A^4$, X und Y die in Anspruch 1 angegebene Bedeutung besitzen,
mit einer gegebenenfalls geschützten Kette von bis zu 20 Aminosäuren, deren C-terminale Aminosäure als freie oder geschützte Säure oder in Form eines Amids vorliegen kann, kuppelt; oder

b) eine Verbindung der allgemeinen Formel

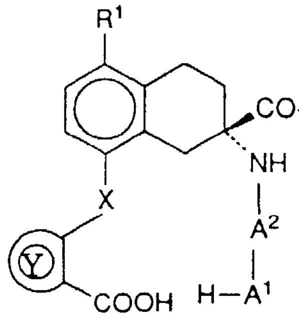 IIIa bzw. 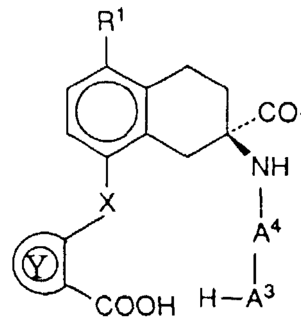 IIIb

worin $R^1$, $R^2$, $A^1$, $A^2$, $A^3$, $A^4$, X und Y die in Anspruch 1 angegebene Bedeutung besitzen, cyclisiert; oder

c) aus einer Verbindung der Formel Ia bzw. Ib, welche mindestens eine Schutzgruppe enthält, die Schutzgruppe(n) abspaltet; oder

d) eine Verbindung der Formel Ia bzw. Ib, welche ein basisches bzw. ein saures Zentrum enthält, mittels einer Säure bzw. einer Base in ein Salz überführt.

**12.** Verbindungen der in Anspruch 11 definierten allgemeinen Formeln IIa, IIb, IIIa und IIIb.

**13.** Verbindungen der allgemeinen Formeln und

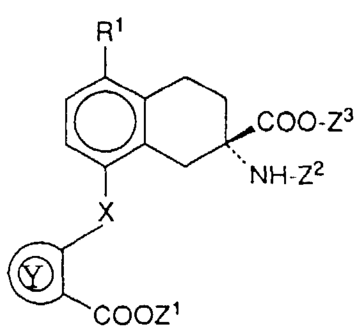 IVa und 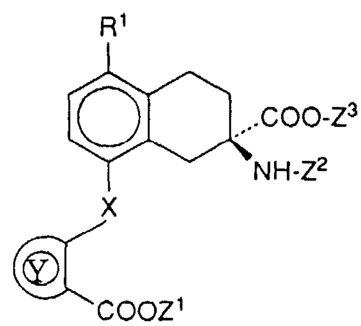 IVb

worin $R^1$ und Y die in Anspruch 1 angegebene Bedeutung besitzen und $Z^1$ und $Z^3$ Carboxylschutzgruppen und $Z^2$ eine Aminoschutzgruppe bedeuten.

**14.** Verbindungen nach Anspruch 13, worin $Z^1$ Benzyl, tert.-Butyl, Prop-2-enyl oder Pentafluorphenyl; $Z^2$ Benzyloxycarbonyl, tert.-Butyloxycarbonyl oder Fluoren-9-ylmethoxycarbonyl; und $Z^3$ Methyl, tert.-Butyl, Benzyl, Trimethylsilyläthyl oder Pentafluorphenyl bedeuten.

**15.** Verwendung von Verbindungen nach einem der Ansprüche 1 bis 10 als "Research Tools" zur Ermittlung biologisch aktiver Peptidsequenzen.

**Claims**

**1.** Tetrahydronaphthalene derivatives of the general formulae

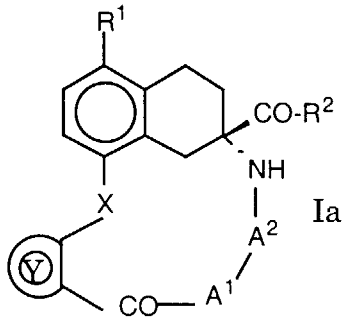

Ia

and

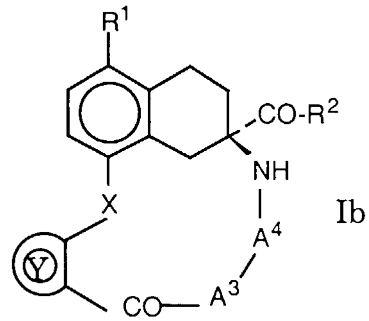

Ib

wherein

| | |
|---|---|
| $R^1$ | signifies hydrogen, bromine, cyano, formyl, hydroxy, lower alkyl, lower alkenyl, lower alkynyl, lower alkoxy, aryloxy, lower aralkoxy or aryl; |
| $R^2$ | signifies an optionally protected chain of up to 20 amino acid residues, the C-terminal amino acid of which can be present as the free or protected acid or in the form of an amide; |
| $A^1$, $A^2$, $A^3$ and $A^4$ | each signify residues of α-amino acids, the α-C atom of which, when it is asymmetric, is present in $A^1$ and $A^2$ in the L configuration and in $A^3$ and $A^4$ in the D configuration; |
| X | signifies oxygen or sulphur; and |
| Y | and the two C atoms together signify an optionally substituted benzene, furan, thiophene, pyridine or pyrimidine ring; |

as well as salts thereof.

**2.** Compounds according to claim 1, wherein $R^1$ in the meaning "aryl" signifies phenyl mono- or disubstituted by lower alkyl or lower alkoxy or phenyl substituted by lower alkylenedioxy.

**3.** Compounds according to claim 1 or 2, wherein $R^2$ contains 9 to 15 amino acid residues.

**4.** Compounds according to claim 3, wherein $R^2$ contains 9, 12 or 15 amino acid residues.

**5.** Compounds according to any one of claims 1 to 4, wherein $R^2$ contains residues of α-amino acids, the α-C atom of which is present in the L configuration when it is asymmetric.

**6.** Compounds according to claim 5, wherein $R^2$ contains residues of L-alanine, L-glutamine, L-glutamic acid, L-isoleucine, L-leucine, L-lysine or 2-amino-2-methylpropionic acid.

**7.** Compounds according to any one of claims 1 to 6, wherein $A^1$ or $A^3$ signifies a residue of L- or D-alanine, asparagine, aspartic acid, glutamine, glutamic acid or lysine or of 2-amino-2-methylpropionic acid and $A^2$ or $A^4$ signifies a residue of L- or D-alanine, asparagine, aspartic acid, glutamine, glutamic acid or lysine.

**8.** Compounds according to claim 7, wherein $A^1$ or $A^3$ signifies L- or D-alanyl or lysyl and $A^2$ or $A^4$ signifies L- or D-alanyl.

**9.** Compounds according to any one of claims 1 to 8 wherein the benzene, furan, thiophene, pyridine or pyrimidine ring denoted by Y is unsubstituted or is mono- or disubstituted by nitro, amino, lower alkanoylamino, hydroxy, lower alkoxy, lower alkanoyloxy, lower alkyl, fluorine, cyano, carboxy or formyl or is condensed with a benzene ring to form a bicyclic system.

**10.** [(12S,15S,18S)-12,15-Dimethyl-10,13,16-trioxo-10,11,12,13,14,15,16,17, 18,19,20,21 -dodecahydro-1,18-etheno-dibenz[b,n][1,5,8,11]oxatriazacyclopentadecen-18-ylcarbonyl]-L-alanyl-L-alanyl-L-alanyl-L-alanyl-L-alanyl-L-alanyl-(2-amino-2-methyl-propionyl)-L-alanyl-L-alanine (4-iodophenyl)amide;

[(12R,15R,18R)-12,15-dimethyl-10,13,16-trioxo-10,11, 12,13,14,15,16,17,18,19,20,21-dodecahydro-1,18-etheno-dibenz[b,n][1,5,8,11]oxatriazacyclopentadecen-18-ylcarbonyl]-L-alanyl-L-alanyl-L-alanyl-L-alanyl-L-alanyl-L-alanyl-(2-amino-2-methyl-propionyl)-L-alanyl-L-alanine (4-iodophenyl)amide;
[(12S,15S,18S)-12,15-dimethyl-10,13,16-trioxo-10,11,12,13,-14,15,16,17,18,19,20,21-dodecahydro-1,18-etheno-dibenz[b,n][1,5,8,11]oxatriaza-cyclopentadecen-18-ylcarbonyl]-L-leucyl-L-lysyl-L-alanyl-L-glutamyl-L-isoleucyl-L-alanyl-L-glutaminyl-L-lysyl-L-leucinamide trifluoroacetate;
[(12R,15R,18R)-12,15-dimethyl-10,13,16-trioxo-10,11, 12,13,14,15,16,17,18,19,20,21-dodecahydro-1,18-etheno-dibenz[b,n][1,5,8,11]-oxatriazacyclopentadecen-18-ylcarbonyl]-L-leucyl-L-lysyl-L-alanyl-L-glutamyl-L-isoleucyl-L-alanyl-L-glutaminyl-L-lysyl-L-leucinamide trifluoroacetate;
[(14aS,17S,20S)-8-acetylamino-17-methyl-10,15,18-trioxo-11,12,13,14,14a,15,16,17,18,19,20,21,22,23-tetradecahydro-1,20-etheno-10H-pyrrolo[1,2-e]-dibenz[b,n][1,5,8,11]-oxatriazacyclopentadecen-20-ylcarbonyl]-L-alanyl-L-alanyl-L-alanyl-L-alanyl-L-alanyl-L-alanyl-(2-amino-2-methyl-propionyl)-L-alanyl-L-alanine (4-iodophenyl)amide;
[(12S,15S,18S)-12-(2-methoxycarbonyl-ethyl)-15-methyl-10,13,16-trioxo-10,11,12,13,14,15,16,17,18,19,20,21-dodecahydro-1,18-etheno-pyrido[2,3-b]benz[n][1,5,8,11]oxatriazacyclopentadecen-18-ylcarbonyl]-L-alanyl-L-alanyl-L-alanyl-L-alanyl-L-alanyl-L-alanyl-(2-amino-2-methyl-propionyl)-L-alanyl-L-alanine (4-iodophenyl)amide;
[(12S,16S,19S)-16-methyl-10,13,17,23-tetraoxo-10,11, 12,13,14,15,16,17,18,19,20,21,22,23-tetradecahydro-1,19-etheno-12,15-metheno-pyrido[2,3-b]benz[o][1,5,9,12]oxatriazacyclohexadecen-19-ylcarbonyl]-L-alanyl-L-alanyl-L-alanyl-L-alanyl-L-alanyl-L-alanyl-(2-amino-2-methyl-propionyl)-L-alanyl-L-alanine (4-iodophenyl)amide;
[(12S,15S,18S)-12-(2-carboxy-ethyl)-15-methyl-10,13,16-trioxo-10,11,12,13,14,15,16,17,18,19,20,21-dodecahydro-1,18-etheno-pyrido[2,3-b]benz[n][1,5,8,11]oxatriazacyclopentadecen-18-ylcarbonyl]-L-alanyl-L-alanyl-L-alanyl-L-alanyl-L-alanyl-L-alanyl-(2-amino-2-methyl-propionyl)-L-alanyl-L-alanine (4-iodophenyl) amide; and
[(12S,15S,18S)-12-carboxymethyl-15-methyl-10,13,16,trioxo-10,11,12,13,14,15,16,17,18,19,20,21-dodecahydro-1,18-etheno-pyrido[2,3-b]-benzo[n][1,5,8,11]oxatriazacyclopentadecen-18-ylcarbonyl]-L-alanyl-L-alanyl-L-alanyl-L-alanyl-L-alanyl-L-alanyl-(2-amino-2-methyl-propionyl)-L-alanyl-L-alanyl (4-iodophenyl) amide.

**11.** A process for the manufacture of compounds according to any one of claims 1 to 10, which process comprises

a) coupling a compound of the general formula

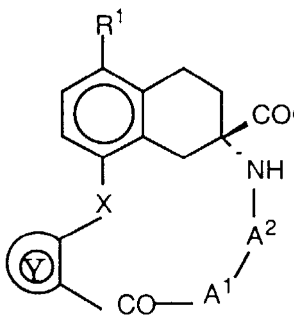
IIa or

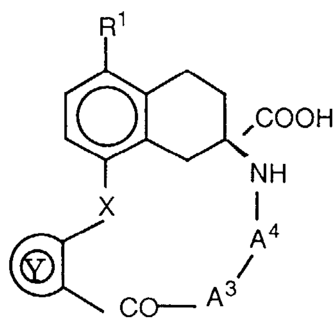
IIb

wherein $R^1$, $A^1$, $A^2$, $A^3$, $A^4$, X and Y have the significance given in claim 1,
with an optionally protected chain of up to 20 amino acids, the C-terminal amino acid of which can be present as the free or protected acid or in the form of an amide; or

b) cyclizing a compound of the general formula

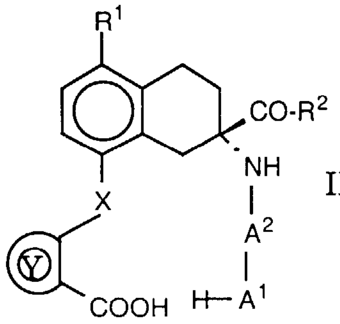 IIIa or 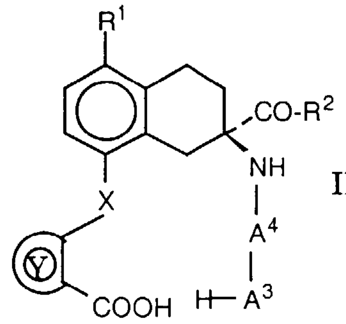 IIIb

wherein $R^1$, $R^2$, $A^1$, $A^2$, $A^3$, $A^4$, X and Y have the significance given in claim 1;

or

c) cleaving off the protecting group(s) from a compound of formula Ia or Ib which contains at least one protecting group; or

d) converting a compound of formula Ia or Ib which contains a basic centre or an acidic centre into a salt using an acid or, respectively, a base.

**12.** Compounds of general formulae IIa, IIb, IIIa and IIIb defined in claim 11.

**13.** Compounds of the general formulae

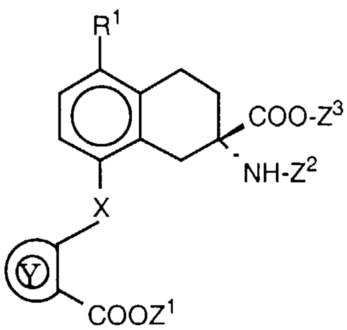 IVa or 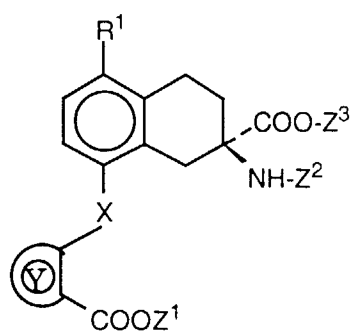 IVb

wherein $R^1$ and Y have the significance given in claim 1 and
$Z^1$ and $Z^3$ signify carboxyl protecting groups and $Z^2$ signifies an amino protecting group.

**14.** Compounds according to claim 13, wherein $Z^1$ signifies benzyl, tert.-butyl, prop-2-enyl or pentafluorophenyl; $Z^2$ signifies benzyloxycarbonyl, tert.-butyloxycarbonyl or fluoren-9-ylmethoxycarbonyl; and $Z^3$ signifies methyl, tert.-butyl, benzyl, trimethylsilylethyl or pentafluorophenyl.

**15.** The use of compounds according to any one of claims 1 to 10 as "research tools" for the determination of biologically active peptide sequences.

## Revendications

**1.** Dérivés de tétrahydronaphtalène, de formules générales

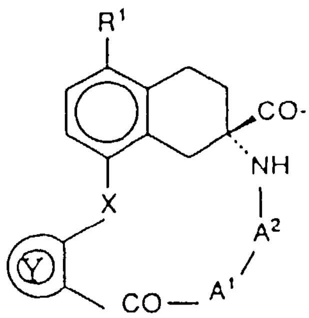 Ia et 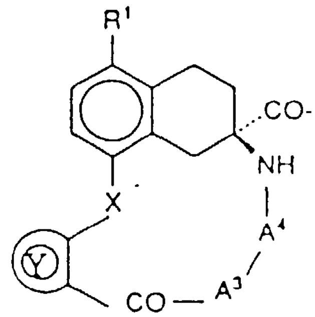 Ib

dans lesquelles

| | |
|---|---|
| $R^1$ | représente un atome d'hydrogène ou de brome, ou un groupe cyano, formyle, hydroxyle, alkyle inférieur, alcényle inférieur, alcynyle inférieur, alcoxy inférieur, aryloxy, aralcoxy inférieur ou aryle; |
| $R^2$ | représente une chaîne éventuellement protégée de jusqu'à 20 résidus d'aminoacides, dont l'aminoacide C-terminal peut se trouver sous forme d'acide libre ou protégé ou sous forme d'un amide; |
| $A^1$, $A^2$, $A^3$ et $A^4$ | représentent chacun un résidu d'α-amino-acide dont l'atome de carbone a, lorsqu'il est asymétrique, se trouve en configuration L dans $A^1$ et $A^2$ et en configuration D dans $A^3$ et $A^4$; |
| X | représente un atome d'oxygène ou de soufre; et |
| Y | représente, conjointement avec les deux atomes de carbone, un cycle benzénique, furanne, thiophène, pyridine ou pyrimidine, éventuellement substitué; |

ainsi que leurs sels.

**2.** Composés selon la revendication 1, dans lesquels $R^1$, dans la possibilité de signification "aryle", représente un groupe phényle mono- ou disubstitué par un ou des groupes alkyle inférieur ou alcoxy inférieur, ou substitué par un groupe alkylènedioxy inférieur.

**3.** Composés selon la revendication 1 ou 2, dans lesquels $R^2$ contient de 9 à 15 résidus d'aminoacides.

**4.** Composés selon la revendication 3, dans laquelle $R^2$ contient 9, 12 ou 15 résidus d'aminoacides.

**5.** Composés selon l'une des revendications 1 à 4, dans lesquels $R^2$ contient des résidus d'a-aminoacides dont l'atome de carbone a, lorsqu'il est asymétrique, se trouve en configuration L.

**6.** Composé selon la revendication 5, dans lesquels $R^2$ contient des résidus de L-alanine, L-glutamine, acide L-glutamique, L-isoleucine, L-leucine, L-lysine ou acide 2-amino-2-méthylpropionique.

**7.** Composés selon l'une des revendications 1 à 6, dans lesquels $A^1$ ou $A^3$ représente un résidu de L- ou D-alanine, asparagine, acide aspartique, glutamine, acide glutamique ou lysine ou d'acide 2-amino-2-méthyl-propionique, et $A^2$ ou $A^4$ représente un résidu de L- ou D-alanine, asparagine, acide aspartique, glutamine, acide glutamique ou lysine.

**8.** Composés selon la revendication 7, dans lesquels $A^1$ ou $A^3$ représente le résidu L- ou D-alanyle ou lysyle et $A^2$ ou $A^3$ représente le résidu L- ou D-alanyle.

**9.** Composés selon l'une des revendications 1 à 8, dans lesquels le cycle benzénique, furanne, thiophène, pyridine ou pyrimidine représenté par Y n'est pas substitué ou est mono- ou disubstitué par l'atome de fluor ou par un ou des groupes nitro, amino, alcanoylamino inférieur, hydroxyle, alcoxy inférieur, alcanoyloxy inférieur, alkyle inférieur, cyano, carboxyle ou formyle, ou est condensé avec une cycle benzénique pour former un système bicyclique.

**10.** [(12S,15S,18S)-12,15-diméthyl-10,13,16-trioxo-10,11,12,13,14,15,16,17,18,19,20,21-dodécahydro-1,18-éthéno-dibenz[b,n][1,5,8,11]oxatriazacyclopentadécyn-18-ylcarbonyl]-L-alanyl-L-alanyl-L-alanyl-L-alanyl-L-alanyl-L-alanyl-(2-amino-2-méthyl-propionyl)-L-alanyl-L-alanine-(4-iodophényl)amide,

[(12R,15R,18R)-12,15-diméthyl-10,13,16-trioxo-10,11,12,13,14,15,16,17,18,19,20,21-dodécahydro-1,18-éthéno-dibenz[b,n][1,5,8,11]oxatriazacyclopentadécyn-18-ylcarbonyl]-L-alanyl-L-alanyl-L-alanyl-L-alanyl-L-alanyl-L-alanyl-(2-amino-2-méthyl-propionyl)-L-alanyl-L-alanine-(4-iodophényl)amide,
[(12S,15S,18S)-12,15-diméthyl-10,13,16-trioxo-10,11,12,13,14,15,16,17,18,19,20,21-dodécahydro-1,18-éthéno-dibenz[b,n][1,5,8,11]oxatriazacyclopentadécyn-18-ylcarbonyl]-L-leucyl-L-lysyl-L-alanyl-L-glutamyl-L-iso-leucyl-L-alanyl-L-glutaminyl-L-lysyl-leucinamide (trifluoroacétate),
[(12R,15R,18R)-12,15-diméthyl-10,13,16-trioxo-10,11,12,13,14,15,16,17,18,19,20,21-dodécahydro-1,18-éthéno-dibenz[b,n][1,5,8,11]oxatriazacyclopentadécyn-18-ylcarbonyl]-L-leucyl-L-lysyl-L-alanyl-L-glutamyl-L-iso-leucyl-L-alanyl-L-glutaminyl-L-lysyl-leucinamide (trifluoroacétate),
[(14aS,17S,20S)-8-acétylamino-17-méthyl-10,15,18-trioxo-11,12,13,14,14a,15,16,17,18,19,20,21,22,23-tétradécahydro-1,20-éthéno-10H-pyrrolo[1,2-e]-dibenz[b,n]-[1,5,8,11]oxatriazacyclopentadécyn-20-ylcarbonyl]-L-alanyl-L-alanyl-L-alanyl-L-alanyl-L-alanyl-L-alanyl-(2-amino-2-méthyl-propionyl)-L-alanyl-L-alanine-(4-iodo-phényl)amide,
[(12S,15S,18S)-12-(2-méthoxycarbonyl-éthyl)-15-méthyl-10,13,16-trioxo-10,11,12,13,14,15,16,17,18,19,20,21-dodécahydro-1,18-éthéno-pyrido[2,3-b]benz[n]-[1,5,8,11]-oxatriazacyclopentadécyn-18-ylcarbonyl]-L-alanyl-L-alanyl-L-alanyl-L-alanyl-L-alanyl-L-alanyl-(2-amino-2-méthyl-propionyl)-L-alanyl-L-alanine-(4-iodophényl)amide,
[(12S,15S,18S)-12-(2-carboxy-éthyl)-15-méthyl-10,13,16-trioxo-10,11,12,13,14,15,16,17,18,19,20,21-dodécahydro-1,18-éthéno-pyrido[2,3-b]benz[n]-[1,5,8,11]-oxatriazacyclopentadécyn-18-ylcarbonyl]-L-alanyl-L-alanyl-L-alanyl-L-alanyl-L-alanyl-L-alanyl-(2-amino-2-méthyl-propionyl)-L-alanyl-L-alanine-(4-iodophényl) amide, et
[(12S,15S,18S)-12-carboxyméthyl-15-méthyl-10,13,16-trioxo-10,11,12,13,14,15,16,17,18,19,20,21-dodécahydro-1,18-éthéno-pyrido[2,3-b]benzo[n]-[1,5,8,11]-oxatriazacyclopentadécyn-18-ylcarbonyl]-L-alanyl-L-alanyl-L-alanyl-L-alanyl-L-alanyl-L-alanyl-(2-amino-2-méthyl-propionyl)-L-alanyl-L-alanyl-(4-iodophényl)amide.

**11.** Procédé pour la préparation des composés selon l'une des revendications 1 à 10, caractérisé en ce que

a) on combine un composé de formule générale

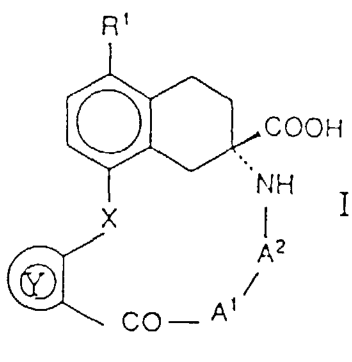

IIa

ou

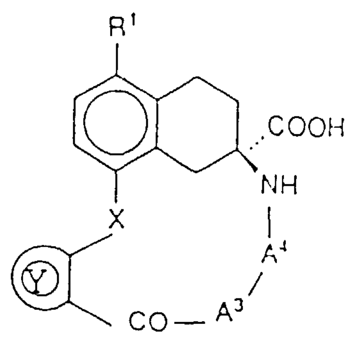

IIb

où $R^1$, $A^1$, $A^2$, $A^3$, X et Y ont les significations données dans la revendication 1,
avec une chaîne éventuellement protégée de jusqu'à 20 aminoacides, dont l'aminoacide C-terminal peut se trouver sous forme d'acide libre ou protégé ou sous forme d'un amide; ou
b) on cyclise un composé de formule générale

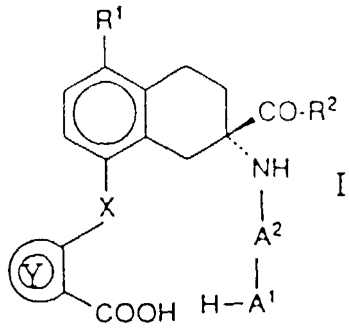

IIIa ou

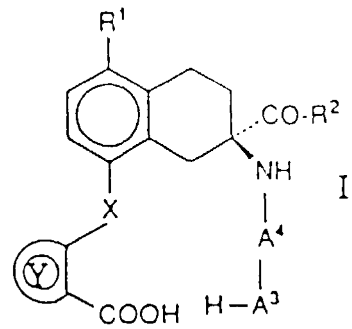

IIIb

où $R^1$, $R^2$, $A^1$, $A^2$, $A^3$, $A^4$, X et Y ont les significations données dans la revendication 1; ou

c) on élimine le(s) groupe(s) protecteur(s) d'un composé de formule Ia ou Ib, qui comporte au moins un groupe protecteur; ou

d) on convertit en un sel, à l'aide d'un acide ou d'une base, un composé de formule Ia ou Ib qui comporte un centre basique ou, respectivement, un centre acide.

**12.** Composés de formules générales IIa, IIb, IIIa et IIIb définies dans la revendication 11.

**13.** Composés de formules générales

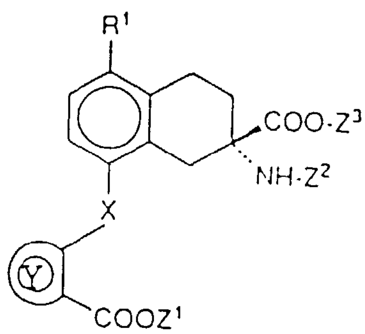

IVa et

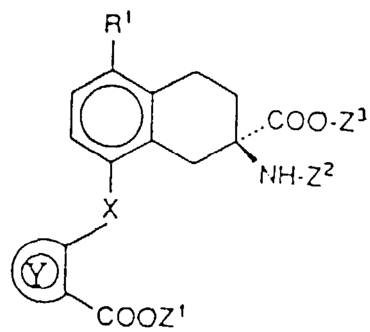

IVb

dans lesquelles $R^1$ et Y ont les significations données dans la revendication 1 et $Z^1$ et $Z^3$ représentent des groupes protecteurs de fonction carboxyle, et $Z^2$ représente un groupe protecteur de fonction amino.

**14.** Composés selon la revendication 13, dans lesquels $Z^1$ représente le groupe benzyle, tert-butyle, prop-2-ényle ou pentafluorophényle; $Z^2$ représente le groupe benzyloxycarbonyle, tert-butyloxycarbonyle ou fluorén-9-ylméthoxycarbonyle; et $Z^3$ représente le groupe méthyle, tert-butyle, benzyle, triméthylsilyléthyle ou pentafluorophényle.

**15.** Utilisation des composés selon l'une des revendications 1 à 10, en tant qu'outils de recherche *(Research Tools)* pour la détermination de séquences de peptides biologiquement actifs.

Fig. 1

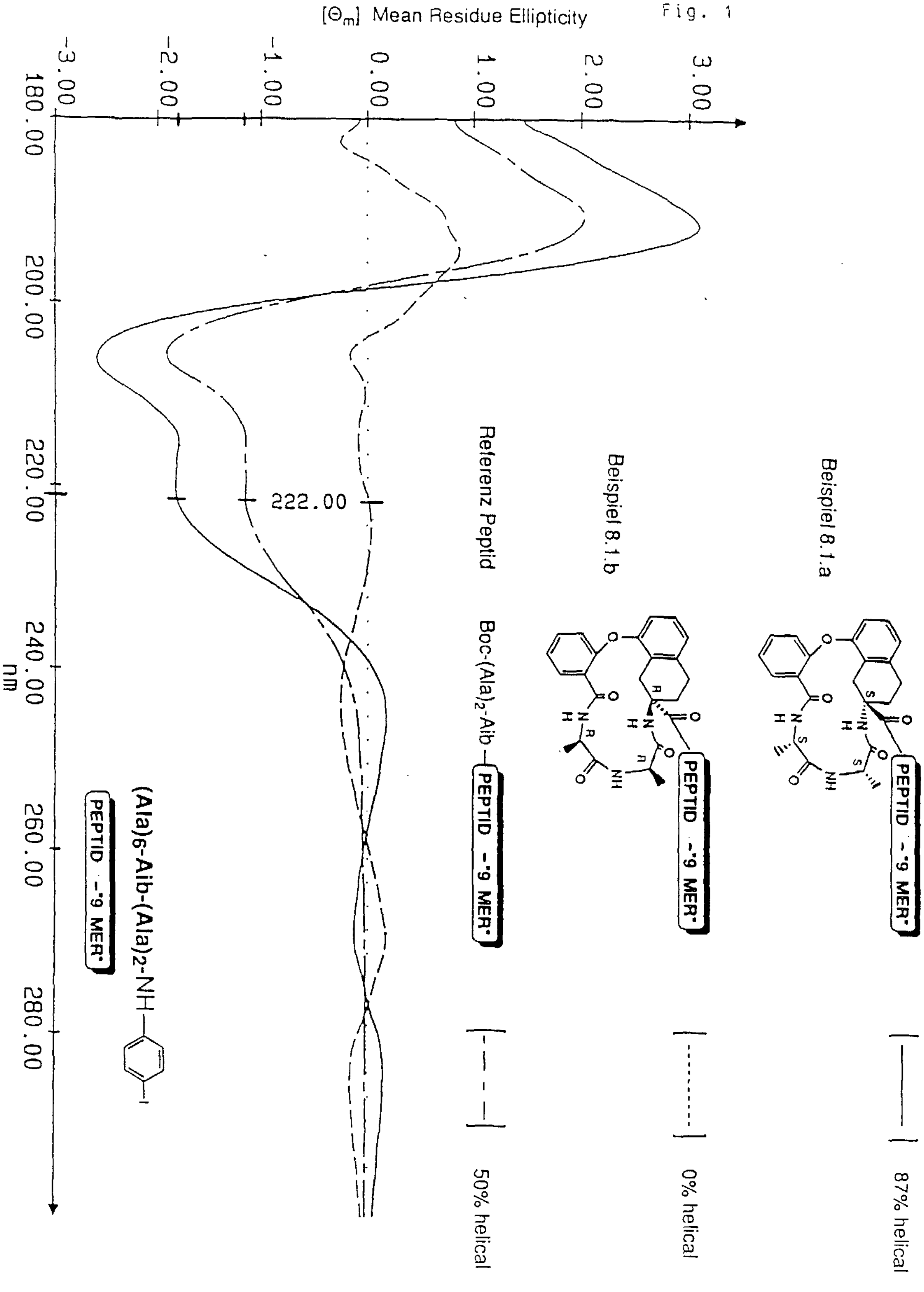
[Θm] Mean Residue Ellipticity
3.00
2.00
1.00
0.00
-1.00
-2.00
-3.00
180.00
200.00
220.00
222.00
240.00
260.00
280.00
nm
Beispiel 8.1.a
PEPTID -'9 MER'
87% helical
Beispiel 8.1.b
PEPTID -'9 MER'
0% helical
Referenz Peptid
Boc-(Ala)2-Aib—PEPTID -'9 MER'
50% helical
(Ala)6-Aib-(Ala)2-NH—
PEPTID -'9 MER'

Fig. 2

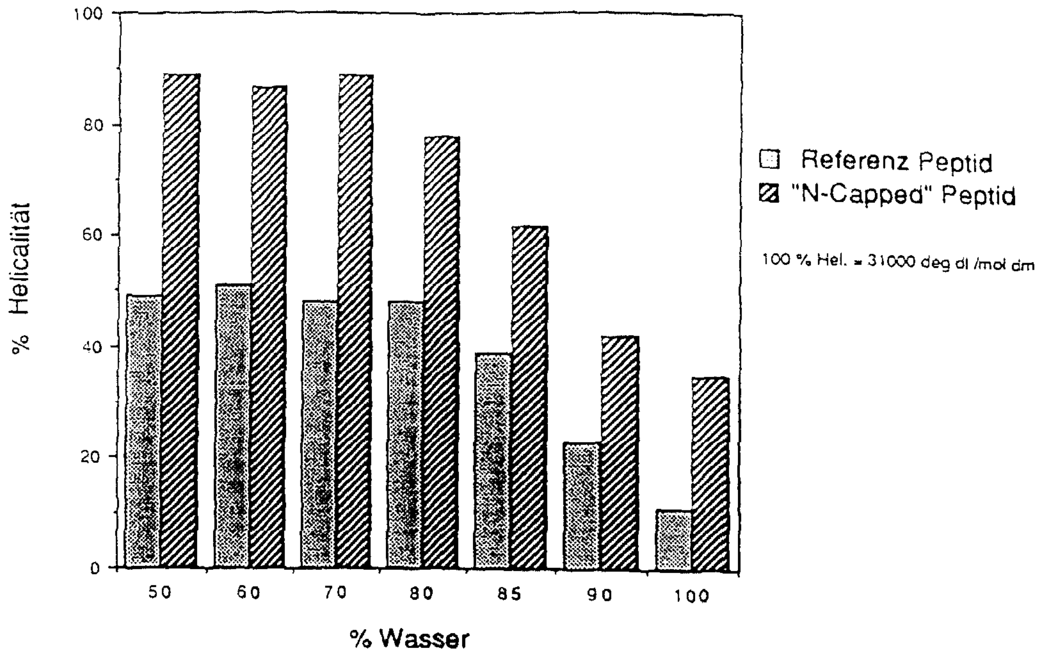


Beispiel 8.2.a